# EUROPEAN PATENT SPECIFICATION

(11) **EP 2 989 088 B1**
(45) Date of publication and mention of the grant of the patent: **27.09.2017**
(21) Application number: 14720946.4
(22) Date of filing: 25.04.2014
(51) Int. Cl.: C07D 401/12, A61K 38/07, A61K 38/40, C07D 417/12, C07D 417/14, C07D 207/08, C07K 5/02, A61P 35/00, A61K 31/401, A61K 31/4025, A61K 31/427, A61K 31/4439

(54) **DERIVATIVES OF DOLASTATIN 10 AND AURISTATINS**
DERIVATE VON DOLASTATIN 10 UND AURISTATINEN
DÉRIVÉS DE DOLASTATINE 10 ET D'AURISTATINES

(30) Priority: 25.04.2013 FR 1353793
(43) Date of publication of application: 02.03.2016
(73) Proprietor: Pierre Fabre Médicament, 92100 Boulogne-Billancourt (FR)
(72) Inventor: PEREZ, Michel, F-81100 Castres (FR); RILATT, Ian, F-81100 Castres (FR); LAMOTHE, Marie, F-81100 Castres (FR)
(74) Representative: Regimbeau
(86) International application number: PCT/EP2014/058427
(87) International publication number: WO 2014/174064

(56) References cited:
- EP-A1- 0 731 106
- WO-A1-2011/154359
- WO-A1-2012/041805
- WO-A1-2012/123423
- WO-A1-2012/166559
- WO-A2-2012/059882

## Description

The subject of the present invention concerns novel derivatives of dolastatin 10 and auristatins, their methods of production, pharmaceutical compositions containing the same and the use thereof as medicinal product in particular in the treatment of cancer.

Dolastatin 10 (D10) is a cytotoxic peptide derivative isolated from a marine mollusc (*Dolabella auricularia*) whose absolute configuration was determined and later confirmed after total synthesis of the product (Pettit G. R. J. Am. Chem. Soc. 1987, 109, 6883; Pettit G. R. J. Am. Chem. Soc. 1987, 109, 7581; Pettit, G. R. Heterocycles 1989, 28, 553; Pettit, G. R. J. Am. Chem. Soc. 1989,111, 5015; Pettit G. R. J. Am. Chem. Soc. 1991, 113, 6692). D10 is formed of 5 units called dolavaline (Dov), valine (Val), dolaisoleucine (Dil), dolaproine (Dap) and dolaphenine (Doe). A certain number of analogues of this compound have been synthesised by modifying the nature of its component amino acids (Pettit G. R. J. Med. Chem. 1990, 33, 3133; Miyazaki K. Peptide Chemistry 1993, 31, 85; Miyazaki K. Chem. Pham. Bull. 1995, 43, 1706). Modifications of the C-terminal part (right end) have also been performed and have led to numerous derivatives which include auristatin E or F (Pettit G. R. Anticancer Drug Design, 1998, 13, 243; Pettit G. R. Antimicrobial Agents And Chemotherapy, 1998, 2961).

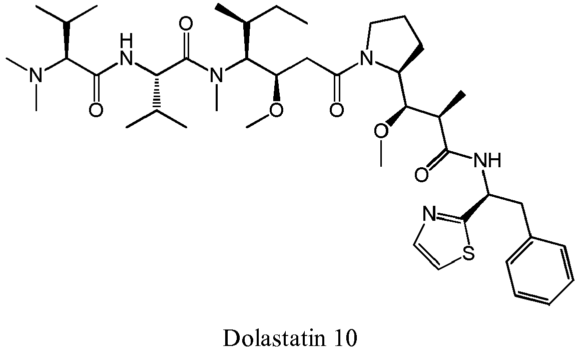

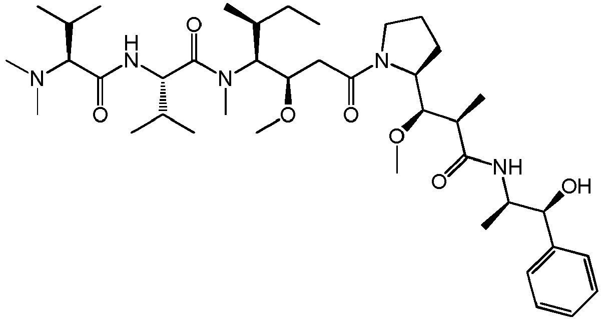

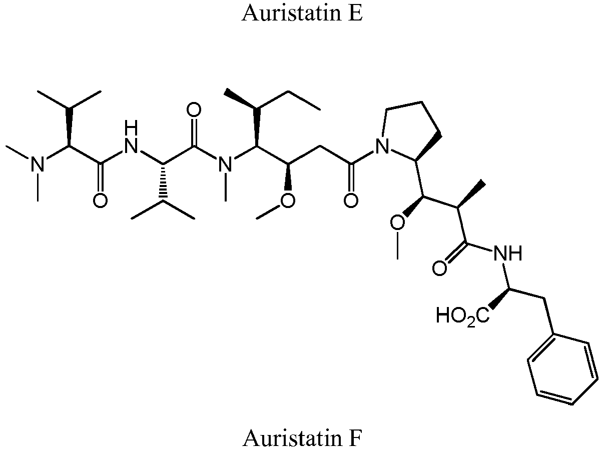

The present invention has focused on modification of the N-terminal part (left end) of derivatives of dolastatin 10 and auristatins E and F. The few examples published in the literature on modifications made at this position have led to losses of activity (Miyazaki K. Chem. Pham. Bull. 1995, 43, 1706) or are less active than the compounds of the present invention (see WO 2012/041805 and WO 2012/123423 which disclose derivatives with a carboxyl substituent instead of a hydroxyl or amino substituent as in the present invention). The compounds described in the present invention differ from the prior art through their original chemical structures and also through their remarkable biological property that is fully unexpected having regard to the elements published in the literature. These remarkable activities result in making these compounds suitable for use in the treatment of cancer.

In addition, these compounds have the advantage of being both active as cytotoxic agents and more soluble than the parent compounds.

The subject of the present invention is thus a compound of following formula (I): 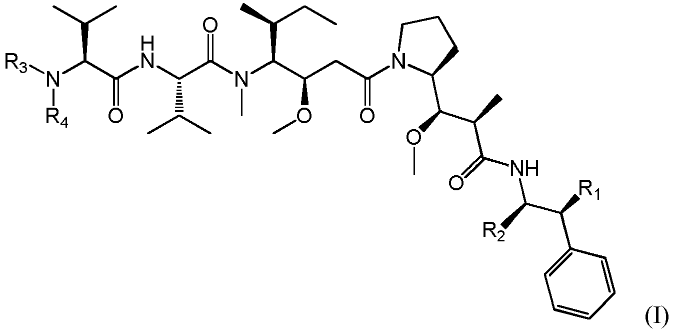 where:
- R₁ = OH and R₂ = methyl, or
   R₁ = H and R₂ = COOH, COOMe or thiazol-2-yl,
- R₃ is a (C₁-C₆)alkyl group, and
- R₄ is an aryl-(Cl -2)alkyl group substituted by one group (preferably on the aryl part) chosen from among OH and NR₉R₁₀ groups with R₉ and R₁₀ each independently of one another representing H or a (C₁-C₆)alkyl group (such as methyl), wherein the aryl-(C1-2)alkyl group is a phenyl-(C₁-C₂)alkyl group linked to the remainder of the molecule via its (C₁-C₂)alkyl moiety, or a pharmaceutically acceptable salt, hydrate or solvate thereof

The radicals R₂ to R₄, and in particular R₄, may be chiral groups and may be in the form of their different stereoisomers and optionally in the form of a mixture of stereoisomers.

By «stereoisomer», in the meaning of the present invention is meant a geometric isomer or an optical isomer.

Geometrical isomers result from the different position of the substituents on a double bond which may therefore have a Z or E configuration.

Optical isomers result in particular from the different position in space of the substituents on a carbon atom comprising 4 different substituents. This carbon atom then forms a chiral or asymmetric centre. Optical isomers comprise diastereoisomers and enantiomers. Optical isomers which are images of one another in a mirror but which cannot be superimposed are called « enantiomers ». Optical isomers which are not superimposable images of one another in a mirror are called « diastereoisomers ».

A mixture containing equal quantities of two individual enantiomer forms of opposite chirality is called a « racemic mixture ».

In the present invention by « pharmaceutically acceptable » is meant that which can be used in the preparation of a pharmaceutical composition which is generally, safe non-toxic and neither biologically nor otherwise undesirable, and which is acceptable for veterinary use as well as for human pharmaceutical use.

By « pharmaceutically acceptable salt, hydrate or solvate » of a compound is meant a salt, hydrate or solvate which is pharmaceutically acceptable as defined herein and which has the desired pharmacological activity of the parent compound.

Pharmaceutically acceptable salts notably comprise:
(1) the addition salts of a pharmaceutically acceptable acid formed with pharmaceutically acceptable inorganic acids such as hydrochloric, hydrobromic, phosphoric, sulfuric and similar acids; or formed with pharmaceutically acceptable organic acids such as acetic, trifluoroacetic, propionic, succinic, fumaric, malic, tartaric, citric, ascorbic, maleic, glutamic, benzoic, salicylic, toluenesulfonic, methanesulfonic, stearic, lactic and similar acids; and
(2) the addition salts of a pharmaceutically acceptable base formed when an acid proton present in the parent compound is either replaced by a metallic ion e.g. an alkaline metal ion, an alkaline-earth metal ion or an aluminium ion; or coordinated with a pharmaceutically acceptable organic base such as lysine, arginine and similar; or with a pharmaceutically acceptable inorganic base such as sodium hydroxide, potash, calcium hydroxide and similar.

These salts can be prepared from the compounds of the invention containing a base or acid function, and the corresponding acids or bases using conventional chemical methods.

The formula (I) compounds of the invention are preferably in salt form, and in particular a pharmaceutically acceptable acid addition salt.

Preferably, the compounds of formula (I) according to the present invention are in the form of a pharmaceutically acceptable acid addition salt, the acid possibly being trifluoroacetic acid, acetic acid or hydrochloric acid for example, and in particular trifluoroacetic acid.

The solvates comprise the conventional solvates obtained at the last preparation step of the compounds of the invention due to the presence of solvent, the solvent possibly being ethanol for example.

By « alkyl » in the present invention is meant a straight-chain or branched, saturated hydrocarbon chain. For example, mention can be made of methyl, ethyl, propyl, isopropyl, butyl, isobutyl, *sec*-butyl, *tert*-butyl, pentyl or hexyl groups.

By « (Cₓ-C_{y})alkyl » in the meaning of the present invention is meant an alkyl chain such as defined above comprising x to y carbon atoms. Therefore, a (C₁-C₆)alkyl group is an alkyl chain having 1 to 6 carbon atoms.

By «aryl» in the meaning of present invention is meant an aromatic hydrocarbon group preferably having 6 to 10 carbon atoms and able to comprise one or two fused rings. For example a phenyl or a naphthyl can be cited. Advantageously it is a phenyl.

By« aryl-(C1-2)alkyl » in the meaning of the present invention is meant an aryl group such as defined above linked to the remainder of the molecule via an alkyl group such as defined above and comprising 1 or 2 carbon atoms. The aryl moiety is a phenyl moiety. The (C1-2)alkyl group is advantageously a benzyl or phenethyl group.

Among the compounds of the invention, one particularly appreciated class of compounds corresponds to the formula (I) compounds in which R₁ is OH and R₂ represents a methyl.

Another particularly appreciated class of compounds corresponds to the formula (I) compounds in which R₁ is a hydrogen and R₂ is a thiazol-2-yl group.

Another class of particularly appreciated compounds corresponds to the formula (I) compounds in which R₁ is a hydrogen and R₂ is COOMe.

Another class of particularly appreciated compounds corresponds to the formula (I) compounds in which R₁ is a hydrogen and R₂ is a COOH group.

Therefore the compounds of the invention are formula (I) compounds in which:
- R₁=OH and R₂=Me (methyl), or
- R₁=H and R₂=COOH, COOMe or thiazol-2-yl.

Preferably, R₁ is H and R₂ is COOH or COOMe.

According to a first preferred embodiment, R₁ is H and R₂ is COOH.

According to a second preferred embodiment, R₁ is H and R₂ is COOMe.

R₃ particularly represents a methyl group.

R₄ represents an aryl-(C₁-C₂)alkyl group substituted by one group chosen from among OH and NR₉R₁₀, and notably being NR₉R₁₀.

R₄ represents advantageously an aryl-(C₁-C₂)alkyl group substituted by one group on the aryl moiety chosen from among OH and NR₉R₁₀, and notably being NR₉R₁₀.

The aryl group is a phenyl group.

Thus R₄ represents a phenyl-(C₁-C₂)alkyl substituted by one group (preferably on the phenyl moiety) chosen from among OH and NR₉R₁₀, and notably being NR₉R₁₀.

R₄ can thus have the following formula: 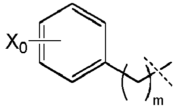 wherein X₀ represents OH or NR₉R₁₀, in particular NR₉R₁₀, and m represents an integer being 1 or 2.

According to a preferred embodiment, R₄ has the following formula: 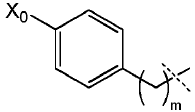 with X₀ and m as defined previously, and in particular with X₀=NR₉R₁₀ and m=1 or 2.

R₄ may in particular be chosen from among:

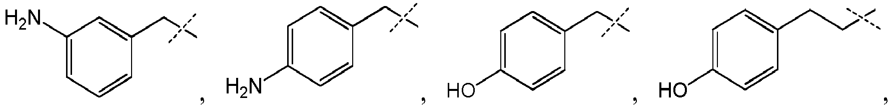

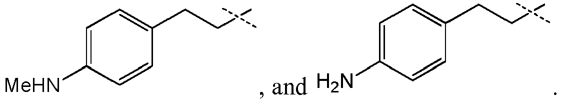

Advantageously, the formula (I) compound is chosen from among the compounds 11-15, 19-20, 23-24, 27-29, 49-51 and 61-64 described in the examples below.

A further subject of the present invention is a formal (I) compound such as defined above for use as medicinal product, in particular for the treatment or prevention of cancer or benign proliferative disorders.

The present invention also concerns the use of a formula (I) compound such as defined above for producing a medicinal product, particularly intended for the treatment or prevention of cancer or benign proliferative disorders.

The cancer to be treated or prevented is more particularly cancer of the lung, pancreas, skin, head, neck, uterus, ovaries, anus, stomach, colon, breast, oesophagus, small intestine, thyroid gland, lymphatic system, prostate, kidney, or bladder, or an acute or chronic leukaemia, or a combination of two or more of these cancers.

By benign proliferative disorders is meant proliferating disorders which cannot give rise to metastases or which have not yet progressed towards a cancer (pre-cancerous tumours).

A further subject of the present invention is a pharmaceutical composition comprising a formula (I) compound such as defined above and at least one pharmaceutically acceptable excipient.

The active ingredient can be administered in unit forms of administration, in a mixture with conventional pharmaceutical carriers, to animals or to human beings. Suitable unit forms of administration comprise forms via oral route, forms for sublingual or buccal administration, forms for administration via parenteral route (subcutaneous, intradermal, intramuscular or intravenous), forms for topical administration (on the skin and mucosa, including intranasal and intraocular administration) and forms for rectal administration.

Such compositions may be in the form of a solid, liquid, emulsion, lotion or cream.

As solid compositions, for oral administration, use can be made of tablets, pills, powders (hard or soft gelatine capsules) or granules. In these compositions, the active ingredient of the invention is mixed with one or more inert diluents such as starch, cellulose, sucrose, lactose or silica, in a stream of argon. These compositions may also comprise substances other than diluents, for example one or more lubricants such as magnesium stearate or talc, a colouring agent, a coating (coated tablets) or a varnish.

As liquid compositions for oral administration, use can be made of solutions, suspensions, emulsions, syrups and elixirs that are pharmaceutically acceptable and contain inert diluents such as water, ethanol, glycerol, vegetable oils or paraffin oil. These compositions may comprise substances other than diluents; for example wetting, sweetening, thickening, flavouring or stabilising products.

The sterile compositions for parenteral administration may preferably be aqueous or non-aqueous solutions, suspensions or emulsions. As solvent or vehicle, use can be made of water, propyleneglycol, a polyethyleneglycol, vegetable oils, in particular olive oil, injectable organic esters e.g. ethyl oleate or other suitable organic solvents. These compositions may also contain adjuvants, in particular wetting, isotonic, emulsifying, dispersing and stabilising agents. Sterilisation can be performed in several manners, for example by sanitising filtration, by incorporating sterilising agents into the composition, by radiation or by heating. They can also be prepared in the form of solid sterile compositions which can be dissolved at the time of use in sterile water or any other injectable sterile medium.

The compositions for rectal administration are suppositories or rectal capsules which, in addition to the active ingredient, contain excipients such as cocoa butter, semi-synthetic glycerides or polyethyleneglycols.

The compositions for topical administration may for example be creams, lotions, eye drops, mouthwash, nasal drops or sprays.

The doses are dependent on the desired effect, on the length of treatment and the route of administration used. In general the physician will determine the suitable dosage in relation to the age, weight and all other factors particular to the subject to be treated.

Another active ingredient may be contained in the pharmaceutical compositions according to the present invention. In particular, it may be an anticancer agent, and in particular a cytotoxic anticancer agent such as navelbine, vinflunine, taxol, taxotere, 5-fluorouracil, methotrexate, doxorabicin, camptothecin, gemcitabin, etoposide, cis-platin or carmustine (also called BCNU); or a hormonal anticancer agent such as tamoxifen or medroxyprogesterone.

Radiation treatment (X-ray or gamma ray) may also be associated with the administering of a compound of the present invention. Such radiation can be given using an external source or by implanting minute internal radioactive sources.

The present invention also concerns the preparation of the formula (I) compounds according to the invention using the general methods described in the following synthesis schemes, optionally supplemented by any standard operation when needed that is described in the literature or well known to persons skilled in the art, or described in the examples in the experimental part hereof.

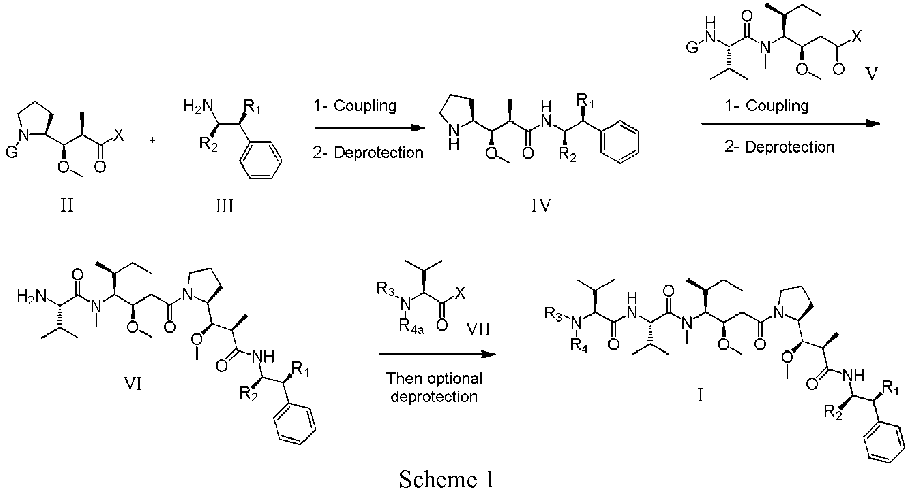

Scheme 1 illustrates the first general method which can be used to prepare formula (I) compounds. In the above general formulas, R₁, R₂, and R₃ are such as previously defined, R₄ₐ represents a R₄ group such as previously defined optionally in protected form and G is a protective group.

The first step consists of the condensing of compound (II), protected on its amine function by a protective group G, with compound (III). X may represent a leaving group such as a chlorine. In this case the first step consists of the reaction between an acid chloride and an amine. This reaction can be conducted using methods and techniques well known to those skilled in the art. In one particularly appreciated method, the two entities are caused to react in the presence of an organic or inorganic base e.g. Et₃N, iPr₂NEt, pyridine, NaH, Cs₂CO₃, K₂CO₃ in a solvent such as THF, dichloromethane, DMF, DMSO, at a temperature notably between -20°C and 100°C. X may also be a hydroxyl (OH). In this case, the first step is a condensation reaction between the carboxylic acid (II) and the amine (III). This reaction can be performed following methods and techniques well known to skilled persons. In one particularly appreciated method, these two entities are caused to react in the presence of a coupling agent such as 1-(3-dimethylaminopropyl)-3-ethyl-carbodiimide (EDC), 3-hydroxy-1,2,3-benzotriazin-4(3H)-one, a tertiary amine such as diisopropylethylamine, in a polar aprotic solvent such as dichloromethane or DMF, at a temperature notably between - 15°C and 40°C. In another particularly appreciated method, these two entities are caused to react in the presence of diethyl phosphorocyanidate (DEPC), a tertiary amine such as triethylamine, in a polar aprotic solvent such as dichloromethane or DMF, at a temperature of between -15°C and 40°C. Another particularly appreciated method consists of causing these two entities to react in the presence of O-(7-azabenzotriazol-1-yl)-1,1,3,3-tetramethyl-uroniumhexafluorophosphate (HATU), a tertiary amine such as diisopropylethylamine, in a polar aprotic solvent such as dichloromethane or DMF, at a temperature of between -15°C and 100°C.

After deprotection of the intermediate using techniques well known to those skilled in the art (« Protective Groups in Organic Synthesis », T.W. Greene, John Wiley & Sons, 2006 and «Protecting Groups », P.J. Kocienski, Thieme Verlag, 1994), compound (IV) can be condensed with compound (V) following the methods and techniques described above to lead to compound (VI) after a deprotection step. This compound can then, after condensation with the intermediate (VII) and optional deprotection, lead to the formation of the formula (I) compounds. Compound (VI) can also be coupled with a compound (VII') in which R'₃ is a precursor of R₃, in particular an R₃ group protected by a protective group. Coupling followed by deprotection of group R'₃ to lead to R₃ can be carried out following the same procedures as described previously.

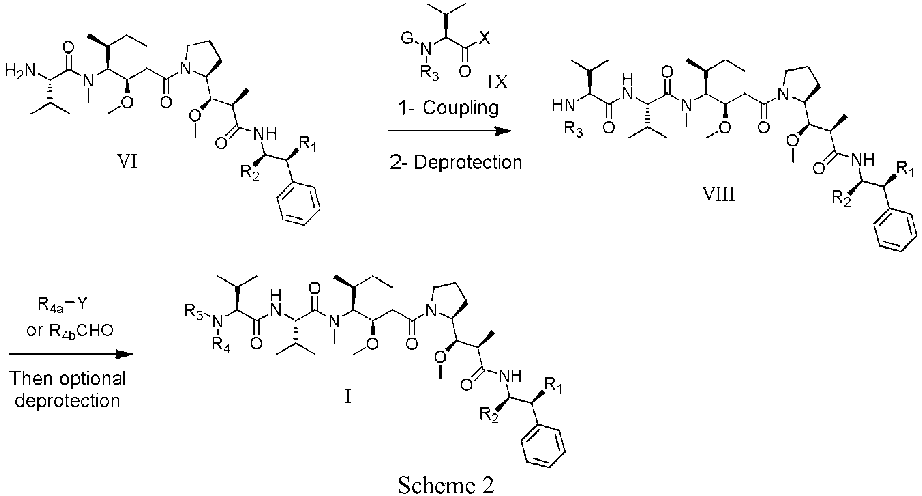

Scheme 2 illustrates the second general method which can be used to prepare formula (I) compounds. In the above general formulas R₁, R₂, and R₃ are such as previously defined, R₄ₐ represents an R₄ group such as previously defined optionally in protected form, R_{4b} is a precursor of an R₄ group and G is a protective group.

At the first step, compound (IX) protected on its amine function by a protective group G is condensed with compound (VI). X may represent a leaving group e.g. a chlorine. In this case, the first step consists of the reaction between an acid chloride and an amine. This reaction can be performed using methods and techniques well known to persons skilled in the art. In one particularly appreciated method the two entities are caused to react in the presence of an organic or inorganic base such as Et₃N, iPr₂NEt, pyridine, NaH, Cs₂CO₃, K₂CO₃ in a solvent such as THF, dichloromethane, DMF, DMSO at a temperature notably between -20° and 100°C. X may also represent a hydroxyl. In this case, the first step is a condensation reaction between the carboxylic acid (IX) and the amine (VI). This reaction can be conducted following methods and techniques well known to skilled persons. In one particularly appreciated method, the two entities are caused to react in the presence of 1-(3-dimethylaminopropyl)-3-ethylcarbodiimide (EDC), 3-hydroxy-1,2,3-benzotriazin-4(3H)-one, a tertiary amine such as diisopropylethylamine, in a polar aprotic solvent such as dichloromethane or DMF, at a temperature notably between -15°C and 40°C. In another particularly appreciated method, these two entities are caused to react in the presence of diethyl phosphorocyanidate (DEPC), a tertiary amine such as triethylamine, in a polar aprotic solvent such as dichloromethane or DMF, at a temperature notably between -15°C and 40°C.

After deprotection of the intermediate, using techniques well known to skilled persons, the obtained compound (VIII) can lead to the formula (I) compounds after reaction with R₄Y. In this case, Y is a leaving group such as Cl, Br, I, OSO₂CH₃, OSO₂CF₃ or O-Tosyl. The reaction is conducted in the presence of an organic or inorganic base such as Et₃N, iPr₂NEt, NaH, Cs₂CO₃, K₂CO₃, in a polar anhydrous solvent such as dichloromethane, THF, DMF, DMSO at a temperature notably between -20° and 100°C. In another particularly appreciated method, compound (VIII) is caused to react with an aldehyde of formula R_{4b}-CHO where R_{4b} corresponds to a precursor of R₄. In this case, the reaction is a reductive amination in the presence of a reducing agent such as NaBH₄, NaBH₃CN, NaBH(OAc)₃, in a polar solvent such as 1,2-dichloroethane, dichloromethane, THF, DMF, MeOH, in the optional presence of titanium isopropoxide (IV), at a pH which can be controlled by the addition of an acid such as acetic acid at a temperature notably between -20°C and 100°C.

In the foregoing synthesis schemes, a formula (I) compound may lead to another formula (I) compound after an additional reaction step such as saponification for example using methods well known to skilled persons whereby an R₂ group representing an ester, preferably a methyl ester, is changed to an R₂ group representing a carboxylic acid.

If it is desired to isolate a formula (I) compound containing at least one base function in the state of an acid addition salt, this is possible by treating the free base of the formula (I) compound (containing at least one base function) with a suitable acid, preferably in equivalent quantity. The suitable acid may in particular be trifluoroacetic acid.

A further subject of the present invention is therefore a first method for preparing a formula (I) compound, comprising a condensation reaction between a compound of following formula (VI): 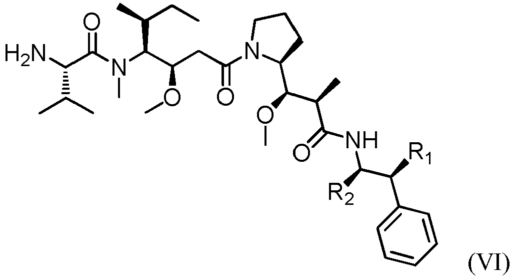
where R₁ and R₂ are such as defined previously, and
a compound of following formula (VII): 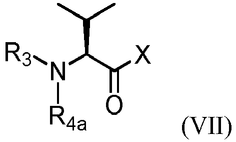
where R₃ is such as previously defined, R₄ₐ corresponds to a R₄ group such as previously defined optionally in protected form, and X is OH or Cl.

When X = OH, the coupling reaction can be performed under peptide coupling conditions well known to persons skilled in the art.

Said peptide coupling can be performed in the presence of a coupling agent such as diisopropylcarbodiimide (DIC), dicyclohexylcarbodiimide (DCC), 1-(3-dimethylaminopropyl)-3-ethylcarbodiimide hydrochloride (EDC), carbonyldiimidazole (CDI), 2-(1H-benzotriazole-1-yl)-1,1,3,3-tetramethyluronium hexafluorophosphate (HBTU), 2-(1H-benzotriazole-1-yl)-1,1,3,3-tetramethyluronium tetrafluoroborate (TBTU), O-(7-azobenzotriazol-1-yl)-1,1,3,3-tetramethyluronium hexafluorophosphate (HATU), diethyl phosphorocyanidate (DEPC) or (benzotriazol-1-yloxy) tripyrrolodinophosphonium hexafluorophosphate (PyBOP), optionally associated with a coupling auxiliary such as N-hydroxy succinimide (NHS), N-hydroxy benzotriazole (HOBt), 3,4-dihydro-3-hydroxy-4-oxo-1,2,3-benzotriazole (HOOBt), 1-hydroxy-7-azabenzotriazole (HAt), N-hydroxysylfosuccinimide (sulfo NHS) or dimethyl aminopyridine (DMAP). Preferably the coupling agent is HATU or DEPC.

The reaction can also be performed in the presence of a base such as DIEA (diisopropylethylamine).

In particular, the peptide coupling is performed in the presence of HATU or DEPC and DIEA.

Said reaction can be carried out in a polar aprotic solvent such as dichloromethane (DCM) or dimethylformamide (DMF), in particular at a temperature of between -15°C and 40°C.

When X = Cl, the condensation reaction will be conducted in the presence of a base which may be organic or inorganic, such as Et₃N, iPr₂NEt, pyridine, NaH, Cs₂CO₃, or K₂CO₃.

The reaction can be carried out in a solvent such as tetrahydrofuran (THF), dichloromethane (DCM), dimethylformamide (DMF), or dimethylsulfoxide (DMSO), in particular at a temperature of between -20° and 100°C.

The compounds of formulas (VI) and (VII) can be prepared following synthesis protocols described in the experimental part below or following techniques known to those skilled in the art.

A further subject of the present invention is a second method for preparing a formula (I) compound comprising a substitution reaction between a compound of following formula (VIII): 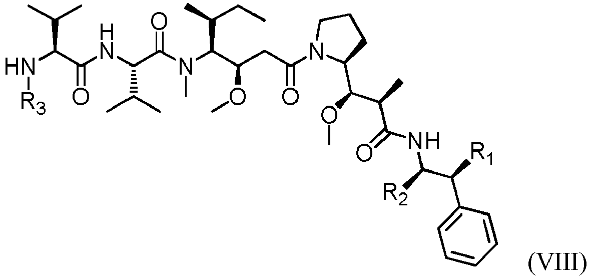
where R₁, R₂ an R₃ are such has previously defined, and
a compound of following formula (X):

   R₄ₐ-Y (X)
where R₄ₐ is an R₄ group such as previously defined optionally in protected form, and Y is a leaving group such as Cl, Br, I, OSO₂CH₃, OSO₂CF₃ or O-Tosyl.

The substitution reaction will be notably conducted in the presence of a base which may be organic or inorganic such as Et₃N, iPr₂NEt, NaH, Cs₂CO₃, or K₂CO₃.

This reaction can be implemented in a polar solvent, preferably anhydrous, such as DCM, THF, DMF or DMSO, in particular at a temperature of between -20° and 100°C.

The compounds of formulas (VIII) and (X) can be prepared following the synthesis protocols described in the experimental part below or using techniques known to those skilled in the art.

A further subject of the present invention is a third method for preparing a formula (I) compound in which R₄ is a -CH₂R_{4b} group with R_{4b} representing a phenyl or phenyl-(C₁)alkyl group substituted by one group (preferably on the phenyl moiety) chosen from among OH and NR₉R₁₀ groups,
comprising a reductive amination reaction between a compound of following formula (VIII): 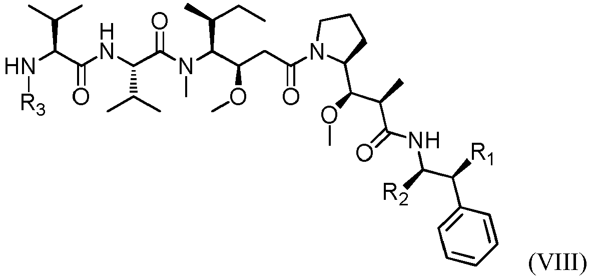
where R₁, R₂ and R₃ are such as previously defined, and
a compound of following formula (XI):

   R_{4b}-CHO (XI)
where R_{4b} is such as defined above.

The reductive amination reaction can be carried out in the presence of a reducing agent such as NaBH₄, NaBH₃CN or NaBH(OAc)₃ and optionally titanium isopropoxide (IV).

The pH can be controlled by adding an acid such as acetic acid, in particular to reach a pH of between 4 and 5.

This reaction can be implemented in a polar solvent such as DCE (1,2-dichloroethane), DCM, THF, DMF or methanol, in particular at a temperature of between-20° and 100°C.

The compounds of formulas (VIII) and (XI) can be prepared following synthesis protocols described in the experimental part below or using techniques known to those skilled in the art.

The compound obtained after the condensation/substitution/reductive amination step of one of the three above methods can be subjected to additional deprotection steps particularly concerning the substituents R₂ and R₄ and optionally additional functionalization steps using methods well known to skilled persons.

When R₂ represents a COOH group, the condensation/substitution/reductive amination step mentioned above can be performed from a compound of formula (VI) with an R₂ group representing a COO-((C₁-C₆)alkyl) ester function, this ester function then possibly being saponified to yield a formula (I) compound with R₂ = COOH.

When the R₄ group comprises a NH function, this can be protected before performing the condensation/substitution/reductive amination reaction by substituting the nitrogen atom by an N-protective group such as a Boc or Fmoc group.

By «protective group » in the present invention is meant a group which selectively blocks a reactive site in a multifunctional compound such that a chemical reaction can selectively be carried out at another non-protected reactive site in the meaning conventionally associated therewith in chemical synthesis.

By « N-protective group » in the present invention is meant any substituent which protects the NH group against undesirable reactions such as the N-protective groups described in Greene, « Protective Groups In Organic synthesis », (John Wiley & Sons, New York (1981)) and Harrison et al. « Compendium of Synthetic Organic Methods », Vols. 1 to 8 (J. Wiley & sons, 1971 to 1996). The N-protective groups comprise carbamates, amides, N-alkylated derivatives, amino acetal derivatives, N-benzyl derivatives, imine derivatives, enamine derivatives and N-heteroatom derivatives. The N-protecting groups can be formyl; an aryl, such as a phenyl, optionally substituted with one or several methoxy groups such as *p*-methoxyphenyl (PMP); an aryl-(C₁-C₆)alkyl, such as a benzyl, the aryl moiety being optionally substituted with one or several methoxy groups, such as benzyl (Bn), *p*-methoxybenzyl (PMB) or 3,4-dimethoxybenzyl (DMPM); -CO-R_{GP1} such as acetyl (Ac), pivaloyl (Piv or Pv), benzoyl (Bz) or *p*-methoxybenzylcarbonyl (Moz); -CO₂-R_{GP1} such as *t*butyloxycarbonyl (Boc), trichloroethoxycarbonyl (TROC), allyloxycarbonyl (Alloc), benzyloxycarbonyl (Cbz or Z) or 9-fluorenylmethyloxycarbonyl (Fmoc); -SO₂-R_{GP1} such as phenylsulfonyl, tosyl (Ts or Tos) or 2-nitrobenzenesulfonyl (also called nosyl - Nos or Ns); and the like, with R_{GP1} representing a (C₁-C₆)alkyl optionally substituted with one or several halogen atoms such as F or Cl; a (C₂-C₆)alkenyl such as an allyl; an aryl, such as a phenyl, optionally substituted with one or several groups chosen among OMe (methoxy) and NO₂ (nitro); an aryl-(C₁-C₆)alkyl, such as a benzyl, the aryl moiety being optionally substituted with one or several methoxy groups; or a 9-fluorenylmethyl group.

In particular, the N-protective group comprises formyl, acetyl, benzoyl, pivaloyl, phenylsulfonyl, benzyl (Bn), t-butyloxycarbonyl (Boc), benzyloxycarbonyl (Cbz), p-methoxybenzyloxycarbonyl, p-nitrobenzyl-oxycarbonyl, trichloroethoxy carbonyl (TROC), allyloxycarbonyle (Alloc), 9-fluorenylmethyloxycarbonyl (Fmoc), trifluoro-acetyl, benzyl carbamates (substituted or not) and similar. It may in particular be a Boc or Fmoc group.

The protection of the NH amine function by a Boc or Fmoc group and its subsequent deprotection, after the condensation/substitution/reductive amination reaction, are well known to persons skilled in the art and are described in particular in the experimental part below.

The formula (I) compound obtained with one of the three methods mentioned above can also be salified by adding a pharmaceutically acceptable base or acid, in particular a pharmaceutically acceptable acid such as trifluoroacetic acid. Said step can optionally be performed at the same time as another reaction step, in particular at the same time as a deprotection step when this must be performed in an acid medium for example.

The compound obtained with one of these three methods, optionally after additional step(s) for deprotection, functionalization and/or salification, can be separated from the reaction medium using methods well known to skilled persons, such as by extraction, solvent evaporation or by precipitation and filtration.

The compound may also be purified if necessary using techniques well known to skilled persons, e.g. by recrystallization if the compound is crystalline, by distillation, by silica gel column chromatography or high performance liquid chromatography (HPLC).

The following examples illustrate the invention.

### EXAMPLES

### I - Synthesis of the compounds of the invention

The following abbreviations are used in the following examples:
- aq.: Aqueous
- ee: enantiomeric excess
- equiv: equivalent
- ESI: Electrospray ionisation
- LC/MS: Liquid Chromatography coupled with Mass Spectrometry
- HPLC: High Performance Liquid Chromatography
- NMR: Nuclear Magnetic Resonance
- sat.: saturated
- UV: ultraviolet

### Reference Example 1

### (S)-2-((S)-2-((3-aminopropyl)(methyl)amino)-3-methylbutanamido)-N-((3R,4S,5S)-3-methoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)-N,3-dimethylbutanamide, bis trifluoroacetic acid

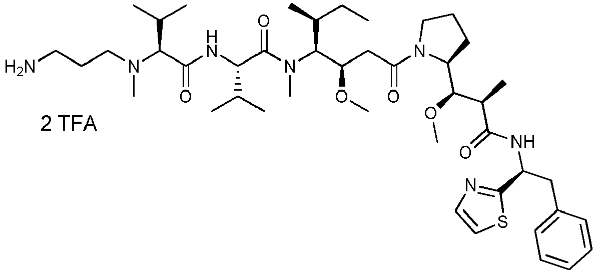

### Example 1A: (4R, 5S)-4-methyl-5-phenyl-3-propanoyl-1,3-oxazolidin-2-one

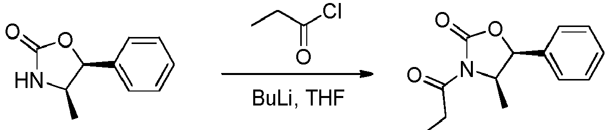

(4R, 5S)-4-methyl-5-phenyl-1,3-oxazolidin-2-one (5.8 g, 32.7 mmol, 1.00 equiv) was dissolved in tetrahydrofuran (THF, 120 mL) in an inert atmosphere. The mixture was cooled to -78°C and *n*-butyllithium (14.4 mL) was added drop-wise. After agitation for 30 minutes at -78°C, propanoyl chloride (5.7 mL) was added. Agitation was continued for 30 minutes at -78°C then overnight at ambient temperature. The reaction mixture was concentrated then re-dissolved in 200 mL of water. The pH of the solution was adjusted to 7 with sodium bicarbonate saturated aqueous solution. This aqueous phase was extracted 3 times with 100 mL of ethyl acetate (EtOAc). The organic phases were combined, dried over sodium sulfate, filtered and concentrated to yield 6.8 g (89 %) of compound **1A** in the form of a yellow oil.

### Example 1B: tert-butyl (2S)-2-[(1R,2R)-1-hydroxy-2-methyl-3-[(4R,5S)-4-methyl-2-oxo-5-phenyl-1,3-oxazolidin-3yl]-3-oxopropyl]pyrrolidine-1-carboxylate

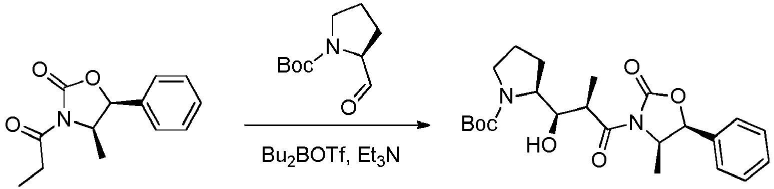

Compound **1A** (17.6 g, 75.45 mmol, 1.00 equiv) was dissolved in dichloromethane (DCM, 286 mL) in an inert atmosphere. This solution was cooled with an ice bath. Triethylamine (TEA, 12.1 mL, 1.15 equiv) and Bu₂BOTf (78.3 mL, 1.04 equiv) were added drop-wise whilst holding the temperature of the reaction mixture below 2°C. Agitation was continued at 0°C for 45 minutes, after which the reaction was cooled to -78°C. A solution of *tert*-butyl (2S)-2-formylpyrrolidine-1-carboxylate (8.5 g, 42.66 mmol, 0.57 equiv) in DCM (42 mL) was added drop-wise. Agitation was continued for 2 hours at -78°C, then for 1 hour at 0°C and finally 1 hour at ambient temperature. The reaction was neutralised with 72 mL of phosphate buffer (pH = 7.2 - 7.4) and 214 mL methanol, and cooled to 0°C. A solution of 30 % hydrogen peroxide in methanol (257 mL) was added drop-wise whilst maintaining the temperature below 10°C. Agitation was continued for 1 hour at 0°C. The reaction was neutralised with 142 mL of water, then concentrated under reduced pressure. The resulting aqueous solution was extracted 3 times with 200 mL EtOAc. The organic phases were combined, dried over sodium sulfate, filtered and concentrated. The residue was purified on a silica column with a mixture of EtOAc and petroleum ether (EtOAc:PE = 1:8) to yield 13.16 g (40 %) of compound **1B** in the form of a colourless oil.

### Example 1C: (2R,3R)-3-[(2S)-1-[(tert-butoxy)carbonyl]pyrrolidin-2-yl]-3-hydroxy-2-methylpropanoic acid

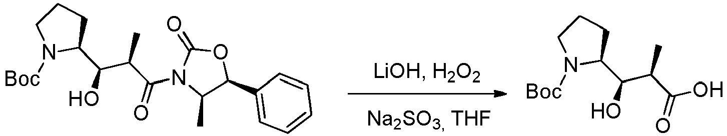

Compound **1B** (13.16 g, 30.43 mmol, 1.00 equiv) was dissolved in THF (460 mL) in the presence of hydrogen peroxide (30 % in water, 15.7 mL), then cooled with an ice bath. An aqueous solution of lithium hydroxide (0.4 mol/L, 152.1 mL) was added drop-wise whilst holding the reaction temperature below 4°C. The reaction mixture was agitated 2.5 hours at 0°C. An aqueous solution of Na₂SO₃ (1 mol/L, 167.3 mL) was added drop-wise whist holding the temperature at 0°C. The reaction mixture was agitated 14 hours at ambient temperature, then neutralised with 150 mL of cold sodium bicarbonate saturated solution and washed 3 times with 50 mL of DCM. The pH of the aqueous solution was adjusted to 2-3 with a 1M aqueous solution of KHSO₄. This aqueous solution was extracted 3 times with 100 mL of EtOAc. The organic phases were combined, washed once with saturated NaCl solution, dried over sodium sulfate, filtered and concentrated to yield 7.31 g (88 %) of compound **1C** in the form of a colourless oil.

### Example 1D: (2R,3R)-3-[(2S)-1-[(tert-butoxy)carbonyl]pyrrolidin-2-yl]-3-methoxy-2-methylpropanoic acid

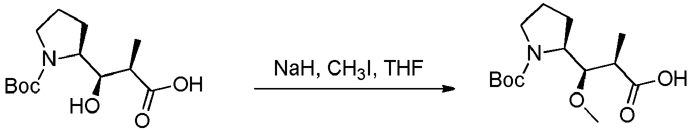

Compound **1C** (7.31 g, 26.74 mmol, 1.00 equiv) was dissolved in an inert atmosphere in THF (135 mL) in the presence of iodomethane (25.3 mL). The reaction medium was cooled with an ice bath after which NaH (60 % in oil, 4.28 g) was added in portions. The reaction was left under agitation 3 days at 0°C and then neutralised with 100 mL of sodium bicarbonate saturated aqueous solution and washed 3 times with 50 mL ether. The pH of the aqueous solution was adjusted to 3 with 1M aqueous KHSO₄ solution. This aqueous solution was extracted 3 times with 100 mL of EtOAc. The organic phases were combined, washed once with 100 mL of Na₂S₂O₃ (5 % in water), once with NaCl-saturated solution, then dried over sodium sulfate, filtered and concentrated to yield 5.5 g (72 %) of compound **1D** in the form of a colourless oil.

### Example 1E: N-methoxy-N-methyl-2-phenylacetamide

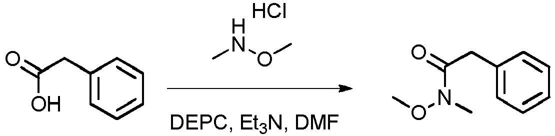

2-phenylacetic acid (16.2 g, 118.99 mmol, 1.00 equiv) was dissolved in dimethylformamide (DMF, 130 mL) then cooled to -10°C. Diethyl phosphorocyanidate (DEPC, 19.2 mL), methoxy(methyl)amine hydrochloride (12.92 g, 133.20 mmol, 1.12 equiv) and triethylamine (33.6 mL) were added. The reaction mixture was agitated 30 minutes at -10°C then 2.5 hours at ambient temperature. It was then extracted twice with 1 litre of EtOAc. The organic phases were combined, washed twice with 500 mL of NaHCO₃ (sat.), once with 400 mL of water, then dried over sodium sulfate, filtered and concentrated. The residue was purified on a silica column with an EtOAc and PE mixture (1:100 to 1:3) to yield 20.2 g (95 %) of compound **1E** in the form of a yellow oil.

### Example 1F: 2-phenyl-1-(1,3-thiazol-2-yl)ethan-1-one

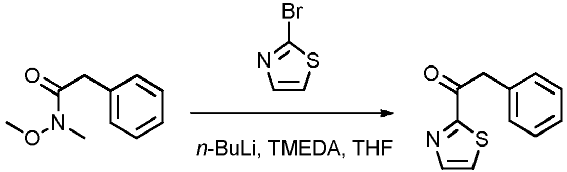

Tetramethylethylenediamine (TMEDA, 27.2 mL) was dissolved in THF 300 mL) in an inert atmosphere, then cooled to -78°C before the drop-wise addition of *n*BuLi (67.6 mL, 2.5 M). 2-bromo-1,3-thiazole (15.2 mL) was added drop-wise and agitation was continued 30 minutes at -78°C. Compound **1E** (25 g, 139.50 mmol, 1.00 equiv) dissolved in THF (100 mL) was added drop-wise. Agitation was continued for 30 minutes at -78°C then 2 hours at -10°C. The reaction was neutralised with 500 mL of KHSO₄ (sat.), then extracted 3 times with 1 litre of EtOAc. The organic phases were combined, washed twice with 400 mL water and twice with 700 mL of NaCl (sat.), then dried over sodium sulfate, filtered and concentrated. The residue was purified on a silica column with a mixture of EtOAc and PE (1:100 to 1:10) to yield 25 g (88 %) of compound **1F** in the form of a yellow oil.

### Example 1G: (1R)-2-phenyl-1-(1,3-thiazol-2-yl)ethan-1-ol

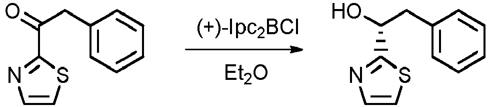

In an inert atmosphere, a solution of compound **1F** (15 g, 73.8 mmol, 1.00 equiv.) in ether (300 mL) was added drop-wise to (+)-B-chloro diisopinocampheylborane ((+)-Ipc₂BCl, 110.8 mL). The reaction mixture was agitated 24 hours at 0°C, then neutralised with 300 mL of a (1:1) mixture of NaOH (10 % in water) and H₂O₂ (30 % in water), and finally extracted three times with 500 mL of EtOAc. The organic phases were combined, washed twice with 300 mL of K₂CO₃ (sat.) and once with 500 mL of NaCl (sat.), then dried over sodium sulfate, filtered and concentrated. The residue was purified on a silica column with a mixture of EtOAc and PE (1:20 to 1:2) to yield 6.3 g (42 %) of compound **1G** in the form of a white solid.

### Example 1H: 2-[(1S)-1-azido-2-phenylethyl]-1,3-thiazole

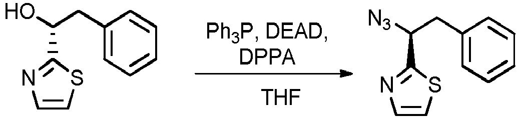

Compound **1G** (6 g, 29.23 mmol, 1.00 equiv.) was dissolved in an inert atmosphere in THF (150 mL) in the presence of triphenylphosphine (13 g, 49.56 mmol, 1.70 equiv.), then cooled to 0°C. Diethylazodicarboxylate (DEAD, 7.6 mL) was added drop-wise, followed by diphenylphosphorylazide (DPPA, 11 mL), the cold bath was then removed and the solution was left under agitation 48 hours at ambient temperature. The medium was concentrated under reduced pressure. The residue was purified on a silica column with a mixture of EtOAc and PE (1:100 to 1:30) to yield 8 g of partly purified compound **1H** in the form of a yellow oil. Compound **1H** was used as such in the following step.

### Example 1I: tert-butyl N-[(1S)-2-phenyl-1-(1,3-thiazol-2-yl)ethyl] carbamate.

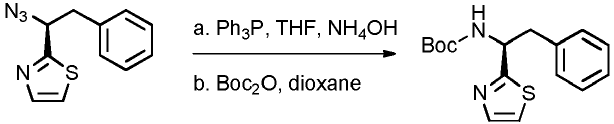

Compound **1H** (6.5 g, 28.2 mmol, 1.00 equiv) was dissolved in an inert atmosphere in THF (100 mL) in the presence of triphenylphosphine (6.5 g, 33.9 mmol, 1.20 equiv.), and heated to 50°C for 2 hours. Ammonia (70 mL) was then added and heating was continued for 3 hours. The reaction was cooled, neutralised with 500 mL water, then extracted 3 times with 500 mL of EtOAc. The organic phases were combined and extracted twice with 500 mL of 1N HCl. The aqueous phases were combined, brought to pH 8-9 by adding a sodium hydroxide solution (10 % in water), then extracted 3 times with 500 mL of DCM. The organic phases were combined, dried over sodium sulfate, filtered and concentrated to yield 4.8 g (83 %) of (1S)-2-phenyl-1-(1,3-thiazol-2-yl)ethan-1-amine in the form of a yellow oil. This compound was then protected with a Boc group ((*tert*-butoxy)carbonyl) so that it could be purified. It was dissolved in an inert atmosphere in 1,4-dioxane (40 mL), then cooled to 0°C. (Boc)₂O (10.26 g, 47.01 mmol, 2.00 equiv) diluted in 20 mL of 1,4-dioxane was added drop-wise. The cold bath was removed and the solution left under agitation overnight at ambient temperature before being neutralised with 300 mL of water and extracted twice with 500 mL of EtOAc. The organic phases were combined, dried over sodium sulfate, filtered and concentrated. The residue was purified on a silica column with a mixture of EtOAc and PE (1:100 to 1:20, ee = 93 %). It was then recrystallized in a hexane/acetone mixture (∼ 5-10 / 1, 1g / 10 mL) to yield 6 g (84 %) of compound **1I** in the form of a white solid (ee > 99 %).

### Example 1J: tert-butyl (2S)-2-[(1R,2R)-1-methoxy-2-methyl-2-[[(1S)-2-phenyl-1-((1,3-thiazol-2-yl)ethyl]carbamoyl]ethyl]pyrrolidine-1-carboxylate

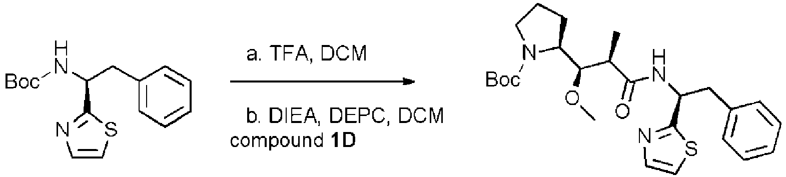

Compound **1I** (3 g, 9.86 mmol, 1.00 equiv) was dissolved in an inert atmosphere in 10 mL DCM. Trifluoroacetic acid (TFA, 10 mL) was added and the solution left under agitation overnight at ambient temperature, then concentrated under reduced pressure to yield 2.0 g (64 %) of (1S)-2-phenyl-1-(1,3-thiazol-2-yl)ethan-1-amine; trifluoroacetic acid in the form of a yellow oil. This intermediate was re-dissolved in 20 mL of DCM after which compound **1D** (1.8 g, 6.26 mmol, 1.05 equiv), DEPC (1.1 g, 6.75 mmol, 1.13 equiv) and diisopropylethylamine (DIEA, 1.64 g, 12.71 mmol, 2.13 equiv) were added. The reaction mixture was left under agitation overnight at ambient temperature, then concentrated under reduced pressure. The residue was purified on a silica column with a mixture of EtOAc and PE (1:100 to 1:3) to yield 2.3 g (81 %) of compound **1J** in the form of a pale yellow solid.

### Example 1K: (2R,3R)-3-methoxy-2-methyl-N-[(1S)-2-phenyl-1-(1,3-thiazol-2-yl)ethyl]-3-[(2S)-pyrrolidin-2-yl]propanamide; trifluoroacetic acid

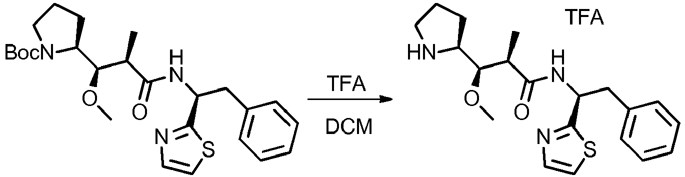

Compound **1J** (2.25 g, 4.75 mmol, 1.00 equiv) was dissolved in an inert atmosphere in 10 mL of DCM. TFA (10 mL) was added and the solution left under agitation overnight at ambient temperature, then concentrated under reduced pressure to yield 2.18 g (94 %) of compound **1K** in the form of a yellow oil.

### Example 1L: (2S,3S)-2-(benzylamino)-3-methylpentanoic acid

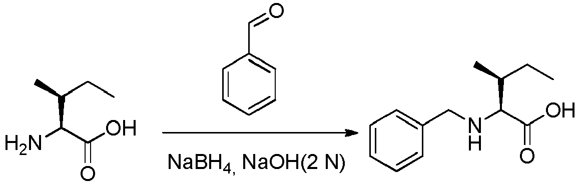

(2S,3S)-2-amino-3-methylpentanoic acid (98.4 g, 750 mmol, 1.00 equiv) was added at ambient temperature and in portions to a 2N sodium hydroxide solution (375 mL). Benzaldehyde (79.7 g, 751.02 mmol, 1.00 equiv) was quickly added and the resulting solution was agitated 30 minutes. Sodium borohydride (10.9 g, 288.17 mmol, 0.38 equiv) was added in small portions, whilst holding the temperature at between 5 and 15°C. Agitation was continued for 4 hours at ambient temperature. The reaction mixture was diluted with 200 mL of water, then washed twice with 200 mL of EtOAc. The pH of the aqueous solution was adjusted to 7 with a 2N hydrochloric acid solution. The formed precipitate was collected by filtering and gave 149.2 g (90 %) of compound **1L** in the form of a white solid.

### Example 1M: (2S,3S)-2-[benzyl(methyl)amino]-3-methylpentanoic acid

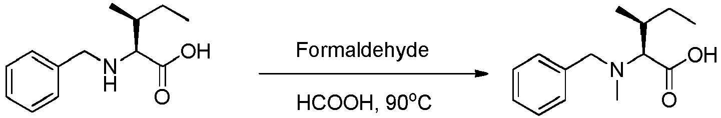

Compound **1L** (25 g, 112.97 mmol, 1.00 equiv) was dissolved in an inert atmosphere in formic acid (31.2 g) in the presence of formaldehyde (36.5 % in water, 22.3 g). The solution was agitated 3 hours at 90°C then concentrated under reduced pressure. The residue was triturated in 250 mL of acetone, then concentrated. This trituration/evaporation operation was repeated twice with 500 mL of acetone to yield 21.6 g (81 %) of compound **1M** in the form of a white solid.

### Example 1N: (2S,3S)-2-[benzyl(methyl)amino]-3-methylpentan-1-ol

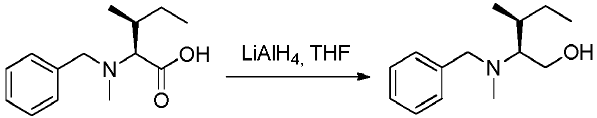

LiAlH₄ (0.36 g) was suspended in 10 mL of THF in an inert atmosphere at 0°C. Compound **1M** (1.5 g, 6.37 mmol, 1.00 equiv) was added in small portions whilst holding the temperature at between 0 and 10°C. The reaction mixture was agitated 2 hours at 65°C, then again cooled to 0°C before being neutralised with successive additions of 360 µL of water, 1 mL of 15 % sodium hydroxide and 360 µL of water. The aluminium salts which precipitated were removed by filtering. The filtrate was dried over sodium sulfate, filtered and concentrated. The residue was purified on a silica column with a mixture of EtOAc and PE (1:50) to yield 820 mg (58 %) of compound **1N** in the form of a pale yellow oil.

### Example 1O: (2S,3S)-2-[benzyl(methyl)amino]-3-methylpentanal

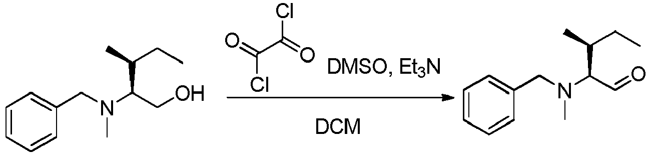

Oxalyl chloride (0.4 mL) was dissolved in DCM (15 mL) in an inert atmosphere. The solution was cooled to -70°C and a solution of dimethylsulfoxide (DMSO (0.5 mL) in DCM (10 mL) was added drop-wise for 15 minutes. The reaction mixture was agitated 30 minutes after which a solution of compound **1N** (820 mg, 3.70 mmol, 1.00 equiv) in DCM (10 mL) was added drop-wise for 15 minutes. The reaction mixture was agitated a further 30 minutes at low temperature, then triethylamine (2.5 mL) was slowly added. The reaction mixture was agitated 1 hour at -50°C, the cold bath was then removed and the reaction neutralised with 25 mL of water whilst allowing the temperature to return to normal. The solution was washed once with 30 mL of NaCl-saturated aqueous solution, then dried over sodium sulfate, filtered and concentrated. The residue was purified on a silica column with a mixture of EtOAc and PE (1:200) to yield 0.42 g (52 %) of compound **1O** in the form of a yellow oil.

### Example 1P: (2S,3S)-N-benzyl-1,1-dimethoxy-N,3-dimethylpentan-2-amine

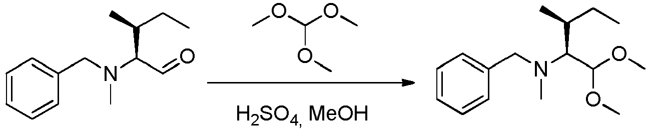

Compound **1O** (4.7 g, 21.43 mmol, 1.00 equiv) was dissolved in 20 mL of methanol at 0°C. Concentrated sulfuric acid (4.3 mL) was added drop-wise and agitation was continued for 30 minutes at 0°C. Trimethyl orthoformate (21.4 mL) was added, the cold bath removed and the reaction medium left under agitation for 3 hours at ambient temperature. The reaction medium was diluted with 200 mL of EtOAc, successively washed with 100 mL of 10 % Na₂CO₃ and 200 mL of saturated NaCl, then dried over sodium sulfate, filtered and concentrated under reduced pressure to yield 3.4 g (60 %) of compound **1P** in the form of a pale yellow oil.

### Example 1Q: [[1-(tert-butoxy)ethenyl]oxy](tert-butyl)dimethylsilane

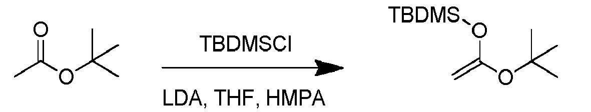

Diisopropylamine (20 g, 186.71 m mol, 1.08 equiv) was dissolved in 170 mL of THF in an inert atmosphere and cooled to -78°C. nBuLi (2.4 M, 78.8 mL) was added drop-wise and the solution agitated 30 minutes at low temperature (to give LDA-lithium diisopropylamide) before adding *tert*-butyl acetate (20 g, 172.18 mmol, 1.00 equiv). The reaction mixture was agitated 20 minutes at -78°C before adding hexamethylphosphoramide (HMPA, 25.8 mL) and a solution of *tert*butyldimethylchlorosilane (TBDMSCl, 28 g, 185.80 mmol, 1.08 equiv) in 35 mL of THF. Agitation was continued for 20 additional minutes at low temperature, and the cold bath was then removed. The solution was concentrated under reduced pressure. The residue was re-dissolved in 100 mL of water and extracted 3 times with 100 mL of PE. The organic phases were combined, washed once with 500 mL of NaCl-saturated aqueous solution, dried over sodium sulfate, filtered and concentrated. The residue was purified by distillation to yield 16.6 g (83 %) of compound **1Q** in the form of a colourless oil.

### Example 1R: tert-butyl (3R,4S,5S)-4-[benzyl(methyl)amino]-3-methoxy-5-methyl heptanoate

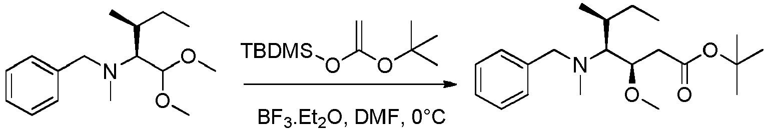

Compound **1P** (2.0 g, 7.54 mmol, 1.00 equiv) and compound **1Q** (2.6 g, 11.28 mmol, 1.50 equiv) were dissolved in 33 mL of DCM in an inert atmosphere. The solution was cooled to 0°C. DMF (1.2 g) was added drop-wise together with a solution of BF₃-Et₂O (2.1 g) in 7.5 mL of DCM. Agitation was continued for 24 hours at 0°C. The reaction medium was washed once with 30 mL of sodium carbonate (10 %) and twice with 50 mL of NaCl-saturated aqueous solution, then dried over sodium sulfate, filtered and concentrated. The residue was purified on a silica column with a mixture of EtOAc and PE (1:100) to yield 1.82 g (91 %) of compound **1R** in the form of a yellow oil.

### Example 1S: (3R,4S,5S)-3-methoxy-5-methyl-4-(methylamino)heptanoate hydrochloride

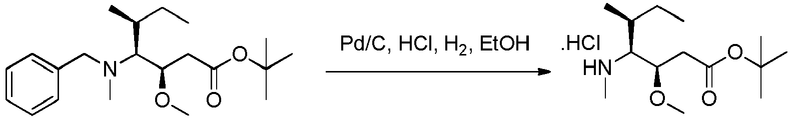

Compound **1R** (2.4 g, 6.87 mmol, 1.00 equiv) was dissolved in an inert atmosphere in 35 mL of ethanol in the presence of Pd/C (0.12 g) and concentrated hydrochloric acid (0.63 mL). The nitrogen atmosphere was replaced by a hydrogen atmosphere and the reaction medium was left under agitation 18 hours at ambient temperature. The reaction medium was filtered and concentrated under reduced pressure. The residue was triturated in 50 mL of hexane and the supernatant removed which, after drying under reduced pressure, gave 1.66 g (82 %) of compound **1S** in the form of a white solid.

### Example 1T: tert-butyl (3R,4S,5S)-4-[(2S)-2-[[(benzyloxy)carbonyl]amino]-N,3-dimethylbutanamido]-3-methoxy-5-methylheptanoate

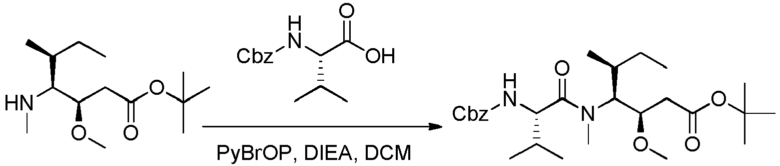

(2S)-2-[[(benzyloxy)carbonyl]amino]-3-methylbutanoic acid (15 g, 0.40 mmol, 1.00 equiv) was dissolved in 300 mL of DCM in the presence of DIEA (38.3 mL) and bromotripyrrolidinophosphonium hexafluorophosphate (PyBrOP, 32.3g). The solution was agitated 30 minutes at ambient temperature before adding compound **1S** (15.99g, 0.42 mmol, 1.07 equiv). The reaction medium was agitated 2 hours and then concentrated. The residue was purified in reverse phase (C18) with a mixture of acetonitrile (ACN) and water (30:70 to 100:0 in 40 minutes) to yield 17 g (58 %) of compound **1T** in the form of a colourless oil.

### Example 1U: tert-butyl (3R,4S,5S)-4-[(2S)-2-amino-N,3-dimethylbutanamido]-3-methoxy-5-methylheptanoate

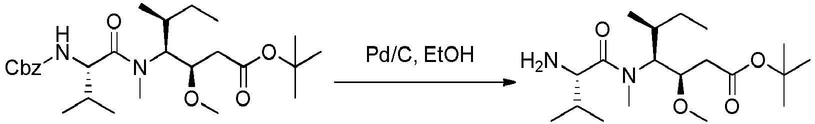

Compound **1T** (76 mg, 0.15 mmol, 1.00 equiv) was dissolved in an inert atmosphere in 10 mL of ethanol in the presence of Pd/C (0.05 g). The nitrogen atmosphere was replaced by a hydrogen atmosphere and the reaction agitated 2 hours at ambient temperature. The reaction medium was filtered and concentrated under reduced pressure to yield 64 mg of compound **1U** in the form of a colourless oil.

### Example 1V: (3R,4S,5S)-4-[(2S)-2-[[(9H-fluoren-9-ylmethoxy)carbonyl] amino]-N,3-dimethylbutanamido]-3-methoxy-5-methylheptanoate

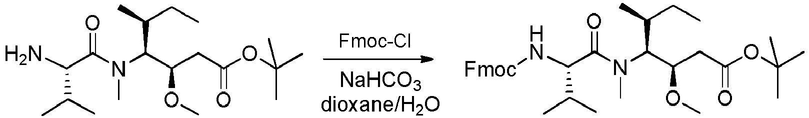

Compound **1U** (18.19 g, 50.74 mmol, 1.00 equiv) was dissolved in 400 mL of a 1,4-dioxane/water mixture (1:1) in the presence of sodium bicarbonate (12.78 g, 152 mmol, 3.00 equiv) and 9H-fluoren-9-ylmethyl chloroformate (Fmoc-Cl, 19.69 g, 76 mmol, 1.50 equiv), then agitated 2 hours at ambient temperature. The reaction medium was then diluted with 500 mL of water and extracted 3 times with 200 mL of EtOAc. The organic phases were combined, washed once with 200 mL of NaCl-saturated aqueous solution, dried over sodium sulfate, filtered and concentrated to yield 40 g of partly purified compound **1V** in the form of a pale yellow oil.

### Example 1W: (3R,4S,5S)-4-[(2S)-2-[[(9H-fluoren-9-ylmethoxy)carbonyl] amino]-N,3-dimethylbutanamido]-3-methoxy-5-methylheptanoic acid

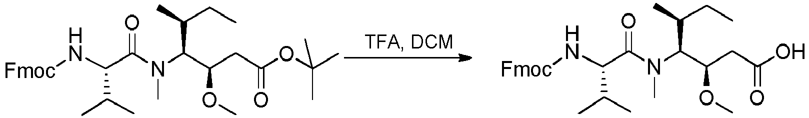

Compound **1V** (40 g, 68.88 mmol, 1.00 equiv) was dissolved in a neutral atmosphere in 600 mL of DCM. TFA (300 mL) was added. The solution was agitated 2 hours at ambient temperature, then concentrated under reduced pressure. The residue was purified on a silica column with a mixture of methanol and DCM (1:10) to yield 23.6 g (65 %) of compound **1W** in colourless oil form.

### Example 1X: 9H-fluoren-9-ylmethyl N-[(1S)-1-[[(3R,4S,5S)-3-methoxy-1-[(2S)-2-[(1R,2R)-1-methoxy-2-methyl-2-[[(1S)-2-phenyl-1-(1,3-thiazol-2-yl)ethyl]carbamoyl]ethyl]pyrrolidin-1-yl]-5-methyl-1-oxoheptan-4-yl](methyl) carbamoyl]-2-methylpropyl]carbamate

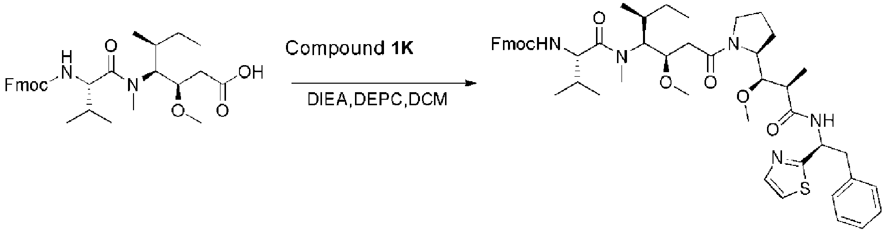

Compound **1W** (2.53 g, 4.82 mmol, 1.08 equiv) was dissolved in 20 mL of DCM in the presence of compound **1K** (2.18 g, 4.47 mmol, 1.00 equiv), DEPC (875 mg, 5.37 mmol, 1.20 equiv) and DIEA (1.25 g, 9.67 mmol, 2.16 equiv). The reaction mixture was left under agitation overnight at ambient temperature, then successively washed with 50 mL of saturated KHSO₄ and 100 mL of water, dried over sodium sulfate, filtered and concentrated. The residue was purified on a silica column with a mixture of methanol and DCM (1:200 to 1:40) to yield 2.8 g (71 %) of compound **1X** in the form of a pale yellow solid.

### Example 1Y: (2S)-2-amino-N-[(3R,5S)-3-methoxy-1-[(2S)-2-[(1R,2R)-1-methoxy-2-methyl-2-[[(1S)-2-phenyl-1-(1,3-thiazol-2-yl)ethyl] carbamoyl] ethyl] pyrrolidin-1-yl]-5-methyl-1-oxoheptan-4-yl]-N,3-dimethylbutanamide

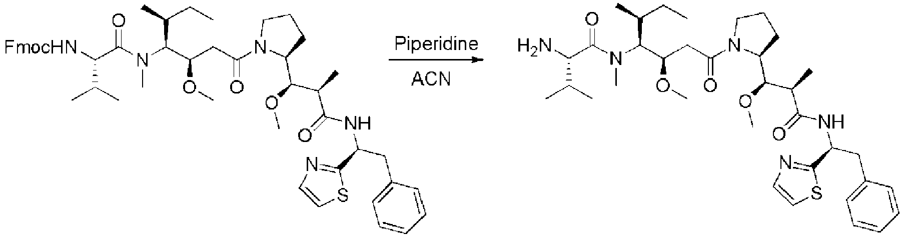

Compound **1X** (2.8 g, 3.18 mmol, 1.00 equiv) was dissolved in acetonitrile (ACN, 12 mL) in the presence of piperidine (3 mL) and left under agitation 18 hours at ambient temperature. The reaction was neutralised with 50 mL of water, then extracted twice with 100 mL of DCM. The organic phases were combined, dried over sodium sulfate, filtered and concentrated. The residue was purified on a silica column with a mixture of methanol and DCM (1:100 to 1:40) to yield 1.2 g (57 %) of compound **1Y** in the form of a yellow solid.

### Example 1ZA: (2S)-2-[[(tert-butoxy)carbonyl](methyl)amino]-3-methyl butanoic acid

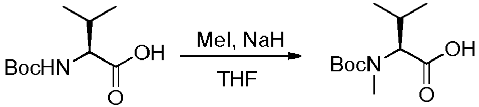

(2S)-2-[[(*tert*-butoxy)carbonyl]amino]-3-methylbutanoic acid (63 g, 289.97 mmol, 1.00 equiv) was dissolved in an inert atmosphere in THF (1000 mL) in the presence of iodomethane (181 mL). The solution was cooled to 0°C before adding sodium hydride (116 g, 4.83 mol, 16.67 equiv) in small portions. The reaction mixture was agitated for 1.5 hours at 0°C, the cold bath was then removed and agitation continued for 18 hours. The reaction was neutralised with 200 mL of water and then concentrated under reduced pressure. The residual aqueous phase was diluted with 4 litres of water, washed once with 200 mL of EtOAc and its pH adjusted to between 3 and 4 with a 1N solution of hydrochloric acid. The mixture obtained was extracted 3 times with 1.2 L of EtOAc. The organic phases were combined, dried over sodium sulfate, filtered and concentrated to yield 60 g (89 %) of compound **1ZA** in the form of a yellow oil.

### Example 1ZB: benzyl (2S)-2-[[(tert-butoxy)carbonyl](methyl)amino]-3-methyl butanoate

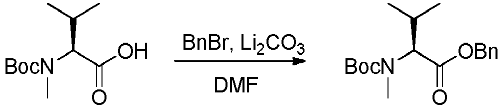

Compound **1ZA** (47 g, 203.21 mmol, 1.00 equiv) was dissolved in DMF (600 mL) in the presence of Li₂CO₃ (15.8 g, 213.83 mmol, 1.05 equiv). The solution was cooled to 0°C then benzyl bromide (BnBr 57.9 g, 338.53 mmol, 1.67 equiv) was added drop-wise. The reaction mixture was left under agitation overnight before being neutralised with 400 mL of water and filtered. The solution obtained was extracted twice with 500 mL of EtOAc. The organic phases were combined, dried over sodium sulfate, filtered and concentrated. The residue was purified on a silica column with a mixture of EtOAc and PE (1:100 to 1:20) to yield 22.5 g (34 %) of compound **1ZB** in the form of a yellow oil.

### Example 1ZC: benzyl (2S)-3-methyl-2-(methylamino)butanoate hydrochloride

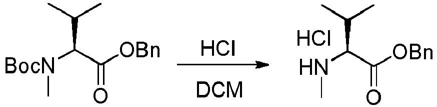

Compound **1ZB** (22.5 g, 70.00 mmol, 1.00 equiv) was dissolved in 150 mL of DCM. Gaseous hydrochloric acid was bubbled. The reaction was agitated 1 hour at ambient temperature and then concentrated under reduced pressure to yield 17 g (94 %) of compound 1ZC in the form of a yellow solid.

### Example 1ZD: tert-butyl N-(3,3-diethoxypropyl)carbamate

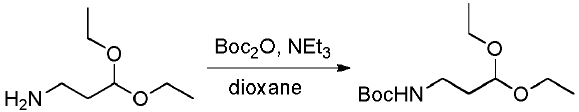

3,3-diethoxypropan-1-amine (6 g, 40.76 mmol, 1.00 equiv) was dissolved in 1,4-dioxane (30 mL) in the presence of TEA (4.45 g, 43.98 mmol, 1.08 equiv), then cooled to 0°C. (Boc)₂O (9.6 g, 43.99 mmol, 1.08 equiv) diluted in 20 mL of 1,4-dioxane was added drop-wise. The solution was agitated 2 hours at 0°C then overnight at ambient temperature before being neutralised with 10 mL of water. The pH was adjusted to 5 with HCl (1 %). The solution was extracted 3 times with 50 mL of EtOAc. The organic phases were combined, dried over sodium sulfate, filtered and concentrated to yield 8.21 g (81 %) of compound **1ZD** in the form of a pale yellow oil.

### Example 1Z: tert-butyl N-(3-oxopropyl) carbamate

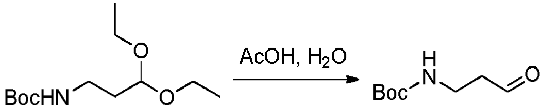

Compound **1ZD** (8.20 g, 33.15 mmol, 1.00 equiv) was dissolved in 18.75 mL of acetic acid and left under agitation overnight at ambient temperature. The reaction medium was then extracted 3 times with 30 mL of EtOAc. The organic phases were combined, washed 3 times with 30 mL of saturated NaCl solution, dried over sodium sulfate, filtered and concentrated to yield 5 g (87 %) of compound **1ZE** in the form of a dark red oil.

### Example 1ZF: (2S)-2-[(3-[[(tert-butoxy)carbonyl]amino]propyl)(methyl) amino]-3-methylbutanoic acid

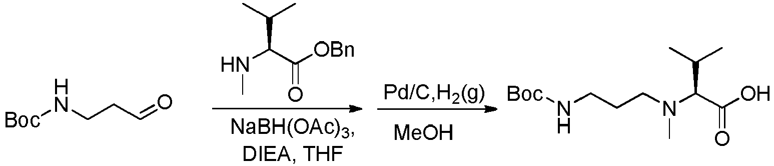

Compound **1ZE** (2.4 g, 13.86 mmol, 1.00 equiv) was dissolved in 50 mL of THF in the presence of compound **1ZC** (3.56 g, 13.81 mmol, 1.00 equiv) and DIEA (9.16 mL, 4.00 equiv). The reaction mixture was agitated 30 minutes at ambient temperature before adding sodium triacetoxyborohydride (5.87 g, 27.70 mmol, 2.00 equiv). Agitation was continued overnight, then the reaction was neutralised with 100 mL of water and extracted 3 times with 50 mL of EtOAc. The organic phases were combined, dried over sodium sulfate, filtered and concentrated. The residue was partly purified on a silica column with a mixture of EtOAc and PE (1:4). The crude product obtained was re-dissolved in 20 mL of methanol in the presence of Pd/C (1.2 g) and hydrogenated for 20 minutes at normal temperature and pressure. The reaction medium was filtered and concentrated under reduced pressure to yield 200 mg (5 %) of compound **1ZF** in the form of a white solid.

### Example 1ZG: tert-butyl N-(3-[[(1S)-1-[[(1S)-1-[[(3R,4S,5S)-3-methoxy-1-[(2S)-2-[(1R,2R)-1-methoxy-2-methyl-2-[[(1S)-2-phenyl-1-(1,3-thiazol-2-yl)ethyl] carbamoyl]thyl]pyrrolidin-1-yl]-5-methyl-1-oxoheptan-4yl](methyl) carbamoyl]-2-methylpropyl]carbamoyl]-2-methylpropyl](methyl)amino]propyl) carbamate

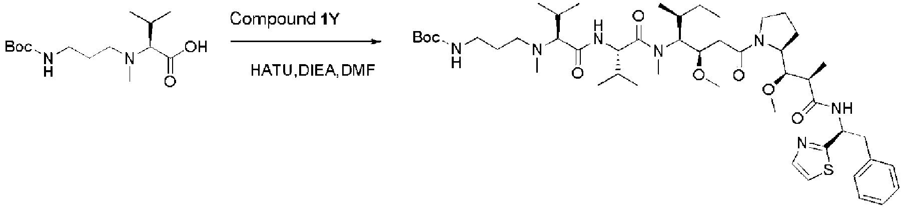

Compound **1Y** (50 mg, 0.08 mmol, 1.00 equiv) was dissolved in 2 mL of DMF in the presence of compound **1ZF** (26.2 mg, 0.09 mmol, 1.20 equiv), DIEA (37.7 mL) and O-(7-azabenzotriazol-1-yl)-*N,N,N',N*'-tetramethyluronium hexafluorophosphate (HATU, 43.3 mg, 0.11 mmol, 1.50 equiv). The reaction was left under agitation overnight at ambient temperature, then diluted with 10 mL of water and extracted 3 times with 5 mL of EtOAc. The organic phases were combined, dried over sodium sulfate, filtered and concentrated to yield 100 mg of compound **1ZG** in the form of a partly purified colourless oil.

**Example 1**: Compound **1ZG** (90 mg, 0.10 mmol, 1.00 equiv) was dissolved in a neutral atmosphere in 2 mL of DCM and the solution was cooled with an ice bath. TFA (1 mL) was added and the reaction agitated for 2 hours at ambient temperature, then concentrated under reduced pressure. The residue was purified by preparative HPLC (Pre-HPLC-001 SHIMADZU, SunFire Prep C18 OBD column, 5 µm, 19 x 150 mm; Eluting phase: water / ACN buffered with 0.05 % of TFA; Gradient of 18 % to 31 % ACN in 7 minutes then 31 % to 100 % ACN in 2 minutes; Waters 2489 UV Detector at 254 nm and 220 nm). Compound **1** was obtained with a yield of 25 % (23 mg) in the form of a white solid.

LC/MS/UV (Atlantis T3 column, 3 µm, 4.6 x 100 mm; 35°C; 1 mL / min, 30 % to 60 % ACN in water (20 mM ammonium acetate in 6 minutes); ESI (C₄₄H₇₃N₇O₆S, exact masse 827.53) *m*/*z*: 829 (MH⁺), 5.84 min (93.7 %, 254 nm).

¹H NMR (300MHz, CD₃OD, *ppm*): δ (Presence of rotamers) 7.85 - 7.80 (m, 1H); 7.69 - 7.66 (m, 1H), 7.40 - 7.10 (m, 5H), 5.80 - 5.63 (m, 1H), 4.80 - 4.65 (m, 2H), 4.22 -4.00 (m, 1H), 3.89 - 0.74 (m, 58H).

### Reference Example 2

### (S)-2-((S)-2-(((2-aminopyridin-4-yl)methyl)(methyl)amino)-3-methylbutanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide, trifluoroacetic acid

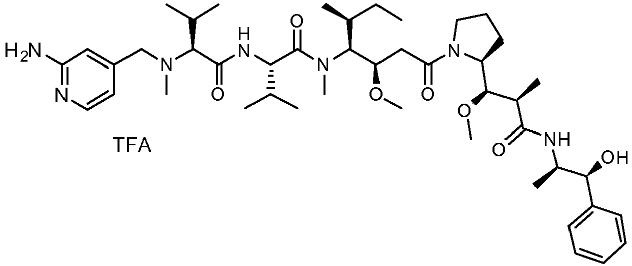

### Example 2A: tert-butyl (S)-2-((1R,2R)-3-(((1S,2R)-1-hydroxy-1-phenylpropan-2-yl)amino)-1-methoxy-2-methyl-3-oxopropyl)pyrrolidine-1-carboxylate

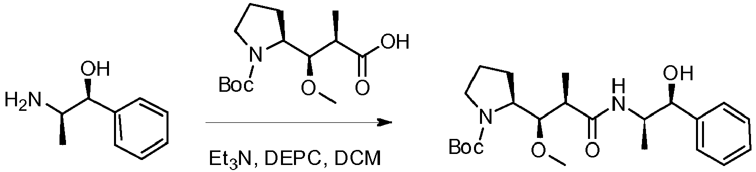

Compound **1D** (2.5 g, 8.70 mmol, 1.00 equiv) and (1S,2R)-2-amino-1-phenylpropan-1-ol (1.315 g, 8.70 mmol, 1.00 equiv) were dissolved in an inert atmosphere in DMF (35 mL). The solution was cooled to 0 °C then DEPC (1.39 mL) and TEA (1.82 mL) were added drop-wise. The reaction mixture was agitated 2 hours at 0 °C then 4 hours at ambient temperature. The reaction mixture was diluted with 200 mL of water and extracted three times with 50 mL of EtOAc. The organic phases were combined, washed once with 50 mL of KHSO₄ (1 mol/L), once with 50 mL of NaHCO₃ (sat.), once with 50 mL of NaCl (sat.), then dried over sodium sulfate, filtered and concentrated under reduced pressure to yield 3.6 g (98 %) of compound **2A** in the form of a yellow solid.

### Example 2B: (2R,3R)-N-((1S,2R)-1-hydroxy-1-phenylpropan-2-yl)-3-methoxy-2-methyl-3-((S)-pyrrolidin-2-yl)propanamide2,2,2-trifluoroacetate

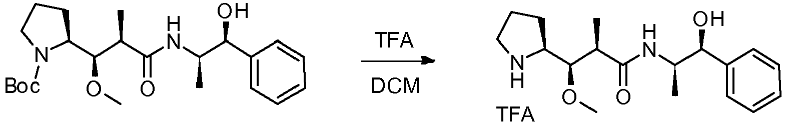

Compound **2A** (2.7 g, 6.42 mmol, 1.00 equiv) was dissolved in an inert atmosphere in DCM (40 mL) then cooled to 0 °C. TFA (25 mL) was added and the solution agitated for 2 hours at 0 °C. The reaction mixture was concentrated under reduced pressure to yield 4.4 g of compound **2B** in the form of a yellow oil.

### Example 2C: (9H-fluoren-9-yl)methyl ((S)-1-(((3R,4S,SS)-1-((S)-2-((1R,2R)-3-(((1S,2R)-1-hydroxy-1-phenylpropan-2-yl)amino)-1-methoxy-2-methyl-3-oxopropyl) pyrrolidin-1-yl)-3-methoxy-5-methyl-1-oxoheptan-4-yl) (methyl)amino)-3-methyl-1-oxobutan-2-yl)carbamate

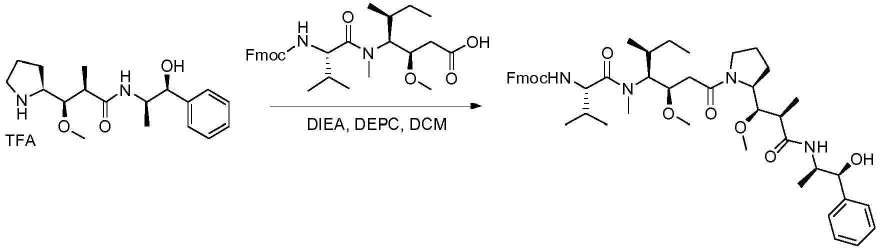

Compounds **2B** (4.4 g, 10.13 mmol, 1.00 equiv) and **1W** (5.31 g, 10.12 mmol, 1.00 equiv) were dissolved in an inert atmosphere in DCM (45 mL). The solution was cooled to 0°C then DEPC (1.62 mL) and DIEA (8.4 mL) were added drop-wise. The reation mixture was agitated for 2 hours at 0 °C then at ambient temperature overnight. The reaction mixture was diluted with 100 mL of water and extracted three times with 50 mL of DCM. The organic phases were combined, washed once with 50 mL of KHSO₄ (1 mol/L), once with 50 mL of NaHCO₃ (sat.), once with 50 mL of NaCl (sat.), then dried over sodium sulfate, filtered and concentrated under pressure to yield 3.3 g (39 %) of compound **2C** in the form of a yellow solid.

### Example 2D: (S)-2-amino-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide

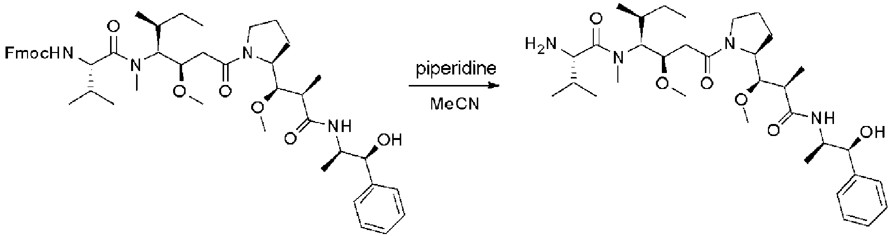

Compound **2C** (300 mg, 0.36 mmol, 1.00 eq.) was dissolved in an inert atmosphere in ACN (2 mL) and piperidine (0.5 mL). The solution was left under agitation at ambient temperature overnight then evaporated to dryness under reduced pressure. The residue was purified on a silica column with a mixture of DCM and MeOH (1:100) to yield 150 mg (68 %) of compound **2D** in the form of a white solid.

### Example 2E: methyl 2-((tert-butoxycarbonyl)amino)isonicotinate

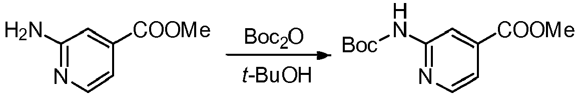

Methyl 2-aminopyridine-4-carboxylate (2 g, 13.14 mmol, 1.00 equiv) was dissolved in *tert*-butanol (20 mL) after which di-*tert*-butyl dicarbonate (4.02 g, 18.42 mmol, 1.40 equiv) was added. The reaction mixture was agitated at 60°C overnight then the reaction was halted through the addition of an aqueous 1M NaHCO₃ solution (50 mL). The solid was recovered by filtration, washed with 50 mL of EtOH then dried *in vacuo* to yield 2.5 g (75 %) of compound **2E** in the form of a white solid.

### Example 2F: tert-butyl (4-(hydroxymethyl)pyridin-2-yl)carbamate

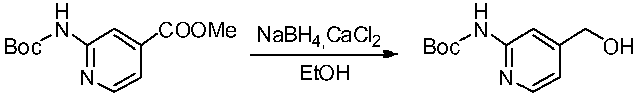

Compound **2E** (2.5 g, 9.91 mmol, 1.00 equiv) and CaCl₂ (1.65 g) were dissolved in EtOH (30 mL). The solution was cooled to 0°C then NaBH₄ (1.13 g, 29.87 mmol, 3.01 equiv) was gradually added. The solution was left under agitation overnight at ambient temperature then the reaction was halted with the addition of water (50 mL). The mixture was extracted three times with 20 mL of EtOAc. The organic phases were combined, washed twice with 20 mL of NaCl (sat.) then dried over sodium sulfate, filtered and concentrated under reduced pressure to yield 2.0 g (90 %) of compound **2F** in the form of a colourless solid.

### Example 2G: tert-butyl (4-formylpyridin-2-yl)carbamate

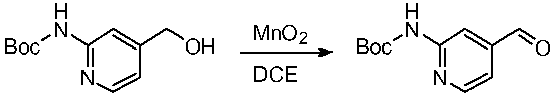

Compound **2F** (2.5 g, 11.15 mmol, 1.00 equiv) was dissolved in DCE (25 mL) then 19.4 g (223.14 mmol, 20.02 equiv) of MnO₂ were added. The mixture was left under agitation overnight at 70 °C then the solids were removed by filtering. The filtrate was evaporated to dryness to yield 1.4 g (57 %) of compound **2G** in the form of a white solid.

### Example 2H: benzyl (S)-2-(((2-((tert-butoxycarbonyl)amino)pyridin-4-yl) methyl) (methyl)amino)-3-methylbutanoate

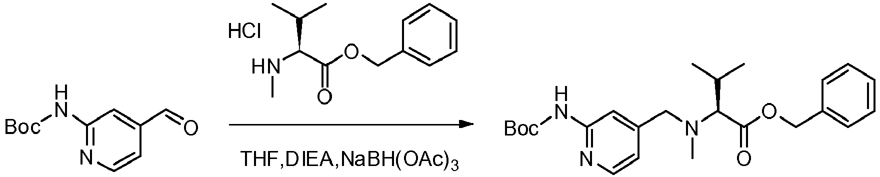

Compound **2G** (2.3 g, 10.35 mmol, 1.00 equiv) was dissolved in 25 mL of THF in the presence of compound **1ZC** (2.93 g, 11.37 mmol, 1.10 equiv), DIEA (5.39 g, 41.71 mmol, 4.03 equiv) and NaBH(OAC)₃ (4.39 g, 20.71 mmol, 2.00 equiv). The reaction mixture was agitated for 6 hours at ambient temperature then neutralised with 60 mL of NaHCO₃ (sat.) and extracted 3 times with 20 mL of AcOEt. The organic phases were combined, washed twice with 20 mL of NaCl (sat.), dried over sodium sulfate, filtered and concentrated. The residue was purified on a silica column with a mixture of EtOAc and PE (1:15) to yield 2.7 g (61 %) of compound **2H** in the form of a white solid.

### Example 2I: (S)-2-(((2-((tert-butoxycarbonyl)amino)pyridin-4-yl)methyl) (methyl)amino)-3-methylbutanoic acid

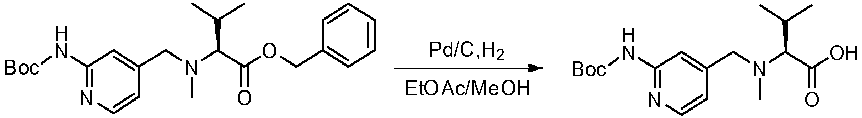

Compound **2H** (500 mg, 1.17 mmol, 1.00 equiv) was dissolved in 10 mL of AcOEt and 2 mL of methanol in the presence of Pd/C (250 mg), and hydrogenated for 3 hours at ambient temperature and atmospheric pressure. The reaction medium was filtered and concentrated under reduced pressure to yield 254 mg (64 %) of compound **2I** in the form of a colourless solid.

### Example 2J: tert-butyl (4-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxo ethyl)-3,6-diisopropyl-2,8-dimethyl-4,7-dioxo-11-oxa-2,5,8-triazadodecyl)pyridin-2-yl) carbamate

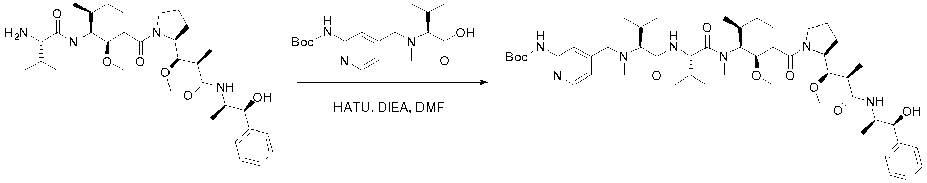

Compound **2J** was prepared in similar manner to compound **1ZG** from the amine **2D** (85.2 mg, 0.14 mmol, 1.50 equiv), the acid **2I** (31.7 mg, 0.09 mmol, 1.00 equiv), HATU (42.9 mg, 0.11 mmol, 1.20 equiv) and DIEA (36.7 mg, 0.28 mmol, 3.02 equiv) in DMF (3 mL). After evaporation to dryness, 100 mg of crude product were obtained in the form of a white solid.

**Example 2:** Compound **2J** (100 mg, 0.11 mmol, 1.00 equiv) was dissolved in 2 mL of DCM and 1 mL of TFA. The reaction was agitated for 1 hour at ambient temperature, then concentrated under reduced pressure. The residue (80 mg) was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm). Compound **2** was obtained with a yield of 6 % (6.3 mg) in the form of a white solid.

LC/MS/UV (Ascentis Express C18 column, 2.7 µm, 4.6 x 100 mm; 40°C; 1.8mL/min, from 10 % to 95 % ACN in water (0.05 % TFA) in 6 minutes); ESI (C₄₅H₇₃N₇O₇, exact mass 823.56) *m*/*z:* 824.5 (MH⁺) and 412.9 (M.2H⁺/2, 100 %), 3.21 min (99.2 %, 210 nm).

¹H NMR (400MHz, CDₛOD, *ppm*): δ (Presence of rotamers) 7.81 - 7.79 (m, 1H); 7.39 - 7.29 (m, 5H); 6.61 - 6.59 (m, 2H); 4.84 - 4.52 (m, 1H); 4.32 - 4.02 (m, 1H); 3.90 - 2.98 (m, 10H); 2.90 - 2.78 (m, 1H); 2.55 - 0.81 (m, 39H).

### Reference Example 3

### methyl ((S)-2-((2R,3R)-3-((S)-1-((3R,4S,5S)-4-((S)-N,3-dimethyl-2-((S)-3-methyl-2-(methyl(pyridin-4-ylmethyl)amino)butanamido)butanamido)-3-methoxy-5-methylheptanoyl)pyrrolidin-2-yl)-3-methoxy-2-methylpropanamido)-3-phenylpropanoate, trifluoroacetic acid

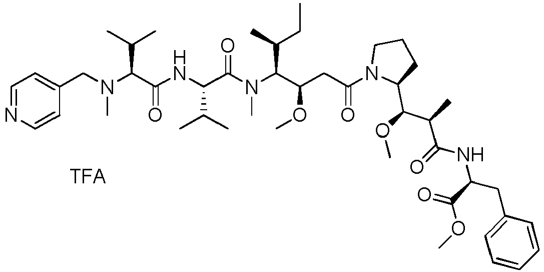

### Example 3A: tert-butyl (S)-2-((1R,2R)-1-methoxy-3-(((S)-1-methoxy-1-oxo-3-phenylpropan-2-yl)amino)-2-methyl-3-oxopropyl)pyrrolidine-1-carboxylate

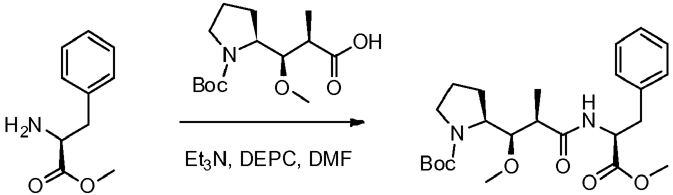

Compound **1D** (3 g, 10.44 mmol, 1.00 equiv) and methyl (S)-2-amino-3-phenylpropanoate (2.25 g, 12.55 mmol, 1.20 equiv) were dissolved in an inert atmosphere in DMF (40 mL). The solution was cooled to 0 °C then DEPC (1.67 mL, 1.05 equiv) and TEA (3.64 mL, 2.50 equiv) were added drop-wise. The reaction mixture was agitated 2 hours at 0 °C then at ambient temperature overnight. The reaction mixture was diluted with 100 mL of water and extracted three times with 50 mL EtOAc. The organic phases were combined, washed once with 100 mL of KHSO₄ (1 mol/L), once with 100 mL of NaHCO₃ (sat.), once with 100 mL of NaCl (sat.), then dried over sodium sulfate, filtered and concentrated under pressure to yield 4 g (85 %) of compound **3A** in the form of a colourless oil.

### Example 3B: 2,2,2-trifluoroacetate of methyl (S)-2-((2R,3R)-3-methoxy-2-methyl-3-((S)-pyrrolidin-2-yl)propanamido)-3-phenylpropanoate

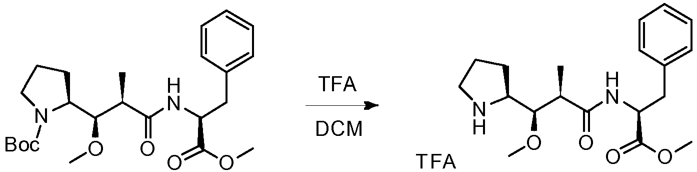

Compound **3A** (5 g, 11.15 mmol, 1.00 equiv) was dissolved in an inert atmosphere in DCM (40 mL). TFA (25 mL) was added and the solution agitated for 2 hours. The reaction mixture was concentrated under reduced pressure to yield 8 g of compound **3B** in the form of a yellow oil.

### Example 3C: methyl (S)-2-((2R,3R)-3-((S)-1-((3R,4S,5S)-4-((S)-2-((((9H-fluoren-9-yl)methoxy)carbonyl)amino)-N,3-dimethylbutanamido)-3-methoxy-5-methyl heptanoyl)pyrrolidin-2-yl)-3-methoxy-2-methylpropanamido)-3-phenylpropanoate

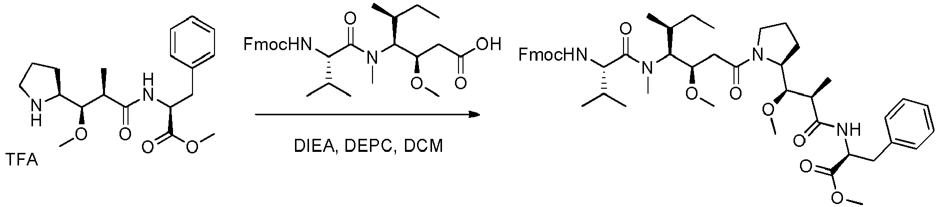

Compounds **3B** (8.03 g, 17.36 mmol, 1.00 equiv) and **1W** (9.1 g, 17.34 mmol, 1.00 equiv) were dissolved in an inert atmosphere in DCM (80 mL). The solution was cooled to 0 °C then DEPC (2.8 mL) and DIEA (12 mL) were added drop-wise. The reaction mixture was agitated for 2 hours at 0 °C then at ambient temperature overnight. The reaction mixture was diluted with 200 mL of water and extracted three times with 50 mL of DCM. The organic phases were combined, washed once with 50 mL of KHSO₄ (1 mol/L), once with 50 mL of NaHCO₃ (sat.), once with 50 mL of NaCl (sat.), then dried over sodium sulfate, filtered and concentrated under reduced pressure to yield 5 g (34 %) of compound **3C** in the form of a yellow solid.

### Example 3D: methyl (S)-2-((2R,3R)-3-((S)-1-((3R,4S,5S)-4-((S)-2-amino-N,3-dimethylbutanamido)-3-methoxy-5-methylheptanoyl)pyrrolidin-2-yl)-3-methoxy-2-methylpropanamido)-3-phenylpropanoate

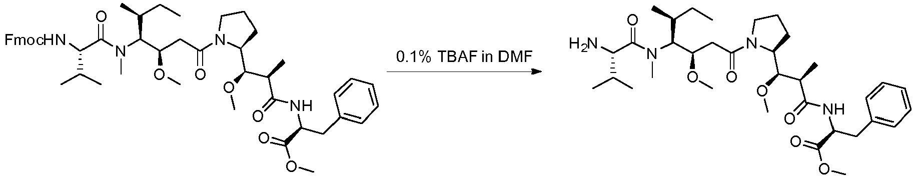

Compound **3C** (5.5 g, 6.43 mmol, 1.00 equiv) was dissolved in an inert atmosphere in a solution of tetrabutylammonium fluoride (TBAF, 2.61 g, 9.98 mmol, 1.55 quiv) in DMF (100 mL). The solution was agitated at ambient temperature for 2 hours then diluted with 100 mL of water and extracted three times with 50 mL of EtOAc. The organic phases were combined then dried over sodium sulfate, filtered and concentrated under reduced pressure to yield 3.3 g (81 %) of compound **3D** in the form of a yellow solid.

### Example 3E: benzyl (S)-3-methyl-2-(methyl(pyridin-4-ylmethyl)amino) butanoate

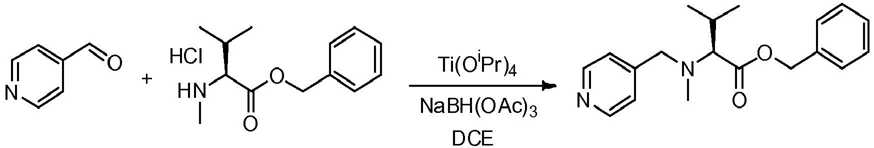

Pyridine-4-carbaldehyde (1 g, 9.34 mmol, 1.00 equiv) was dissolved in 10 mL of 1,2-dichloroethane (DCE) in the presence of compound **1ZC** (2.9 g, 11.25 mmol, 1.21 equiv) and titanium isopropoxide (IV) (4.19 mL, 1.40 equiv). The mixture was agitated at ambient temperature for 30 minutes then 2.77 g of NaBH(OAc)₃ (13.07 mmol, 1.40 equiv) were added. The reaction medium was left under agitation overnight then neutralised with 100 mL of water and the mixture extracted 3 times with 50 mL of AcOEt. The organic phases were combined and evaporated to dryness. The residue was purified on a silica column with a mixture of EtOAc and PE (1:20) to yield 1.3 g (45 %) of compound **3E** in the form of a colourless oil.

### Example 3F: (S)-3-methyl-2-(methyl(pyridin-4-ylmethyl)amino)butanoic acid

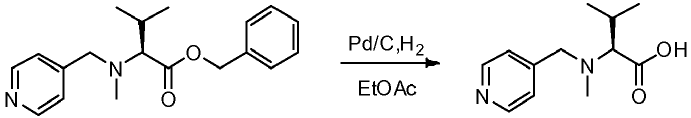

Compound **3E** (800 mg, 2.56 mmol, 1.00 equiv) was dissolved in 30 mL of AcOEt in the presence of Pd/C (300 mg) and hydrogenated for 3 hours at ambient temperature and atmospheric pressure. The reaction medium was filtered and concentrated under reduced pressure. The residue was purified on a silica column with a mixture of DCM and MeOH (100:1 to 5:1) to yield 100 mg (18 %) of compound **3F** in the form of a white solid.

**Example 3:** Compounds **3D** (50 mg, 0.08 mmol, 1.00 equiv) and **3F** (26.34 mg, 0.12 mmol, 1.50 equiv) were dissolved in 3 mL of DCM. The solution was cooled to 0 °C then 0.018 mL of DEPC and 0.0392 mL of DIEA were added. The reaction was agitated at 0°C for 2 hours then at ambient temperature overnight. The reaction medium was concentrated under reduced pressure and the residue (70 mg) was purified by preparative HPLC (Pre-HPLC-001 SHIMADZU, SunFire Prep C18 OBD column, 5 µm, 19 x 150 mm; Eluting phase: water / ACN buffered with 0.05 % of TFA; Gradient of 20 % to 40 % ACN in 10 minutes then 40 % to 100 % ACN in 2 minutes; Waters 2545 UV Detector at 254 nm and 220 nm). Compound **3** was obtained with a yield of 27 % (20 mg) in the form of a white solid.

LC/MS/UV (Ascentis Express C18 column, 2.7 µm, 4.6 x 100 mm; 40°C; 1.5 mL/min, 10 % to 95 % ACN in water (0.05 % TFA) in 8 minutes); ESI (C₄₆H₇₂N₆O₈, exact mass 836.5) *m*/*z*: 837.5 (MH⁺) and 419.4 (M.2H⁺/2 (100 %)), 7.04 min (90.0 %, 210 nm).

¹H NMR (400MHz, CD₃OD, *ppm*): δ (Presence of rotamers) 8.76 - 8.74 (m, 2H); 8.53 - 8.48 (m, 0.4H, NHCO incomplete exchange); 8.29 - 8.15 (m, 0.8H, NHCO incomplete exchange); 8.01 (s, 2H), 7.31 - 7.22 (m, 5H), 4.88 - 4.68 (m, 3H); 4.31 - 4.07 (m, 2H); 3.94 - 2.90 (m, 18H); 2.55 - 0.86 (m, 38H).

### Reference Example 4

### (S)-2-((2R,3R)-3-((S)-1-((3R,4S,5S)-4-((S)-N,3-dimethyl-2-((S)-3-methyl-2-(methyl(pyridin-4-ylmethyl)amino)butanamido)butanamido)-3-methoxy-5-methylheptanoyl)pyrrolidin-2-yl)-3-methoxy-2-methylpropanamido)-3-phenylpropanoic acid, trifluoroacetic acid

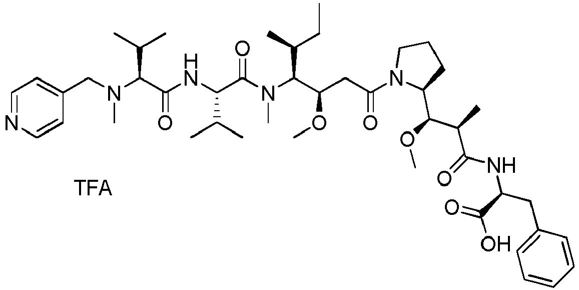

**Example 4:** Compound 3 (100 mg, 0.11 mmol, 1.00 equiv) was dissolved in a mixture of water (5 mL), ACN (5 mL) and piperidine (2.5 mL). The reaction mixture was left under agitation overnight then concentrated under reduced pressure. The residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2545 UV Detector at 254 nm and 220 nm), to yield 20 mg (20 %) of compound **4** in the form of a white solid.

LC/MS/UV (Ascentis Express C18 column, 2.7 µm, 4.6 x 100 mm; 40°C; 1.5 mL/min, 10 % to 95 % ACN in water (0.05 % TFA) in 8 minutes); ESI (C₄₅H₇₀N₆O₈, exact mass 822.5) *m*/*z*: 823.5 (MH⁺) and 412.4 (M.2H⁺/2, 100 %), 6.84 min (89.1 %, 210 nm).

¹H NMR (400MHz, CD₃OD, *ppm*): δ (Presence of rotamers) 8.79 - 8.78 (m, 2H); 8.09 (m, 2H); 7.30 - 7.21 (m, 5H); 4.80 - 4.80 (m, 1H), 4.36 - 0.87 (m, 58H).

### Reference Example 6

### methyl (S)-2-((2R,3R)-3-((S)-1-((3R,4S,5S)-4-((S)-2-((S)-2-((3-aminopropyl) (methyl)amino)-3-methylbutanamido)-N,3-dimethylbutanamido)-3-methoxy-5-methylheptanoyl)pyrrolidin-2-yl)-3-methoxy-2-methylpropanamido)-3-phenylpropanoate, bis trifluoroacetic acid

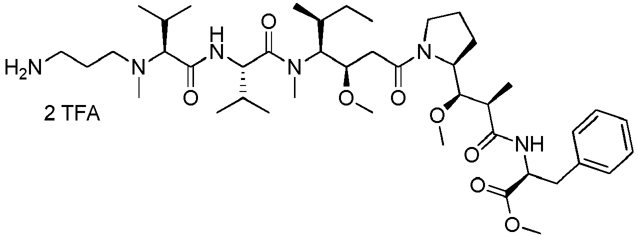

### Example 6A: methyl (2S)-2-[(2R)-2-[(R)-[(2S)-1-[(3R,4S,5S)-4-[(2S)-2-[(2S)-2-[(3-[[(tert-butoxy)carbonyl]amino]propyl)(methyl)amino]-3-methyl butanamido]-N,3-dimethylbutanamido]-3-methoxy-5-methylheptanoyl]pyrrolidin-2-yl](methoxy)methyl] propanamido]-3-phenylpropanoate

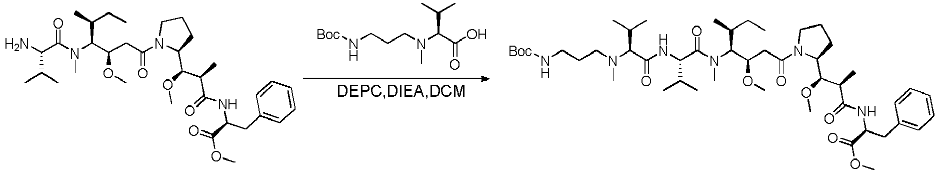

Compound **3D** (157.5 mg, 0.25 mmol, 1.00 equiv) was dissolved at 0°C in an inert atmosphere in 3 mL of DCM in the presence of carboxylic acid **1ZF** (78.7 mg, 0.27 mmol, 1.10 equiv), DEPC (46 µl) and DIEA (124 µl). The reaction mixture was agitated 2 hours at low temperature and the cold bath was then removed and agitation continued for 4 hours. It was then concentrated under reduced pressure to yield 200 mg of compound **6A** in the form of a crude yellow oil. It was used as such in the following step.

**Example 6:** Compound **6A** (200 mg, 0.22 mmol, 1.00 equiv) was dissolved in an inert atmosphere at 0°C in 2 mL of DCM. TFA (1 mL) was added drop-wise and the cold bath removed. The reaction mixture was agitated 1 hour at ambient temperature then concentrated under reduced pressure. The residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm), to yield 60 mg (26 %, yield in 2 steps) of compound **6** in the form of a white solid.

LC/MS/UV (Zorbax Eclipse Plus C8, 3.5 µm, 4.6 x 150 mm; 1 mL/min, 40°C, 30 to 80 % methanol in water (0.1 % H₃PO₄) in 18 minutes); ESI (C₄₃H₇₄N₆O₈, exact mass 802.56) *m*/*z*: 804 (MH⁺); 11.50 min (91.5 %, 210 nm).

¹H NMR (300MHz, CD₃OD, *ppm*): δ (Presence of rotamers) 8.52 (d, 0.3H, NHCO incomplete exchange); 8.25 (d, 0.5H, NHCO incomplete exchange); 7.30-7.22 (m, 5H); 4.9-4.6 (m, 3H); 4.2-4.0 (m, 1H); 4.0-0.86 (m, 61H).

### Reference Example 7

### (S)-2-((2R,3R)-3-((S)-1-((3R,4S,5S)-4-((S)-2-((S)-2-((3-aminopropyl) (methyl)amino)-3-methylbutanamido)-N,3-dimethylbutanamido)-3-methoxy-5-methylheptanoyl) pyrrolidin-2-yl)-3-methoxy-2-methylpropanamido)-3-phenylpropanoic acid, bis trifluoroacetic acid

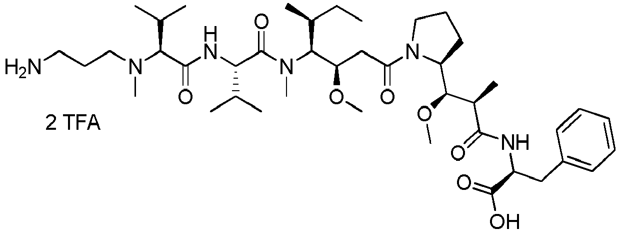

**Example 7:** Compound **6** (70 mg, 0.08 mmol, 1.00 equiv) was dissolved in a mixture of water (5 mL), ACN (2.5 mL) and piperidine (5 mL). The reaction mixture was left under agitation overnight at ambient temperature, then concentrated under reduced pressure. The residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; UV Waters 2489 UV Detector at 254 nm and 220 nm), to yield 14.6 mg (21 %) of compound 7 in the form of a white solid.

LC/MS/UV (Ascentis Express C18, 2.7 µm, 4.6 x 100 mm; 1.5 mL/min, 40°C, 0 to 80 % methanol in water (0.05 % TFA) in 8 minutes); ESI (C₄₂H₇₂N₆O₈, exact mass 788.54) *m*/*z:* 790 (MH⁺), 5.71 min (96.83 %, 210 nm).

¹H NMR (300MHz, CD₃OD, *ppm*): δ (Presence of rotamers) 8.42 (d, 0.3H, NHCO incomplete exchange); 8.15 (d, 0.2H, NHCO incomplete exchange); 7.31-7.21 (m, 5H); 4.9-4.6 (m, 3H); 4.25-4.0 (m, 1H); 4.0-0.86 (m, 59H).

### Example 11

### (S)-N-((3R,4S,5S)-3-methoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)-N,3-dimethyl-2-((S)-3-methyl-2-(methyl(4-(methylamino)phenethyl)amino) butanamido)butanamide, trifluoroacetic acid

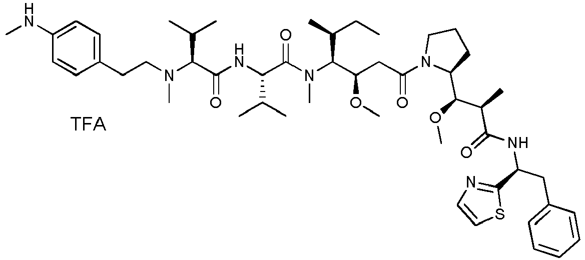

### Example 11A: tert-butyl N-[4-(2-hydroxyethyl)phenyl]carbamate

Compound **11A** was obtained with a yield of 75 % after reaction at ambient temperature of 2-(4-aminophenyl)ethanol with BOC₂O in THF.

### Example 11B: tert-butyl N-[4-(2-oxoethyl)phenyl]carbamate

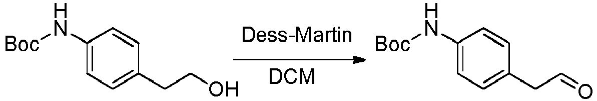

Compound **11A** (2.5 g, 10.5 mmol, 1.00 equiv) was dissolved in 25 mL of DCM then cooled to -78°C. A Dess-Martin Periodinane solution (DMP, 6.71 g, 15.8 mmol, 1.5 equiv) in DCM (10 mL) was added drop-wise. The cold bath was removed and agitation continued for 1 hour at ambient temperature. The reaction was neutralised with 60 mL of a 50/50 mixture of sodium bicarbonate-saturated aqueous solution and Na₂S₂O₃-saturated aqueous solution. The resulting solution was extracted 3 times with 30 mL of EtOAc. The organic phases were combined, washed twice with NaCl-saturated aqueous solution, dried over anhydrous sodium sulfate, filtered and concentrated under reduced pressure. The residue was purified on silica gel (EtOAc/PE 1/15) to yield 1.0 g (40 %) of compound **11B** in the form of a pale yellow solid.

### Example 11C: benzyl (2S)-2-[[2-(4-[[(tert-butoxy)carbonyl]amino]phenyl) ethyl](methyl)amino]-3-methylbutanoate

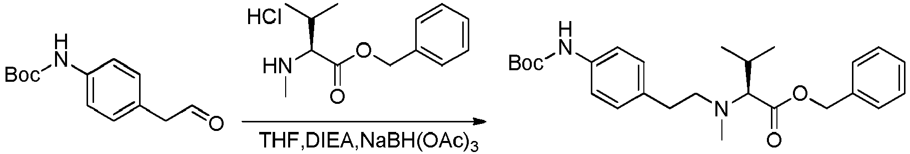

Compound **1ZC** (3.5 g, 13.6 mmol, 1.1 equiv) was dissolved in THF (30 mL) in the presence of DIEA (6.4 g, 49.7 mmol, 4.0 equiv), aldehyde **11B** (2.9 g, 12.3 mmol, 1.0 equiv) and sodium triacetoxyborohydride (5.23 g, 49.7 mmol, 2.0 equiv). The reaction mixture was left under agitation overnight at ambient temperature, then neutralised with 60 mL of sodium bicarbonate-saturated solution. The resulting solution was extracted 3 times with 30 mL EtOAc. The organic phases were combined, washed twice with NaCl-saturated aqueous solution, dried over anhydrous sodium sulfate, filtered and concentrated under reduced pressure. The residue was purified on silica gel (EtOAc/PE 1:20) to yield 3.7 g (68 %) of compound **11C** in the form of a yellow oil.

### Example 11D: (2S)-2-[[2-(4-[[(tert-butoxy)carbonyl]amino]phenyl)ethyl] (methyl)amino]-3-methylbutanoic acid

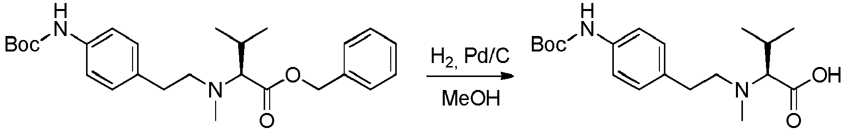

Compound **11C** (2 g, 4.5 mmol, 1 equiv) was dissolved in 10 mL of methanol in the presence of Pd/C (2 g) and hydrogenated for 2 hours at normal temperature and pressure. The reaction medium was filtered and concentrated under reduced pressure to yield 1.2 g (75 %) of compound **11D** in the form of a yellow oil.

### Example 11E: (2S)-2-[[2-(4-[[(tert-butoxy)carbonyl](methyl)amino]phenyl) ethyl](methyl) amino]-3-methylbutanoic acid

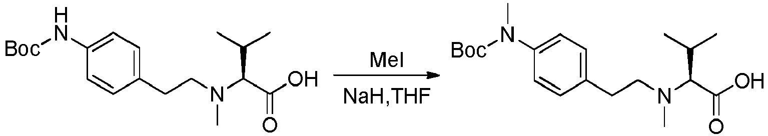

Compound **11D** (1.2 g, 3.4 mmol, 1.00 equiv) was dissolved in an inert atmosphere in THF (20 mL). The reaction medium was cooled with an ice bath after which NaH (60 % in oil, 549 mg, 13.7 mmol, 4.0 equiv) was added in portions, followed by iodomethane (4.9 g, 34 mmol, 10 equiv). The reaction was left under agitation overnight at ambient teperature, then neutralised with water and washed with 100 mL of EtOAc. The pH of the aqueous solution was adjusted to 6-7 with 1N HCl. This aqueous solution was extracted 3 times with 100 mL of EtOAc. The organic phases were combined, dried over sodium sulfate, filtered and concentrated to yield 800 mg (64 %) of compound **11E** in the form of a yellow solid.

### Example 11F: tert-butyl N-[4-(2-[[(1S)-1-[[(1S)-1-[[(3R,4S,5S)-3-methoxy-1-[(2S)-2-[(1R,2R)-1-methoxy-2-methyl-2-[[(1S)-2-phenyl-1-(1,3-thiazol-2-yl) ethyl]carbamoyl]ethyl]pyrrolidin-1-yl]-5-methyl-1-oxoheptan-4yl] (methyl)carbamoyl]-2-methylpropyl]carbamoyl]-2-methylpropyl](methyl)amino] ethyl)phenyl]-N-methyl carbamate

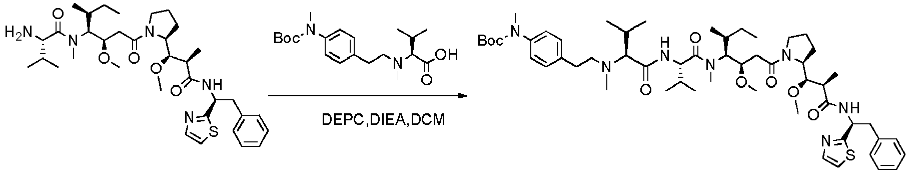

Compound **11F** was prepared in similar manner to compound **6A** from the amine **1Y** (150 mg, 0.22 mmol, 1.2 equiv) and the acid **11E** (70 mg, 0.19 mmol, 1.0 equiv). After purification on silica gel (EtOAc/PE 1:1) 100 mg (52 %) of desired product were obtained in the form of a pale yellow solid.

**Example 11:** Compound **11** was prepared in the same manner as for compound **1** from the intermediate **11F** (100 mg, 0.1 mmol). The residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm). Compound **11** was obtained with a yield of 39 % (39.7 mg) in the form of a white solid.

LC/MS/UV (Eclipse Plus C8, 3.5 µm, 4.6 x 150 mm; 1 mL/min, 40°C, 50 to 95 % methanol in water (0.05 % TFA) in 18 minutes); ESI (C₅₀H₇₇N₇O₆S, exact mass 903.57) *m*/*z:* 904.5 (MH⁺), 7.53 min (93.68 %, 254 nm).

¹H NMR (300MHz, CD₃OD, *ppm*): δ (Presence of rotamers) 8.84 (d, 0.5H, NHCO incomplete exchange); 8.7-8.5 (m, 0.9H, NHCO incomplete exchange); 7.76-7.73 (m, 1H); 7.55 - 7.4 (m, 1H); 7.28-7.22 (m, 7H); 7.08-7.05 (m, 2H); 5.51-5.72 (m, 1H); 4.9-4.80 (m, 2H); 4.3-0.7 (m, 60H).

### Example 12

### methyl (S)-2-((2R,3R)-3-((S)-1-((3R,4S,5S)-4-((S)-N,3-dimethyl-2-((S)-3-methyl-2-(methyl(4-(methylamino)phenethyl)amino)butanamido)butanamido)-3-methoxy-5-methylheptanoyl)pyrrolidin-2-yl)-3-methoxy-2-methylpropanamido)-3- phenylpropanoate, trifluoroacetic acid

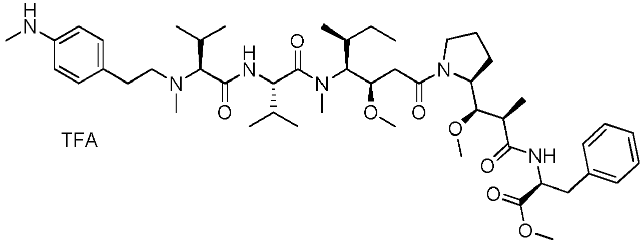

**Example 12**: In the same manner as for the final phases in the synthesis of compound **1,** compound **12** was prepared in two steps from the amine **3D** (118 mg, 0.19 mmol) and the acid **11E** (82 mg, 0.22 mmol). The final residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm). Compound **12** was obtained with a yield of 7 % (13.7 mg) in the form of a white solid.

LC/MS/UV (Eclipse Plus C8, 3.5 µm, 4.6 x 150 mm; 1 mL/min, 40°C, 40 to 95 % methanol in water (0.05 % TFA) in 18 minutes); ESI (C₄₉H₇₈N₆O₈, exact mass 878.59) *m*/*z*: 879.7 (MH⁺), 10.07 min (90.6 %, 254 nm).

¹H:NMR (300MHz, CD₃OD, *ppm*): δ (Presence of rotamers) 7.40 (se, 2H); 7.38-7.22 (m, 7H); 4.95-4.7 (m, 3H); 4.2-4.0 (m, 1H); 3.9-0.86 (m, 62H).

### Example 13

### (S)-2-((2R,3R)-3-((S)-1-((3R,4S,5S)-4-((S)-N,3-dimethyl-2-((S)-3-methyl-2-(methyl(4-(methylamino)phenethyl)amino)butanamido)butanamido)-3-methoxy-5-methyl heptanoyl)pyrrolidin-2-yl)-3-methoxy-2-methylpropanamido)-3-phenylpropanoic acid, trifluoroacetic acid

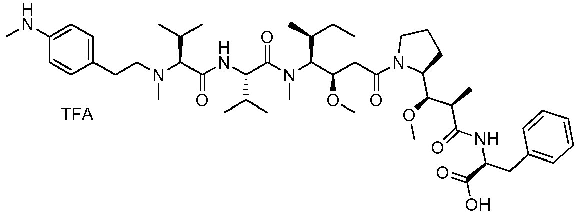

**Example 13**: Compound **13** was prepared in the same manner as for compound **7** from compound **12** (100 mg, 0.10 mmol). The residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm). Compound **13** was obtained with a yield of 20 % (20 mg) in the form of a white solid.

LC/MS/UV (Ascentis Express C18, 2.7 µm, 4.6 x 100 mm; 1.5 mL/min, 40°C, 10 to 95 % methanol in water (0.05 % TFA) in 8 minutes); ESI (C₄₈H₇₆N₆O₈, exact mass 864.57) *m*/*z:* 865.6 (MH⁺), 6.05 min (90.9 %, 210 nm).

¹H NMR: (300MHz, CD₃OD, *ppm*): δ (Presence ofrotamers) 7.32-7.19 (m, 9H); 4.9-4.65 (m, 3H); 4.2-4.0 (m, 1H); 3.9-0.86 (m, 59H).

### Example 14

### (S)-2-((S)-2-((3-aminobenzyl)(methyl)amino)-3-methylbutanamido)-N-((3R,4S,5S)-3-methoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)-N,3-dimethylbutanamide, trifluoroacetic acid

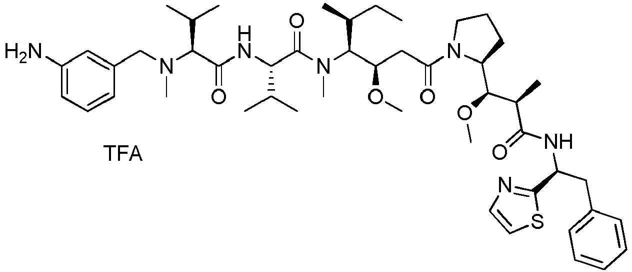

### Example 14A: tert-butyl (3-(hydroxymethyl)phenyl) carbamate

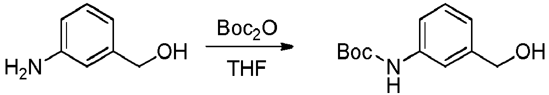

(3-aminophenyl)methanol (3 g, 24.36 mmol, 1.00 equiv) was dissolved in THF (60 mL) after which di-*tert*-butyl dicarbonate (6.38 g, 29.23 mmol, 1.20 equiv) was then added. The reaction mixture was left under agitation overnight at ambient temperature and the reaction was then diluted by adding 200 mL of water. The product was extracted 3 times with 100 mL of AcOEt and the organic phases were then recombined, dried over sodium sulfate, filtered and concentrated under reduced pressure to yield the crude product (13.85 g of compound **14A)** in the form of a yellow oil.

### Example 14B: tert-butyl (3-formylphenyl)carbamate

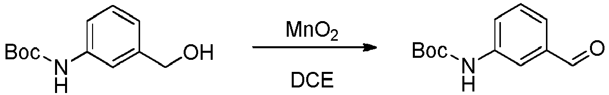

Compound **14A** (13.8 g, 61.81 mmol, 1.00 equiv) was dissolved in DCE (400 mL) and MnO₂ (54 g, 621.14 mmol, 10.05 equiv) was then added. The mixture was left under agitation at ambient temperature for 3 days after which the solids were removed by filtering. The filtrate was evaporated to dryness and the residue was purified on a silica column with a mixture of EtOAc and PE (1:30) to yield 3 g (22 %) of compound **14B** in the form of a white solid.

### Example 14C: benzyl (S)-2-((3-((tert-butoxycarbonyl)amino)benzyl) (methyl) amino)-3-methylbutanoate

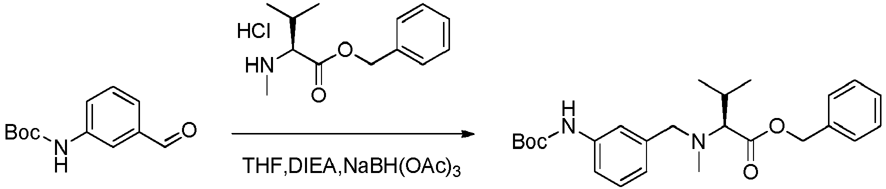

Compound **14B** (1 g, 4.52 mmol, 1.00 equiv) was dissolved in 20 mL of THF in the presence of compound **1ZC** (1.16 g, 4.50 mmol, 1.00 equiv), DIEA (3 mL) and NaBH(OAc)₃ (1.92 g, 9.06 mmol, 2.01 equiv). The reaction mixture was left under agitation overnight at ambient temperature and then neutralised with 100 mL of water and extracted 3 times with 50 mL of AcOEt. The organic phases were combined, dried over sodium sulfate, filtered and concentrated. The residue was purified on a silica column with a mixture of EtOAc and PE (1:50) to yield 1.9 g (99 %) of compound **14C** in the form of a white solid.

### Exemple 14D: (S)-2-((3-((tert-butoxycarbonyl)amino)benzyl)(methyl)amino)-3-methylbutanoic acid

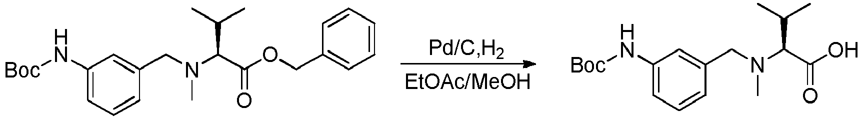

Compound **14C** (1 g, 2.34 mmol, 1.00 equiv) was dissolved in 30 mL of AcOEt and 4 mL of methanol in the presence of Pd/C (400 mg) and hydrogenated for 1 hour at ambient temperature and atmospheric pressure. The reaction medium was filtered and concentrated under reduced pressure to yield 680 mg (86 %) of compound **14D** in the form of a white solid.

### Example 14E: tert-butyl (3-((3S,6S,9S,10R)-9-((S)-sec-butyl)-3,6-diisopropyl-10-(2-((S)-2-((1R,2R)-1-methoxy-2-methyl-3-oxo-3-(((S)-2-phenyl-1-(thiazol-2-yl) ethyl)amino)propyl)pyrrolidin-1-yl)-2-oxoethyl)-2,8-dimethyl-4,7-dioxo-11-oxa-2,5,8-triazadodecyl)phenyl) carbamate

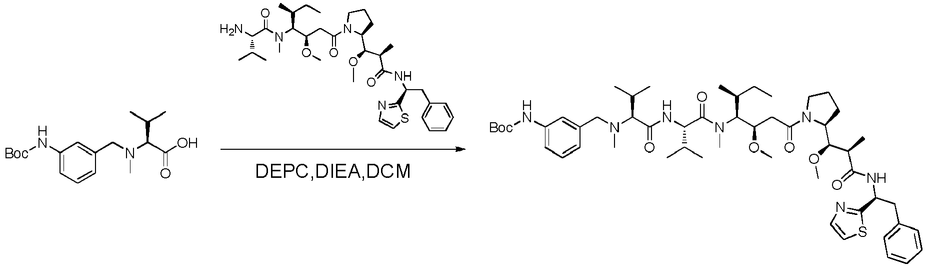

Compound **14E** was synthesised in the same manner as for compound 3 from the amine **1Y** (100 mg, 0.15 mmol, 1.00 equiv), the acid **14D** (102.27 mg, 0.30 mmol, 2.00 equiv), DEPC (0.053 mL) and DIEA (0.046 mL) in DCM (3 mL). The crude product (80 mg) was purified on a silica column with a mixture of EtOAc and PE (1:1) to yield 100 mg (67 %) of compound **14E** in the form of a pale yellow solid.

**Example 14:** Compound **14** was synthesised in the same manner as for compound **2** from the intermediate **14E** (100 mg, 0.10 mmol, 1.00 equiv). The crude product (80 mg) was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2545 UV Detecctor at 254 nm and 220 nm). Compound **14** was obtained with a yield of 10 % (10 mg) in the form of a white solid.

LC/MS/UV (Eclipse plus C8 column, 3.5 µm, 4.6 x 150 mm; 40°C; 1.0 mL / min, 40 % to 95 % MeOH in water (0.05 % TFA) in 18 minutes); ESI (C₄₈H₇₃N₇O₆S, exact mass 875.5) *m*/*z*: 876.5 (MH⁺) and 438.9 (M.2H⁺/2, 100 %), 11.35 min (95.6 %, 210 nm).

¹H NMR (400MHz, CD₃OD, *ppm*): δ (Presence of rotamers) 8.92 - 8.86 (m, 0.4H, NH incomplete exchange); 8.70 - 8.54 (m, 0.6H, NH incomplete exchange); 7.88 - 7.78 (m, 1H); 7.60 - 7.50 (m, 1H); 7.45 - 6.97 (m, 9H); 5.80 - 5.65 (m, 1H); 4.85 - 4.70 (m, 1H); 4.40 - 0.80 (m, 56H).

### Example 15

### methyl (S)-2-((2R,3R)-3-((S)-1-((3R,4S,5S)-4-((S)-2-((S)-2-((3-aminobenzyl) (methyl)amino)-3-methylbutanamido)-N,3-dimethylbutanamido)-3-methoxy-5-methylheptanoyl)pyrrolidin-2-yl)-3-methoxy-2-methylpropanamido)-3-phenylpropanoate, trifluoroacetic acid

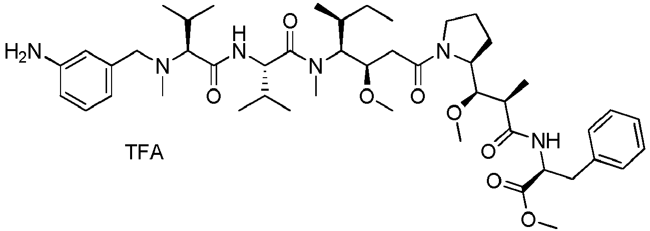

### Example 15A: methyl (S)-2-((2R,3R)-3-((S)-1-((3R,4S,SS)-4-((S)-2-((S)-2-((3-((tert-butoxycarbonyl)amino)benzyl)(methyl)amino)-3-methylbutanamido)-N, 3-dimethylbutanamido)-3-methoxy-5-methylheptanoyl)pyrrolidin-2-yl)-3-methoxy-2-methylpropanamido)-3-phenylpropanoate

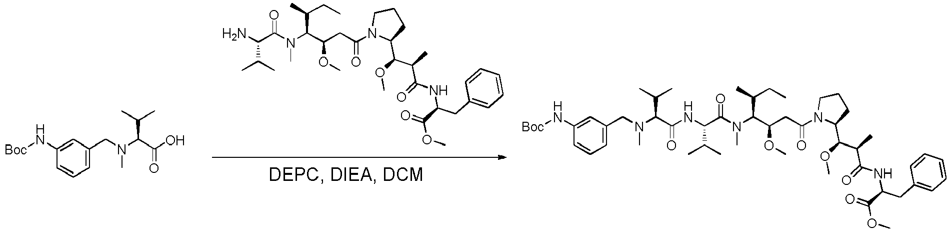

Compound **15A** was synthesised in the same manner as for compound **3** from the amine **3D** (200 mg, 0.32 mmol, 1.00 equiv), the acid **14D** (212.6 mg, 0.63 mmol, 2.00 equiv), DEPC (0.1103 mL) and DIEA (0.157 mL, 3.00 equiv) in DCM (5 mL). The crude product was purified on a silica column with a mixture of EtOAc and PE (1:1) to yield 200 mg (67 %) of compound **15A** in the form of a yellow solid.

**Example 15:** Compound **15** was synthesised in the same manner as for compound **2** from the intermediate **15A** (200 mg, 0.21 mmol, 1.00 equiv). The crude product was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters UV Detector 2545 at 254 nm and 220 nm). Compound 15 was obtained with a yield of 19 % (38.6 mg) in the form of a white solid.

LC/MS/UV (Ascentis Express C18 column, 2.7 µm, 4.6 x 100 mm; 40°C; 1.5 mL/min, 10 % to 95 % MeOH in water (0.05 % TFA) in 8 minutes); ESI (C₄₇H₇₄N₆O₈, exact mass 850.5) *m*/*z*: 851.5 (MH⁺) and 426.4 (M.2H⁺/2, 100 %), 6.61 min (91.1 %, 210 nm).

¹H NMR (400MHz, CD₃OD, *ppm):* δ (Presence of rotamers) 7.53 - 7.42 (m, 1H); 7.35 - 7.18 (m, 8H); 4.88 - 4.79 (m, 2H); 4.42 - 4.00 (m, 3H); 3.93 - 2.71 (m, 22H); 2.61 - 0.81 (m, 33H).

### Examples 19 and 20

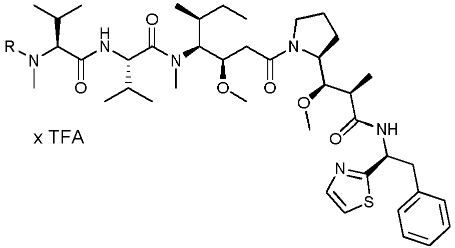

Compounds **19** and **20** were prepared in the same manner as for compound **1,** from the amines **1Y** and **1ZC** and corresponding aldehydes.

The *tert*-butyl 4-formylphenyl carbonate involved in the preparation of compound **19** was prepared in a single step as follows: 4-hydroxybenzaldehyde (2.5 g, 20.5 mmol, 1.0 equiv) was dissolved in an inert atmosphere in THF (20 mL) in the presence of 18-crown-6 (0.25 g) and potassium carbonate (5 g). The reaction mixture was cooled to 0°C and the di-*tert*-butyl dicarbonate (5.8 g, 26.58 mmol, 1.30 equiv) was then added. Agitation was continued for 1 hour at low temperature after which the reaction was neutralised with 30 mL of water. The resulting solution was extracted three times with 200 mL of EtOAc. The organic phases were combined, dried over anhydrous sodium sulfate filtered and concentrated under reduced pressure. The residue was purified on silica gel (EtOAc/PE 1:10) and yielded 4.2 g (92 %) of *tert*-butyl 4-formylphenyl carbonate in the form of a pale yellow solid.

The 4-nitrobenzaldehyde involved in the preparation of compound **20** was commercial.

The synthesis of compound **20** was completed by reducing the nitro group. This was performed as follows: (2S)-*N*-[(3R,4S,5S)-1-[(2S)-2-[(1R,2R)-2-[[(1S,2R)-1-hydroxy-1-phenylpropan-2-yl]carbamoyl]-1-methoxy-2-methylethylpyrrolidin-1-yl]-3-methoxy-5-methyl-1-oxoheptan-4-yl]-*N*,3-dimethyl-2-[(2S)-3-methyl-2-[methyl[(4-nitrophenyl)methyl]amino]butanamido]butanamide (40 mg, 0.05 mmol, 1.0 equiv) was dissolved in 15 mL of ethanol. Dihydrated tin chloride (II) (317 mg, 1.4 mmol, 30 equiv) was added and the solution left under agitation for 3 days at ambient temperature. The reaction was neutralised with 50 mL of water, then extracted three times with 50 mL of EtOAc. The organic phases were combined, dried over anhydrous sodium sulfate, filtered and concentrated under reduced pressure to yield compound **20** in the crude state.

| **N°** | **Name** | **x** | **R** | **Purity*** | **Quantity** |
|---|---|---|---|---|---|
| **19** | (S)-2-((S)-2-((4-hydroxybenzyl) (methyl)amino)-3-methyl butanamido)-*N-*((3R,4S,5S)-3-methoxy-1-((S)-2-((1R,2R)-1-methoxy-2-methyl-3-oxo-3-(((S)-2-phenyl-1-(thiazol-2-yl)ethyl)amino) propyl)pyrrolidin-1-yl)-5-methyl-1-oxoheptan-4-yl)-*N*,3-dimethyl butanamide, trifluoroacetic acid | 1 | 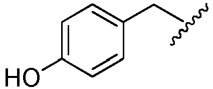 | 93.2 % | 21.6 mg |
| **20** | (S)-2-((S)-2-((4-aminobenzyl) (methyl)amino)-3-methyl butanamido)-*N-*((3R,4S,5S)-3-methoxy-1-((S)-2-((1R,2R)-1-methoxy-2-methyl-3-oxo-3-(((S)-2-phenyl-1-(thiazol-2-yl)ethyl)amino) propyl)pyrrolidin-1-yl)-5-methyl-1-oxoheptan-4-yl)-*N*,3-dimethyl butanamide, trifluoroacetic acid | 1 | 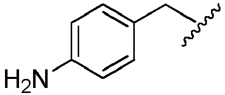 | 96.7 % | 21.1 mg |

| | | | | | |
|---|---|---|---|---|---|
| * The compounds were purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm), to give the corresponding TFA salts in the form of white solids. | | | | | |

Characterization of the end products: Compound **19** LC/MS/UV ESI: (C₄₈H₇₂N₆O₇S, exact mass 876.52) *m*/*z* 877 (MH⁺), 439 [100 %, (M.2H⁺)/2]; UV: RT = 1.76 min (93.2 %, 220 nm). Compound **20** ¹H NMR: (400MHz, CD₃OD, *ppm):* δ (Presence ofrotamers) 7.85-7.80 (m, 1H); 7.6-7.5 (m, 1H); 7.4-7.15 (m, 5H); 7.1-7.05 (m, 2H); 6.73-6.70 (m, 2H); 5.8-5.55 (m, 1H); 5.0-4.7 (m, 2H); 4.25-4.05 (m, 1H); 4.0-0.8 (m, 54H). LC/MS/UV ESI: (C₄₈H₇₃N₇O₇S, exact mass 875.53) *m*/*z* 876 (MH⁺), 439 [75 %, (M.2H⁺)/2]; UV: RT = 4.83 min (96.8 %, 254 nm). ¹H NMR (400MHz, CD₃OD, *ppm*): δ (Presence of rotamers) 7.85-7.80 (m, 1H); 7.6-7.5 (m, 1H); 7.4-7.1 (m, 7H); 6.76-6.72 (m, 2H); 5.8-5.55 (m, 1H); 4.9-4.65 (m, 2H); 4.25-4.05 (m, 1H); 4.0-0.8 (m, 54H).

### Examples 23 and 24

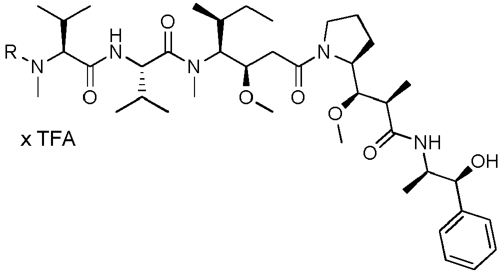

Compounds **23** and **24** were prepared in the same manner as for compounds **19** and **20,** replacing the amine **1Y** by the amine **2D.**

| **N°** | **Name** | **x** | **R** | **Purity*** | **Quantity** |
|---|---|---|---|---|---|
| **23** | (S)-*N*-((3R,4S,5S)-1-((S)-2-((1R,2R)-3-(((1S,2R)-1-hydroxy-1-phenyl propan-2-yl)amino)-1-methoxy-2-methyl-3-oxopropyl)pyrrolidin-1-yl)-3-methoxy-5-methyl-1-oxoheptan-4-yl)-2-((S)-2-((4-hydroxybenzyl) (methyl)amino)-3-methylbutanamido) -*N*,3-dimethylbutanamide, trifluoroacetic acid | 1 | 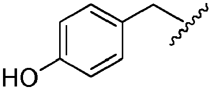 | 98.5 % | 5.8 mg |
| **24** | (S)-2-((S)-2-((4-aminobenzyl) (methyl)amino)-3-methylbutanamido) -*N-*((3R,4S,SS)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-*N*,3-dimethylbutanamide, trifluoroacetic acid | 1 | 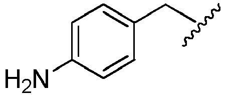 | 99.1 % | 6.9 mg |

| | | | | | |
|---|---|---|---|---|---|
| * The compounds were purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2489 UV Detector at 254 nm and 220 nm), to give the corresponding TFA salts in the form of white solids. | | | | | |

Characterization of the end products: Compound **23** LC/MS/UV (ESI) (C₄₆H₇₃N₅O₈, exact mass 823.55) *m*/*z* 824 (MH⁺), 846 (MNa⁺), 413 (100 %, (M.2H⁺)/2); UV: 4.76 min (98.5 %, 215 nm). ¹H NMR (400MHz, CDCl₃, *ppm):* δ (Presence of rotamers) 7.5-7.2 (m, 5H); 7.9-7.75 (m, 2H); 5.5-5.3 (m, 1H); 4.9-4.6 (m, 2H); 4.55-4.15 (m, 4H); 4.0-0.8 (m, 55H). Compound **24** LC/MS/UV (ESI) (C₄₆H₇₄N₆O₇, exact mass 822.56) *m*/*z* 823 (MH⁺), 845 (MNa⁺), 861 (MK⁺); UV: 3.68 min (99.15 %, 254 nm). ¹H NMR (400MHz, CD₃OD, *ppm):* δ (Presence of rotamers) 8.0-7.7 (m, 0.5H, NHCO incomplete exchange); 7.5-7.0 (m, 7H); 6.75-6.65 (m, 2H); 4.85-4.5 (m, 2H); 4.4-4.05 (m, 2H); 4.0-0.8 (m, 56H).

### Example 27

### methyl (S)-2-((2R,3R)-3-((S)-1-((3R,4S,5S)-4-((S)-2-((S)-2-((4-hydroxyphenethyl)(methyl)amino)-3-methylbutanamido)-N,3-dimethylbutanamido)-3-methoxy-5-methylheptanoyl)pyrrolidin-2-yl)-3-methoxy-2-methylpropanamido)-3-phenylpropanoate, trifluoroacetic acid

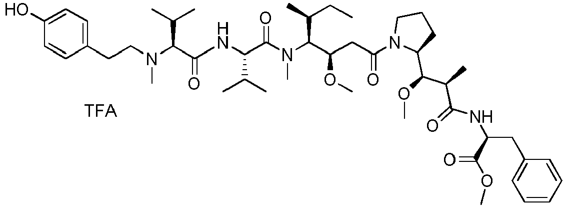

**Example 27:** Compound **27** was prepared in the same manner as for compound **3** from the amine **3D** (70 mg, 0.11 mmol, 1.00 equiv), the acid **49C** (55.5 mg, 0.22 mmol, 2.00 equiv), DEPC (0.034 mL, 2.00 equiv) and DIEA (0.055 mL, 3.00 equiv) in DCM (3 mL). The crude product was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 45 % ACN in 10 minutes then 40 % to 100 % ACN in 2 minutes; Waters 2545 UV Detector at 254 nm and 220 nm). Compound **27** was obtained with a yield of 3 % (2.9 mg) in the form of a white solid.

LC/MS/UV (Eclipse Plus C8 column, 3.5 µm, 4.6 x 150 mm; 40°C; 1.5 mL/min, 10 % to 95 % MeOH in water (0.05 % TFA) in 8 minutes); ESI (C₄₈H₇₅N₅O₉, exact mass 866.56) *m*/*z:* 866.5 (MH⁺) and 433.9 (M.2H⁺/2, 100 %), 6.61 min (89.1 %, 210 nm).

¹H NMR (400MHz, CD₃OD, *ppm*): δ (Presence of rotamers) 8.70 - 8.49 (m, 0.9 H, NH/OH incomplete exchange); 8.30 - 8.22 (m, 0.3H, NH incomplete exchange); 7.36 - 7.02 (m, 7H); 6.86 - 6.62 (m, 2H); 4.82 - 4.69 (m, 2H); 4.20 - 4.03 (m, 1H); 3.91 - 3.33 (m, 12H); 3.30 - 2.90 (m, 17H); 2.55 - 0.80 (m, 35H).

### Example 28

### (S)-2-((S)-2-((3-aminobenzyl)(methyl)amino)-3-methylbutanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide, trifluoroacetic acid

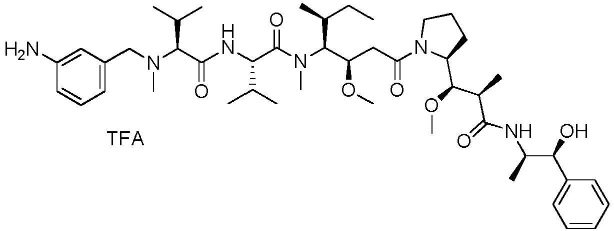

### Example 28A: tert-butyl (3-((3S,6S,9S,10R)-9-((S)-sec-butyl)-10-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-3,6-diisopropyl-2,8-dimethyl-4,7-dioxo-11-oxa-2,5,8-triazadodecyl)phenyl)carbamate

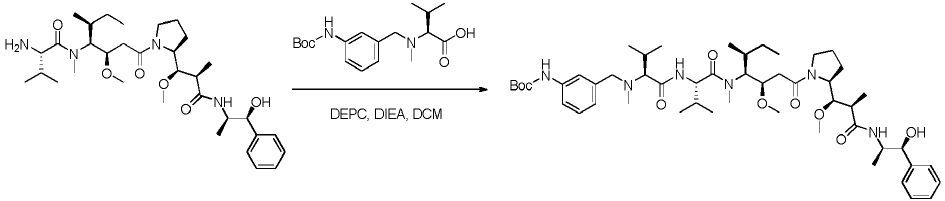

Compound **28A** was prepared in the same manner as for compound **3** from the amine **2D** (100 mg, 0.17 mmol, 1.00 equiv), the acid **14D** (111.25 mg, 0.33 mmol, 2.00 equiv), DEPC (0.058 mL) and DIEA (0.05 mL) in DCM (3 mL). The residue was purified on a silica column with a mixture of EtOAc and hexane (1:1) to yield 100 mg (66 %) of compound **28A** in the form of a white solid.

**Example 28:** Compound **28** was synthesised in the same manner as for compound **2** from the intermediate **28A** (100 mg, 0.11 mmol, 1.00 equiv). The crude product (80 mg) was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2545 UV Detector at 254 nm and 220 nm). Compound **28** was obtained with a yield of 20 % (20 mg) in the form of a white solid.

LC/MS/UV (Ascentis Express C18 column, 2.7 µm, 4.6 x 100 mm; 40°C; 1.5 mL/min, 10 % to 95 % MeOH in water (0.05 % TFA) in 8 minutes); ESI (C₄₆H₇₄N₆O₇, exact mass 822.56) *m*/*z:* 823.5 (MH⁺) and 412.4 (M.2H⁺/2, 100 %), 12.45 min (87.2 %, 210 nm).

¹H NMR: (400MHz, CD₃OD, *ppm*): δ (Presence of rotamers) 7.47 - 7.20 (m, 5H); 7.10 - 7.01 (m, 1H); 6.80 - 6.56 (m, 3H); 4.82 - 4.52 (m, 3H); 4.33 - 4.03 (m, 2H); 3.91 - 3.82 (m, 0.5H); 3.75 - 3.35 (m, 9.5H); 3.28 - 3.10 (m, 2H); 2.79 - 2.90 (m, 1H); 2.60 - 2.40 (m, 2H); 2.30 - 0.80 (m, 40H).

### Example 29

### (S)-2-((2R,3R)-3-((S)-1-((3R,4S,5S)-4-((S)-2-((S)-2-((3-aminobenzyl) (methyl)amino)-3-methylbutanamido)-N,3-dimethylbutanamido)-3-methoxy-5-methylheptanoyl)pyrrolidin-2-yl)-3-methoxy-2-methylpropanamido)-3-phenylpropanoic acid, trifluoroacetic acid

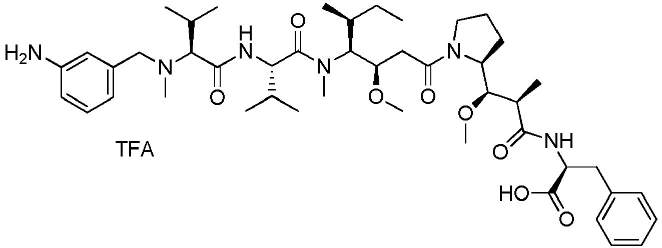

**Example 29:** Compound **15** (100 mg, 0.10 mmol, 1.00 equiv) was dissolved in a mixture of water (5 mL), ACN (5 mL) and piperidine (2.5 mL). The reaction mixture was left under agitation overnight at ambient temperature and then concentrated under reduced pressure. The residue was purified by preparative HPLC (Pre-HPLC-001 SHIMADZU, SunFire Prep C18 OBD column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2545 UV Detector at 254 nm and 220 nm), to yield 20 mg (20 %) of compound **29** in the form of a white solid.

LC/MS/UV (Eclipse Plus C8 column, 3.5 µm, 4.6 x 150 mm; 40°C; 1.0 mL/min, 40 % to 95 % MeOH in water (0.05 % TFA) in 18 minutes); ESI (C₄₆H₇₂N₆O₈, exact mass 836.54) *m*/*z:* 837.5 (MH⁺) and 419.4 (M.2H⁺/2, 100 %), 10.61 min (92.5 %, 210 nm).

¹H NMR: (400MHz, CD₃OD, *ppm*): δ (Presence of rotamers) 7.38 - 7.15 (m, 6H); 7.00 - 6.99 (m, 3H); 4.85 - 4.68 (m, 2H); 4.37 - 3.38 (m, 11H); 3.31 - 2.70 (m, 8H); 2.60 - 0.82 (m, 35H).

### Example 49

### (S)-2-((S)-2-((4-hydroxyphenethyl)(mthyl)amino)-3-methylbutanamido)-N-((3R,4S,5S)-3-mthoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)-N,3-dimethylbutanamide, trifluoroacetic acid

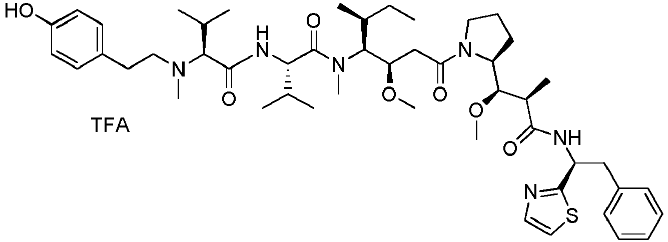

### Example 49A: 2-(4-hydroxyphenyl)acetaldehyde

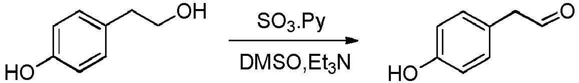

4-(2-hydroxyethyl)phenol (4 g, 28.95 mmol, 1.00 quiv) was dissolved in DMSO (32 mL) and TEA (8.8 mL, 2.20 equiv) was then added dropwise. A solution of SO₃.Py (10 g, 2.20 equiv) in DMSO (36 mL) was added and the mixture was left under agitation overnight at ambient temperature. The reaction mixture was neutralised with 250 mL of water and extracted 3 times with 100 mL of AcOEt. The organic phases were combined, washed 5 times with water (100 mL) then twice with 150 mL of NaCl (sat.), dried over sodium sulfate, filtered and concentrated. The residue was purified on silica gel (EtOAc/PE (1:10) to yield 1 g (25 %) of compound **49A** in the form of a colourless oil.

### Example 49B: benzyl (S)-2-((4-hydroxyphenethyl)(methyl)amino)-3-methyl butanoate

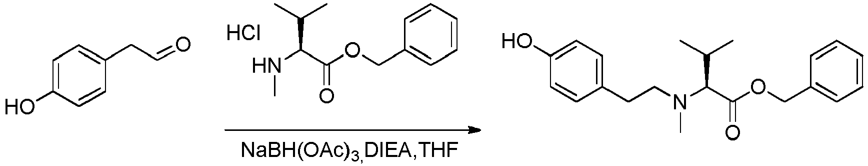

Compound **49B** was synthesised in the same manner as for compound **14C** from the amine **1ZC** (1.5 g, 5.82 mmol, 0.99 equiv), the aldehyde **49A** (800 mg, 5.88 mmol, 1.00 equiv), NaBH(OAc)₃ (2.7 g, 12.74 mmol, 2.17 equiv) and DIEA (4.23 mL) in THF (25 mL). The reaction mixture was neutralised with 50 mL of water and extracted 3 times with 50 mL of AcOEt. The organic phases were combined, dried over sodium sulfate, filtered and concentrated. The residue was purified on silica gel (EtOAc/PE (1:10) to yield 600 mg (37 %) of compound **49B** in the form of a white solid.

### Example 49C: (S)-2-((4-hydroxyphenethyl)(methyl)amino)-3-methylbutanoic acid

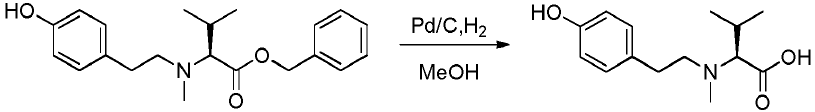

Compound **49B** (0.5 g, 1.46 mmol, 1.00 equiv) was dissolved in 40 mL of MeOH in the presence of Pd/C (250 mg) and hydrogenated for 3 hours at ambient temperature and atmospheric pressure. The reaction medium was filtered and concentrated under reduced pressure to yield 0.4 g of compound **49C** in the form of a white solid.

**Example 49:** Compound **49** was synthesised in the same manner as for compound **3** from the amine **1Y** (53.4 mg, 0.08 mmol, 2.00 equiv), the acid **49C** (70 mg, 0.28 mmol, 1.00 equiv), DEPC (0.032 mL, 2.00 equiv) and DIEA (0.053 mL, 3.00 equiv) in DCM (3 mL). The residue was purified by preparative HPLC (Pre-HPLC-001 SHIMADZU, Atlantis Prep OBD T3 column, 5 µm, 19 x 150 mm; Eluting phase: water / ACN buffered with 0.05 % TFA; Gradient of 20 % to 45 % ACN in 10 minutes then 45 % to 100 % ACN in 2 minutes; Waters 2545 UV Detector at 254 nm and 220 nm), to yield 3 mg (1 %) of compound **49** in the form of a white solid.

LC/MS/UV (Ascentis Express C18 column, 2.7 µm, 4.6 x 100 mm; 40°C; 1.5 mL/min, 10 % to 95 % MeOH in water (0.05 % TFA) in 8 minutes); ESI (C₄₉H₇₄N₆O₇S exact mass 890.5) *m*/*z:* 891.5 (MH⁺) and 446.4 (M.2H⁺/2, 100 %), 6.69 min (100 %, 210 nm).

¹H NMR: (400MHz, CD₃OD, *ppm*): δ (Presence of rotamers) 8.92 - 8.87 (m, 0.5 H, NHCO, incomplete exchange), 8.70 - 8.63 (m, 0.4 H, NHCO, incomplete exchange), 8.85 - 8.77 (m, 1H), 7.59 - 7.51 (m, 1H), 7.35 - 7.03 (m, 7H), 6.82 - 6.71 (m, 2H), 5.77 - 5.58 (m, 1H), 5,81 - 5.70 (m, 1H), 4.21 - 0.80 (m, 58H).

### Example 50

### (S)-2-((2R,3R)-3-((S)-1-((3R,4S,5S)-4-((S)-2-((S)-2-((4-hydroxyphenethyl)(methyl)amino)-3-methylbutanamido)-N,3-dimethylbutanamido)-3-methoxy-5-methylheptanoyl)pyrrolidin-2-yl)-3-methoxy-2-methylpropanamido)-3-phenylpropanoic acid, trifluoroacetic acid

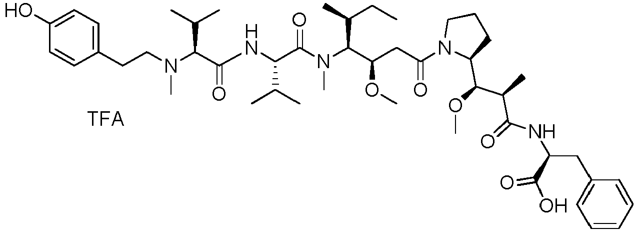

**Example 50:** Compound **50** was prepared in the same manner as for compound **4,** from compound **27** (100 mg, 0.10 mmol, 1.00 equiv). The residue was purified by preparative HPLC (Pre-HPLC-001 SHIMADZU, Atlantis Prep OBD T3 column, 5 µm, 19 x 150 mm; Eluting phase: water / ACN buffered with 0.05 % TFA; Gradient of 20 % to 40 % ACN in 10 minutes then 40 % to 100 % ACN in 2 minutes; Waters 2545 UV Detector at 254 nm and 220 nm), to yield 10.7 mg (11 %) of compound **50** in the form of a white solid.

LC/MS/UV (Ascentis Express C18 column, 2.7 µm, 4.6 x 100 mm; 40°C; 1.5 mL/min, 10 % to 95 % MeOH in water (0.05 % TFA) in 8 minutes); ESI (C₄₇H₇₃N₅O₉, exact mass 851.5) *m*/*z:* 852.5 (MH⁺) and 426.8 (M.2H⁺/2, 100 %), 6.46 min (91.7 %, 210 nm).

¹H NMR: (400MHz, CD₃OD, *ppm*): δ (Presence of rotamers) 7.34 - 7.15 (m, 5H); 7.15 - 7.04 (se, 2H), 6.82 - 6.83 (m, 2H), 4.83 - 4.70 (m, 1H), 4.21 - 4.00 (m, 1H), 3.90 - 3.80 (m, 1H), 3.74 - 3.62 (m, 1H), 3.57 - 2.86 (m, 20H), 2.56 - 0.80 (m, 36H).

### Example 51

### methyl (S)-2-((2R,3R)-3-((S)-1-((3R,4S,5S)-4-((S)-2-((S)-2-((4-hydroxybenzyl)(methyl)amino)-3-methylbutanamido)-N,3-dimethylbutanamido)-3-methoxy-5-methylheptanoyl)pyrrolidin-2-yl)-3-methoxy-2-methylpropanamido)-3- phenylpropanoate, trifluoroacetic acid

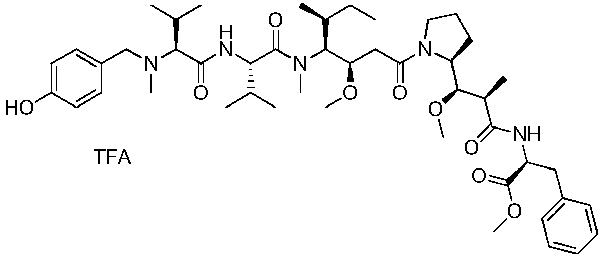

### Example 51A: tert-butyl (4-formylphenyl)carbonate

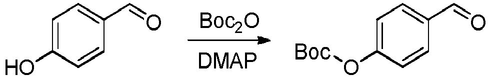

4-hydroxybenzaldehyde (3.0 g, 24 mmol) was dissolved in 30 mL of DCM in the presence of 4-DMAP (300 mg, 2.46 mmol, 0.1 equiv.) and di-*tert*-butyl dicarbonate (5.35 g, 24 mmol, 1.0 equiv.) and agitated 1 hour at ambient temperature. The solution was then diluted with 200 mL of water and extracted 3 times with 100 mL of DCM. The organic phases were combined, dried over sodium sulfate, filtered and concentrated under reduced pressure to yield 5 g (92 %) of compound **51A** in the form of a white solid.

### Example 51B: benzyl (S)-2((4-((tert-butoxycarbonyl)oxy)benzyl)(methyl) amino)-3-methylbutanoate

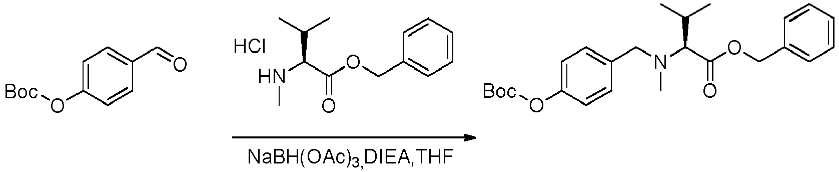

Compound **51A** (220 mg, 0.99 mmol) was dissolved in 5 mL of THF in the presence of compound **1ZC** (255 mg, 0.99 mmol, 1.0 equiv.), NaBH(OAc)₃ (420 mg, 2 mmol, 2.0 equiv.) and DIEA (654 µl) and agitated overnight at ambient temperature. The solution was then diluted with 100 mL of water and extracted 3 times with 50 mL of EtOAc. The organic phases were combined, dried over sodium sulfate, filtered and concentrated under reduced pressure. The residue was purified on a silica column with a mixture of EtOAc and PE (1:100) to yield 200 mg (47 %) of compound **51B** in the form of a white solid.

### Example 51C: (S)-2-((4-((tert-butoxycarbonyl)oxy)benzyl)(methyl)amino)-3-methyl butanoic acid

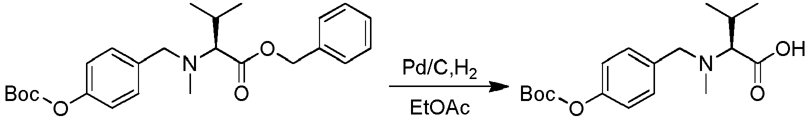

Compound **51C** was prepared by hydrogenation of compound **51B** (200 mg), following the protocol used for the preparation of compound **3F.**

### Example 51D: methyl (S)-2-((2R,3R)-3-((S)-1-((3R,4S,5S)-4-((S)-2-((S)-2-((4-((tert-butoxycarbonyl)oxy)benzyl)(methyl)amino)-3-methylbutanamido)-N,3-dimethylbutanamido)-3-methoxy-5-methylheptanoyl)pyrrolidin-2-yl)-3-methoxy-2-methylpropanamido)-3-phenylpropanoate

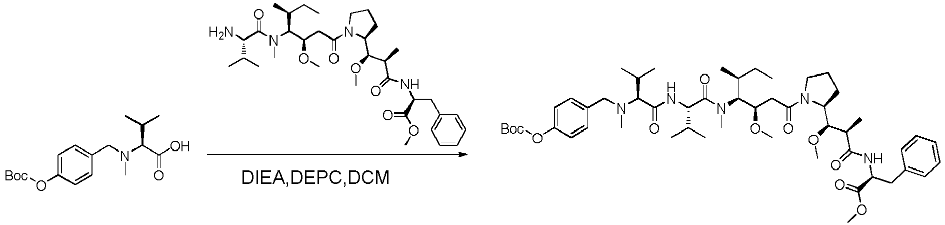

Compound **51D** was prepared by coupling compound **51C** with amine **3D,** following the protocol used for the preparation of compound **3** to obtain the desired product in the form of yellow oil with a yield of 60%.

**Example 51:** Compound **51D** (80 mg, 0.08 mmol) was dissolved in 1 mL of DCM in the presence of 0.5 mL TFA, agitated 2 hours at ambient temperature and then concentrated under reduced pressure. The residue was purified by preparative HPLC (Pre-HPLC-010, SunFire Prep C18 OBD column, 5 µm, 19 x 150 mm; Eluting phase: water / ACN buffered with 0.05 % TFA; Gradient of 23 % to 40 % ACN in 10 minutes then 40 % to 95 % ACN in 2 minutes; Waters 2489 UV Detector at 254 nm and 220 nm). Compound **51** was obtained with a yield of 24 % (20 mg) in the form of a white solid.

LC/MS/UV (Zorbax SB-Aq, 1.8 µm, 4.6 x 100 mm; 2 % MeOH in water (0.05 % TFA) for 1 minute then 2 % to 95 % MeOH in 13 minutes); ESI (C₄₇H₇₃N₅O₉, exact mass 851.54) *m*/*z*: 874.5 (MNa⁺), 426.9 (M.2H⁺/2); 12.48 min (96 %, 210 nm).

¹H NMR: (300MHz, CD₃OD, *ppm*): δ (Presence of rotamers) 8.1 - 8.6 (m, 0.9H, NHCO incomplete exchange); 7.29 - 7.27 (m, 2H), 7.25 - 6.86 (m, 5H), 6.84 - 6.83 (m, 2H), 4.83 - 4.72 (m, 3H), 4.26 - 0.82 (m, 58H).

### Example 61

### (S)-2-((S)-2-((4-aminophenethyl)(methyl)amino)-3-methylbutanamido)-N-((3R,4S,5S)-3-methoxy-1-((S)-2-((lR,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)-N,3-dimethylbutanamide

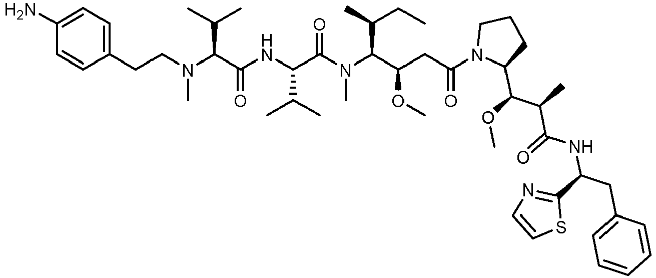

### Example 61A: N-(4-aminophenethyl)-N-methyl-L-valine dihydrochloride

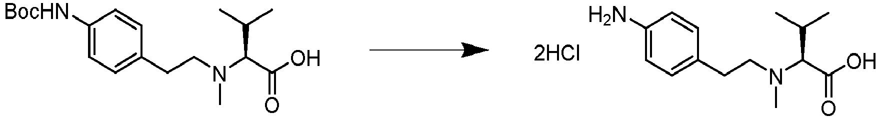

Compound **11D** (962 mg, 2.75 mmol) was dissolved in 10 ml of a commercially available solution of HCl in propan-2-ol (5 - 6 M), and stirred at room temperature for 2 hours. TLC analysis indicated complete consumption of starting material. The solvent was evaporated under reduced pressure, and the resulting yellow solid triturated with Et₂O (2 x 10 ml). The product was dried under vacuum to furnish compound **61A** as a yellow solid (322 mg, 47 %).

**Example 61:** Carboxylic acid **61A** (73 mg, 0.23 mmol, 1 eq.) and amine **1Y** (150 mg, 0.23 mmol, 1 eq.) were dissolved in dry DMF (2 ml). DIEA (158 µl, 0.90 mmol, 4 eq.) and DECP (51 µl, 0.34 mmol, 1.5 eq.) were added and the reaction stirred for 4 hours at room temperature. Analysis by LC-MS showed complete consumption of the starting material. The solvent was evaporated under reduced pressure, and the residue purified by flash chromatography on silica gel (DCM/MeOH) to furnish compound **61** as a light yellow solid (83 mg, 40 %).

¹H NMR: (500MHz, DMSO-*d*₆, *ppm*): δ (Presence of rotamers), 8.86 (d, 0.5H, NHCO); 8.65 (d, 0.5H, NHCO), 8.11-8.05 (m, 1H, NHCO), 7.80 (d, 0.5H, thiazole), 7.78 (d, 0.5H, thiazole), 7.65 (d, 0.5H, thiazole), 7.63 (d, 0.5H, thiazole), 7.32 - 7.12 (m, 5H), 6.83 (d, *J*=8.3 Hz, 2H), 6.45 (d, *J*=8.3 Hz, 2H), 5.56 - 5.49 (m, 0.5 H), 5.42 - 5.35 (m, 0.5H), 4.78 (s, 2H, NH₂), 4.74 - 4.46 (m, 2H), 4.01 - 0.66 (m, 57H).

HPLC (Xbridge Shield C18, 3.5 µm, 4.6 x 50 mm ; 3.5 ml/min, 40°C, 0 to 95 % MeCN in water (0.1 % TFA) in 2.25 minutes then 95 % MeCN for 0.5 minutes, Tr = 1.31 min (96.5 %, 220 nm).

*m*/*z* (Q-TOF ESI⁺) 890.5558 (2%, MH⁺, C₄₉H₇₆N₇O₆S requires 890.5572), 445.7834 (100 %, (MH₂)²⁺, C₄₉H₇₇N₇O₆S requires 445.7823).

### Example 62

### Methyl ((2R,3R)-3-((S)-1-((3R,4S,5S)-4-((S)-2-((S)-2-((4-aminophenethyl)(methyl)amino)-3-methylbutanamido)-N,3-dimethylbutanamido)-3-methoxy-5-methylheptanoyl)pyrrolidin-2-yl)-3-methoxy-2-methylpropanoyl)-L-phenylalaninate

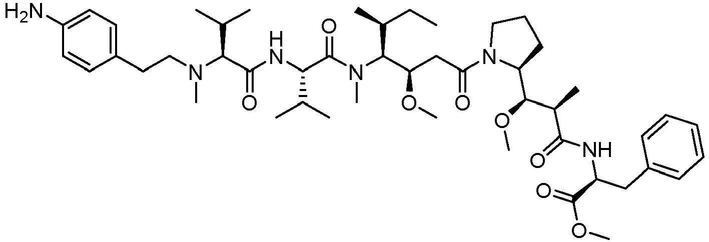

**Example 62:** Compound **62** was prepared in the same manner as for compound **61,** using carboxylic acid **61A** (69 mg, 0.21 mmol, 1 eq.), amine **3D** (135 mg, 0.21 mmol, 1 eq.), DIEA (75 µl, 0.43 mmol, 2 eq.) and DECP (49 µl, 0.32 mmol, 1.5 eq.). The crude product was purified by flash chromatography on silica gel (DCM/MeOH) to furnish compound **62** as a yellowish solid (82 mg, 45 %).

¹H NMR: (500MHz, DMSO-*d*₆, *ppm):* δ (Presence of rotamers), 8.50 (d, *J*=8.3, 0.5H, NHCO); 8.27 (d, *J*=8.0, 0.5H, NHCO), 8.15-8.04 (m, 1H, NHCO), 7.27 - 7.13 (m, 5H), 6.86 - 6.79 (m, 2H), 6.48 - 6.42 (m, 2H), 4.78 (s, 2H, NH₂), 4.74 - 4.44 (m, 3H), 4.01 - 3.72 (m, 1.5H), 3.66 (s, 1.5H, CO₂Me) 3.63 (s, 1.5H, CO₂Me) 3.57 - 0.65 (m, 55.5H).

HPLC (Xbridge Shield C18, 3.5 µm, 4.6 x 50 mm ; 3.5 ml/min, 40°C, 0 to 95 % MeCN in water (0.1 % TFA) in 2.25 minutes then 95 % MeCN for 0.5 minutes, Tr = 1.29 min (95.3 %, 220 nm).

*m*/*z* (Q-TOF ESI⁺) 865.5800 (2%, MH⁺, C₄₈H₇₇N₆O₈ requires 865.5797), 433.2937 (100 %, (MH₂)²⁺, C₄₈H₇₈N₆O₈ requires 433.2935).

### Example 63

### ((2R,3R)-3-((S)-1-((3R,4S,5S)-4-((S)-2-((S)-2-((4-aminophenethyl)(methyl)amino)-3-methylbutanamido)-N,3-dimethylbutanamido)-3-methoxy-5-methylheptanoyl)pyrrolidin-2-yl)-3-methoxy-2-methylpropanoyl)-L-phenylalanine 2,2,2-trifluoroacetate

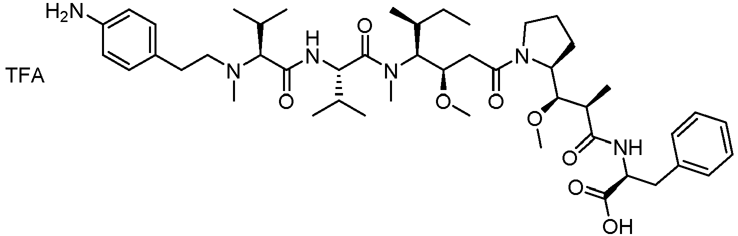

**Example 63:** Compound **62** (23 mg, 0.03 mmol) was dissolved in a mixture of water (1 ml) and acetonitrile (1 ml). Piperidine (0.75 ml) was added and the mixture stirred at room temperature for 5 hours. TLC analysis indicated complete consumption of the starting material. The solvent was evaporated under reduced pressure, and the residue purified by preparative HPLC (SunFire Prep column C18 OBD, 5 µm, 19 x 150 mm; Mobile phase: water/MeCN buffered with 0.1 % TFA; Gradient of 20 % to 40 % MeCN in 10 minutes, then from 40 % to 100 % MeCN in 2 minutes; Detector UV Waters 2545 at 254 nm et 220 nm). Compound **63** was obtained as a white solid (14 mg, 66 %).

¹H NMR: (500MHz, DMSO-*d*₆, *ppm*): δ (Presence ofrotamers), 12.7 (s(br), 1H, CO₂H), 9.58 (m(br), 1H); 9.04 - 8.89 (m, 1H), 8.41 (d, 0.6H, NHCO), 8.15 (d, 0.4H, NHCO), 7.27 - 7.13 (m, 5H), 7.13 - 6.99 (m(br), 2H), 6.90 - 6.64 (s(br), 2H), 4.77 - 3.40 (m, 10H), 3.34 - 2.75 (m, 20H), 2.34 - 1.94 (m, 4H), 1.90 - 0.7 (m, 25H).
HPLC (Xbridge Shield C18, 3.5 µm, 4.6 x 50 mm ; 3.5 ml/min, 40°C, 0 to 95 % MeCN in water (0.1 % TFA) in 2.25 minutes then 95 % MeCN for 0.5 minutes, Tr = 1.24 min (100 %, 220 nm).

*m*/*z* (Q-TOF ESI⁺) 851.5641 (6%, MH⁺, C₄₇H₇₅N₆O₈ requires 851.5641), 426.2854 (100 %, (MH₂)²⁺, C₄₇H₇₆N₆O₈ requires 426.2857).

### Example 64

### (S)-2-((S)-2-((4-aminophenethyl)(methyl)amino)-3-methylbutanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide

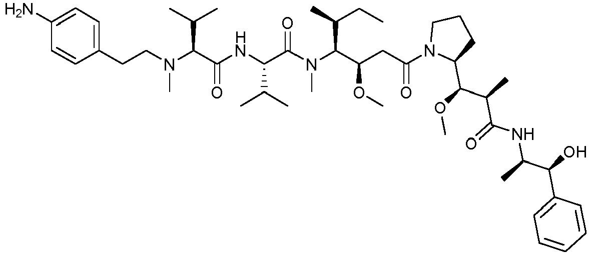

Compound **64** was prepared in the same manner as for compound **61,** using carboxylic acid **61A** (93 mg, 0.29 mmol, 1 eq.), amine **2D** (174 mg, 0.29 mmol, 1 eq.), DIEA (100 µl, 0.58 mmol, 2 eq.) and DECP (66 µl, 0.43 mmol, 1.5 eq.). The crude product was purified by flash chromatography on silica gel (DCM/MeOH) to furnish compound **64** as an off-white solid (51 mg, 21 %).

¹H NMR: (500MHz, DMSO-*d*₆, *ppm*): δ (Presence of rotamers), 9.61 (m(br), 1H); 9.05 - 8.89 (m, 1H), 7.93 (d, 0.6H, NHCO), 7.64 (d, 0.4H, NHCO), 7.36 - 6.98 (m, 7H), 6.92 - 6.70 (m(br), 2H), 5.45 (s(br), 1H), 4.80 - 4.41 (m, 3H), 4.06 - 3.44 (m, 4H), 3.37 - 2.79 (m, 18H), 2.45 - 2.21 (m, 3H), 2.17 - 0.70 (m, 35H).

HPLC (Xbridge Shield C18, 3.5 µm, 4.6 x 50 mm ; 3.5 ml/min, 40°C, 0 to 95 % MeCN in water (0.1 % TFA) in 2.25 minutes then 95 % MeCN for 0.5 minutes, Tr = 1.20 min (100 %, 220 nm).

*m*/*z* (Q-TOF ESI⁺) 837.5826 (33%, MH⁺, C₄₇H₇₇N₆O₇ requires 837.5848), 419.2956 (100 %, (MH₂)²⁺, C₄₇H₇₆N₆O₈ requires 419.2961).

### II - Biological activity of the compounds of the invention

### Method:

**Cell culture.** A549 (Non Small Cell Lung Cancer - ATCC CCL-185) and MDA-MB-231 (breast adenocarcinoma - ATCC HTB-26) cells were cultured in Minimum Essential Medium Eagle (MEM) with 5% fetal calf serum (FCS) and Dulbecco's modified Eagle Medium (DMEM) with 10% FCS respectively. MCF7 (breast ductal carcinoma - ATCC HTB-22) and SN-12C (kidney carcinoma - ATCC) cells were maintained in RPMI1640 medium (without phenol red for MCF7 cells) containing 10% FCS. All the media were supplemented with fungizone (1.25 µg/mL) and penicillin-streptomycin (100 U / 100 µg/mL). Cells were cultured under standard conditions in an incubator at 37°C, 5% CO₂ and 95% atmospheric humidity.

**Antiproliferative activity on 4 tumor cell lines.** Compounds according to the invention were investigated for their antiproliferative activity using an ATPlite proliferation assay (Perkin Elmer, Villebon sur Yvette, France) on a comprehensive panel of 4 cell lines. Cells were seeded in 96 well plates (10³ cells/well for A549, 2.10³ for MCF7, MDA-MB-231 and SN12C) at day 0 at a concentration to ensure cells remained in logarithmic cell growth phase throughout the 72 h drug treatment period. After a 24h incubation period, all the cells were treated with serial dilutions of the tested compounds (11µL of a 10X solution in 1% DMSO - 6 wells/ condition). To avoid adherence of the compounds onto the tips, tips were changed between two consecutive dilutions. Cells were then placed in 37°C, 5% CO₂ incubator. On day 4, cell viability was evaluated by dosing the ATP released by viable cells. The number of viable cells was analyzed in comparison with the number of solvent treated cells. The EC₅₀ values were determined with curve fitting analysis (non linear regression model with a sigmoidal dose response, variable hill slope coefficient), performed with the algorithm provided by the GraphPad Software (GraphPad Software Inc., CA, USA).

### Results:

### Various compounds:

Various compounds according to the invention were tested to determine their antiproliferative activity on the MDA-MB-231 cell line following the above-described method. The measured activities gave values of EC₅₀ < 0.1 µM.

The few following examples chosen from among the compounds according to the invention illustrate their fully remarkable antiproliferative properties:
Example 12: EC₅₀ = 5.80x10⁻¹⁰ M; Example 13: EC₅₀ = 7.95x10⁻⁸ M; Example 15: EC₅₀ = 1.70x10⁻¹⁰ M; Example 27: EC₅₀ = 1.20x10⁻¹⁰ M.

### Various cell lines:

Compound 15 was tested on different cell lines (A549, MDA-MB-231, MCF-7, SN12C) following the above-described method. The measured activities gave values of EC₅₀ < 0.1µM.

| **EC₅₀ (M)** | **A549** | **MDA-MB-231** | **MCF-7** | **SN12C** |
|---|---|---|---|---|
| Compound **15** | 1.45x10⁻¹⁰ | 1.70x10⁻¹⁰ | 7.15x10⁻¹⁰ | 2.18x10⁻¹⁰ |

### Comparative examples:

The substitution on the phenyl ring (amino/hydroxyl v. carboxyl) was studied in the comparative examples below showing the improved antiproliferative activity of the drugs according to the invention comprising an amino or hydroxyl substituent in comparison to a drug comprising a carboxyl substituent according to the prior art (WO 2012/041805 and WO 2012/123423).

| **N°** | **Structure** | **EC₅₀ (M)** | |
|---|---|---|---|
| | | **A549** | **MDA-MB-231** |
| 12 | 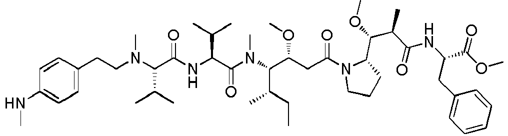 | 1.48x10⁻¹⁰ | 5.80x10⁻¹⁰ |
| 15 | 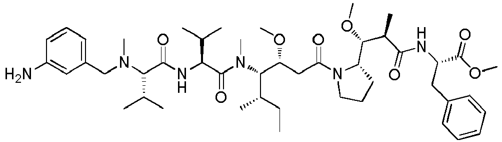 | 1.45x10⁻¹⁰ | 1.70x10⁻¹⁰ |
| 27 | 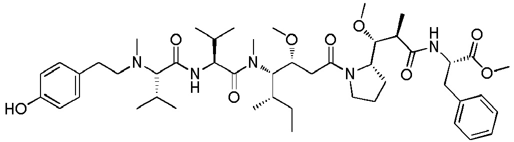 | 8.60x10⁻¹¹ | 1.20x10⁻¹⁰ |
| Comparative example | 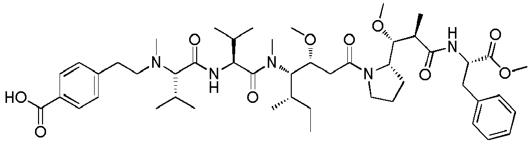 | 3.76x10⁻⁹ | 2.29x10⁻⁹ |
| 13 | 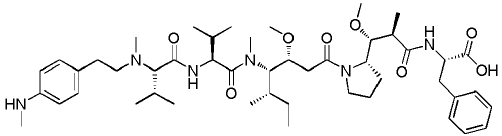 | 2.71x10⁻⁸ | 7.95x10⁻⁸ |
| Comprative example | 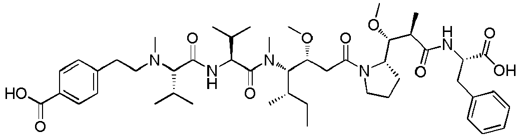 | 4.03x10⁻⁷ | 9.75x10⁻⁷ |

## Claims

1. A compound of following formula (I): 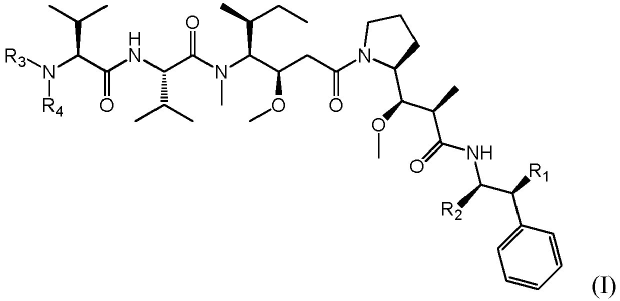 where:
- R₁ = OH and R₂ = methyl, or
R₁ = H and R₂ = COOH, COOMe or thiazol-2-yl,
- R₃ is a (C₁-C₆)alkyl group, and
- R₄ is a phenyl-(C₁-C₂)alkyl group substituted by one group chosen from among OH and NR₉R₁₀ groups with R₉ and R₁₀ each independently of one another representing H or a (C₁-C₆)alkyl group, wherein the phenyl-(C₁-C₂)alkyl group is linked to the remainder of the molecule via its (C₁-C₂)alkyl moiety,
or a pharmaceutically acceptable salt, hydrate or solvate thereof.

2. The compound according to claim 1, **characterized in that** R₁ represents H and R₂ represents COOH or COOMe.

3. The compound according to any one of claims 1 and 2, **characterized in that** R₃ represents a methyl group.

4. The compound according to any one of claims 1 to 3, **characterized in that** R₄ represents a phenyl-(C₁-C₂)alkyl group substituted by one group on the phenyl moiety chosen from among OH and NR₉R₁₀.

5. The compound according to claim 4, **characterized in that** R₄ represents a phenyl-(C₁-C₂)alkyl group substituted by one NR₉R₁₀ group on the phenyl moiety.

6. The compound according to any one of claims 1 to 3, **characterized in that** R₄ has the following formula: 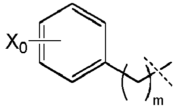 wherein X₀ represents OH or NR₉R₁₀ and m is 1 or 2.

7. The compound according to claim 6, **characterized in that** X₀ is NR₉R₁₀.

8. The compound according to any one of claims 1 to 3, **characterized in that** R₄ has the following formula: 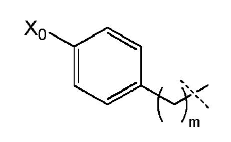 wherein X₀ represents NR₉R₁₀ and m represents 1 or 2.

9. The compound according to claim 1, chosen from among: 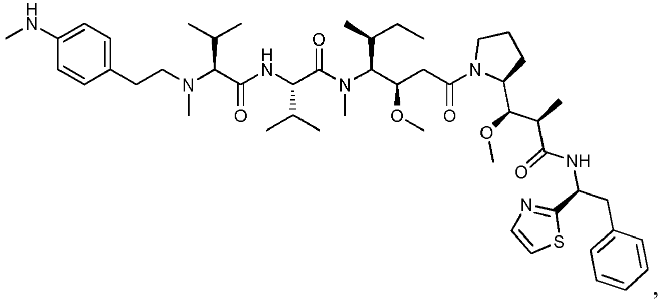 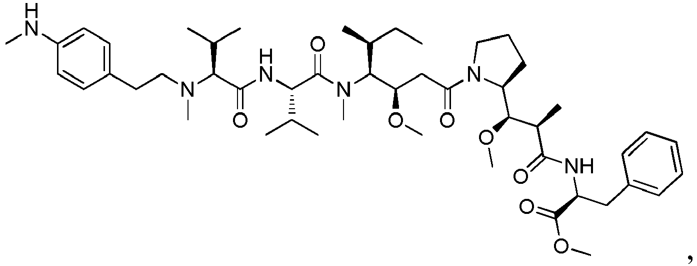 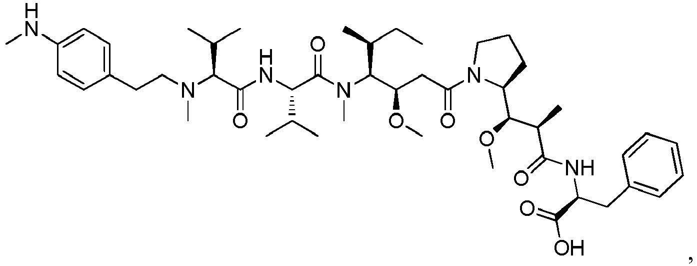 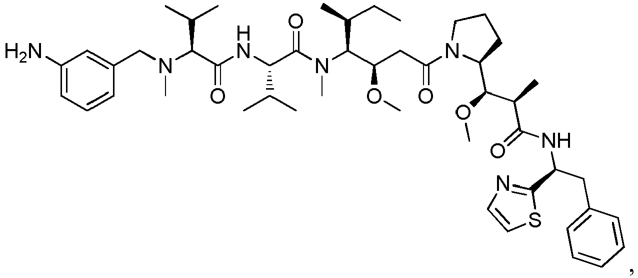 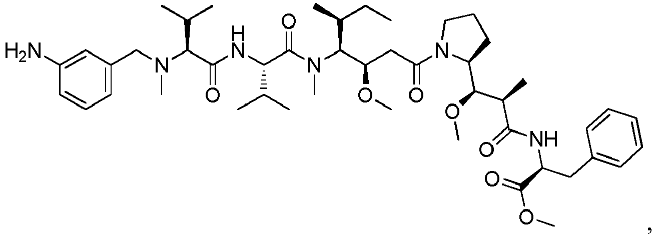 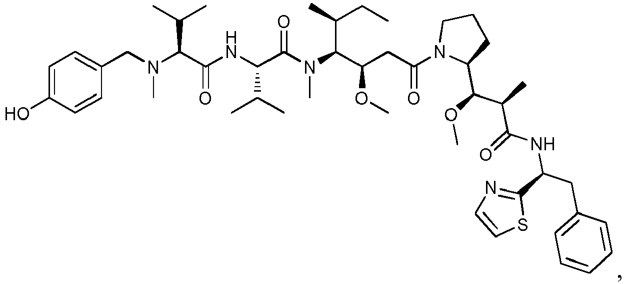 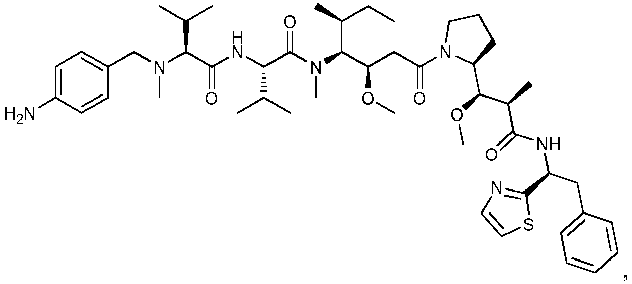 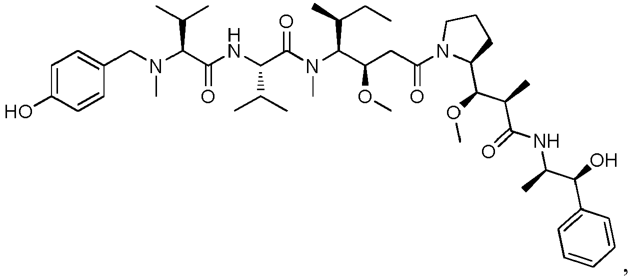 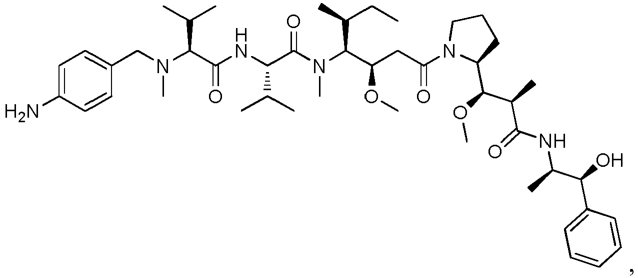 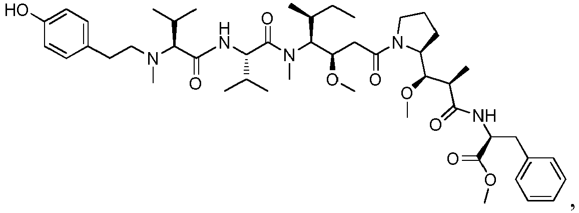 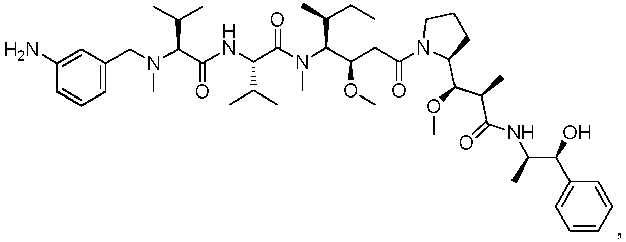 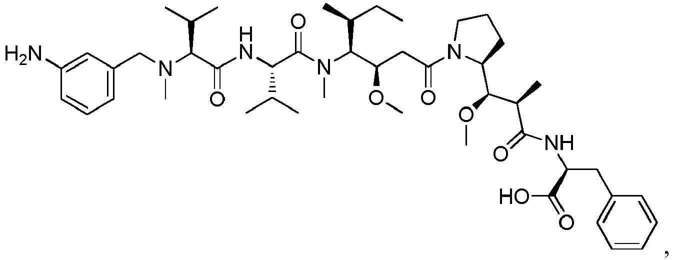 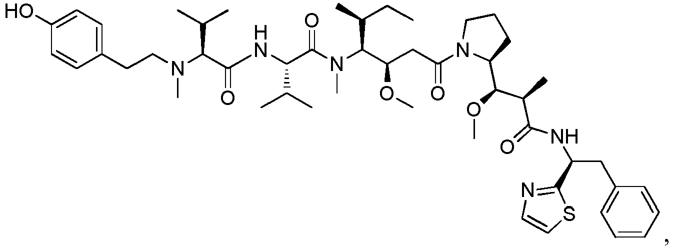 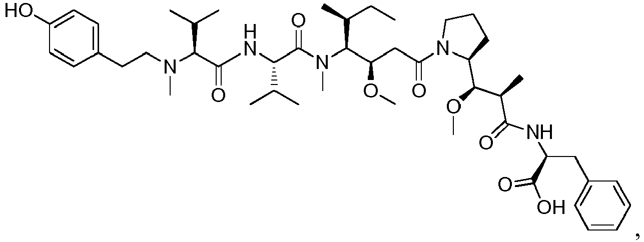 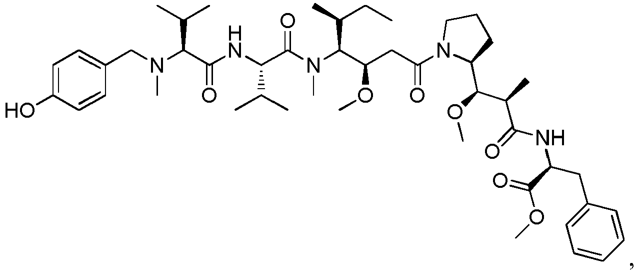 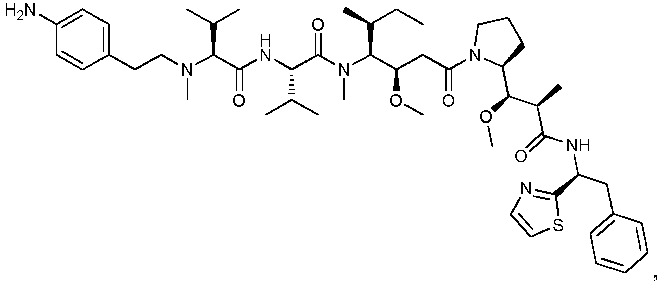 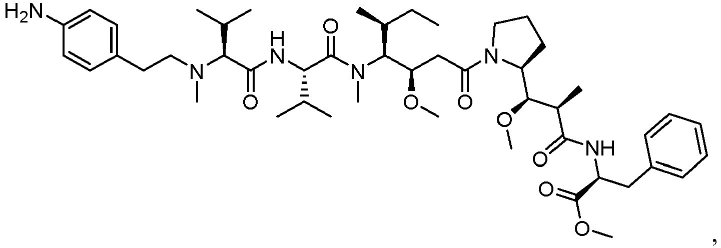 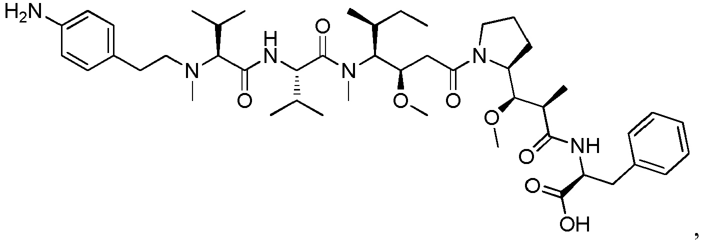 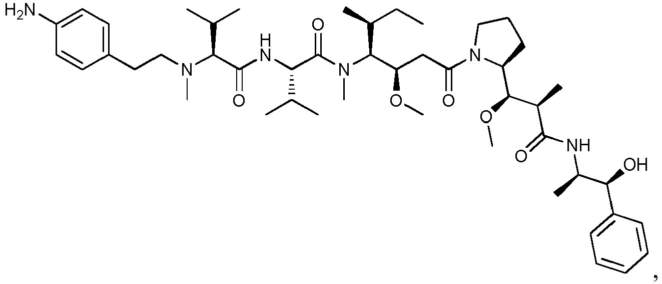 and the pharmaceutically acceptable salts thereof such as the salts formed with trifluoroacetic acid.

10. A compound according to any one of claims 1 to 9 for use as a medicinal product.

11. A compound according to any one of claims 1 to 9 for use as medicinal product intended for the treatment of cancer or benign proliferative disorders.

12. A pharmaceutical composition comprising a formula (I) compound according to any one of claims 1 to 9 and at least one pharmaceutically acceptable excipient.

13. The pharmaceutical composition according to claim 12, further comprising another active ingredient, advantageously chosen from among anticancer agents, in particular comprising cytotoxic anticancer agents such as navelbine, vinflunine, taxol, taxoter, 5-fluorouracil, methotrexate, doxorabicin, camptothecin, gemcitabin, etoposide, cis-platin or carmustin; and hormonal anticancer agents such as tamoxifen or medroxyprogesterone.

14. A method for preparing a formula (I) compound according to any one of claims 1 to 9 comprising a condensation reaction between a compound of following formula (VI): 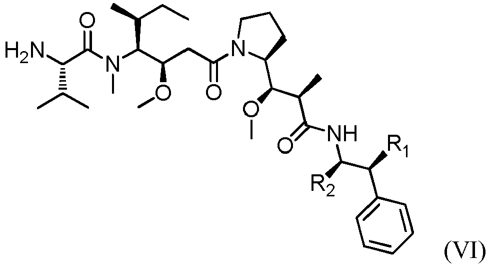
where R₁ and R₂ are as defined in claim 1, and
a compound of following formula (VII): 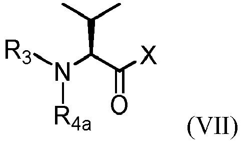
where R₃ is as defined in claim 1, R₄ₐ represents an R₄ group as defined in claim 1, optionally in protected form, and X is OH or Cl.

15. A method for preparing a formula (I) compound according to any one of claims 1 to 9 comprising a substitution reaction between a compound of following formula (VIII): 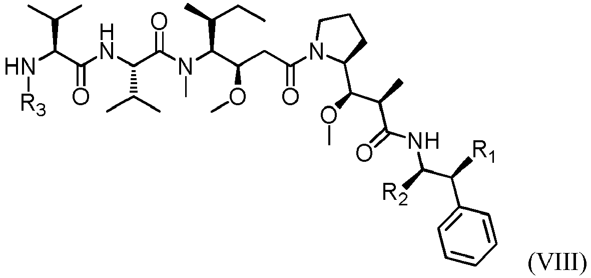
where R₁, R₂ and R₃ are as defined in claim 1, and
a compound of following formula (X):
R₄ₐ-Y (X)
where R₄ₐ represents an R₄ group as defined in claim 1 optionally in protected form, and
Y is a leaving group such as Cl, Br, I, OSO₂CH₃, OSO₂CF₃ or O-Tosyl.

16. A method for preparing a formula (I) compound according to any one of claims 1 to 9 where R₄ represents a -CH₂R_{4b} group with R_{4b} representing a phenyl or phenyl-methyl group substituted by one group chosen from among OH and NR₉R₁₀ groups,
comprising a reductive amination reaction between a compound of following formula (VIII): 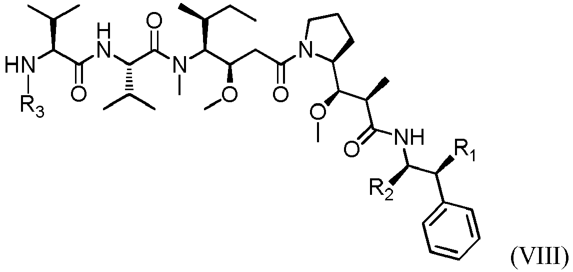
where R₁, R₂ and R₃ are as defined in claim 1, and
a compound of following formula (XI):
R_{4b}-CHO (XI)
where R_{4b} is as previously defined.

## Patentansprüche

1. Verbindung der folgenden Formel (I): 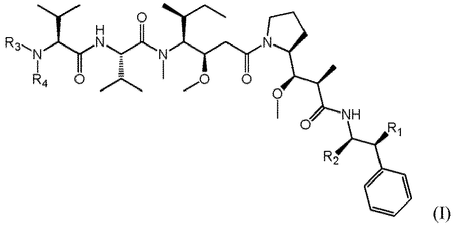 worin:
- R₁ = OH und R₂ = Methyl sind, oder
R₁ = H und R₂ = COOH, COOMe oder Thiazol-2-yl sind,
- R₃ eine (C₁-C₆) -Alkylgruppe ist und
- R₄ eine Phenyl- (C₁-C₂) -Alkylgruppe ist, die durch eine Gruppe substituiert ist, die ausgewählt ist von OH- und NR₉R₁₀-Gruppen, wobei R₉ und R₁₀ jeweils unabhängig voneinander für H oder eine (C₁-C₆)-Alkylgruppe stehen, wobei die Phenyl-(C₁-C₂)-Alkylgruppe mit dem Rest des Moleküls über die (C₁-C₂)-Alkyleinheit verknüpft ist,
oder ein pharmazeutisch verträgliches Salz, Hydrat oder Solvat davon.

2. Verbindung nach Anspruch 1, **dadurch gekennzeichnet, dass** R₁ für H steht und R₂ für COOH oder COOMe steht.

3. Verbindung nach einem der Ansprüche 1 und 2, **dadurch gekennzeichnet, dass** R₃ für eine Methylgruppe steht.

4. Verbindung nach einem der Ansprüche 1 bis 3, **dadurch gekennzeichnet, dass** R₄ für eine Phenyl-(C₁-C₂)-Alkylgruppe steht, die durch eine Gruppe auf der Phenyleinheit substituiert ist, die ausgewählt ist aus OH und NR₉R₁₀.

5. Verbindung nach Anspruch 4, **dadurch gekennzeichnet, dass** R₄ für eine Phenyl-(C₁-C₂)-Alkylgruppe steht, die durch eine NR₉R₁₀-Gruppe auf der Phenyleinheit substituiert ist.

6. Verbindung nach einem der Ansprüche 1 bis 3, **dadurch gekennzeichnet, dass** R₄ die folgende Formel hat: 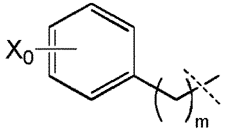 worin X₀ für OH oder NR₉R₁₀ steht und m 1 oder 2 ist.

7. Verbindung nach Anspruch 6, **dadurch gekennzeichnet, dass** X₀ NR₉R₁₀ ist.

8. Verbindung nach einem der Ansprüche 1 bis 3, **dadurch gekennzeichnet, dass** R₄ die folgende Formel hat: 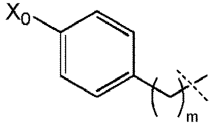 worin X₀ für NR₉R₁₀ steht und m 1 oder 2 ist.

9. Verbindung nach Anspruch 1, ausgewählt aus: 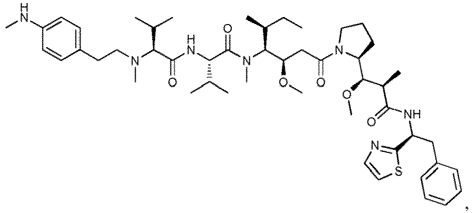 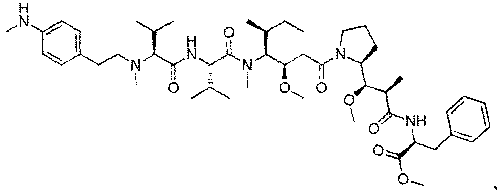 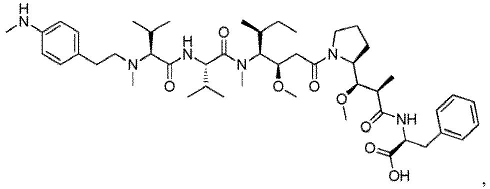 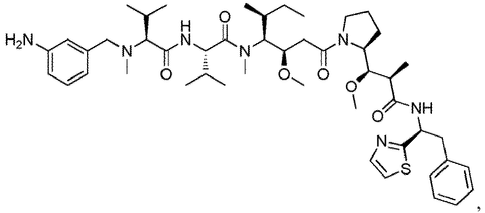 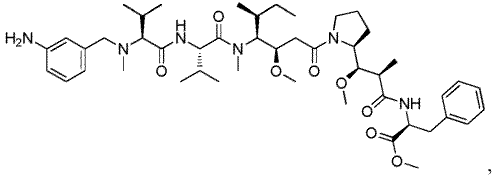 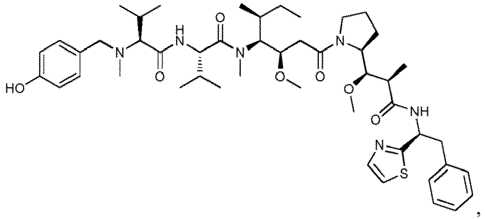 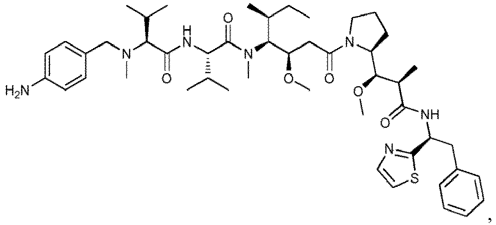 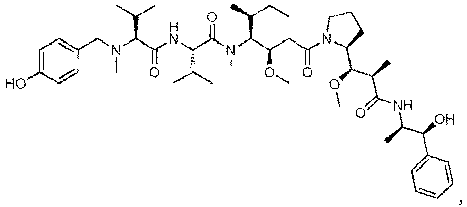 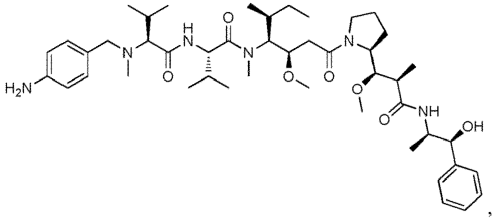 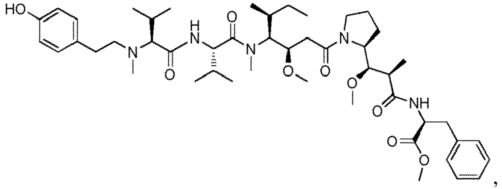 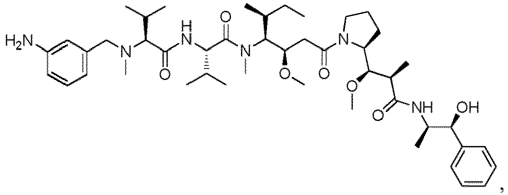 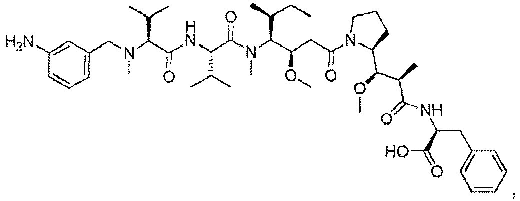 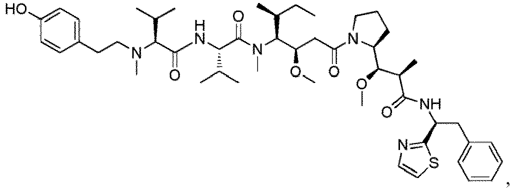 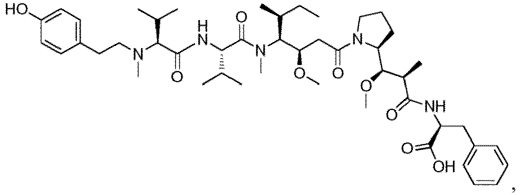 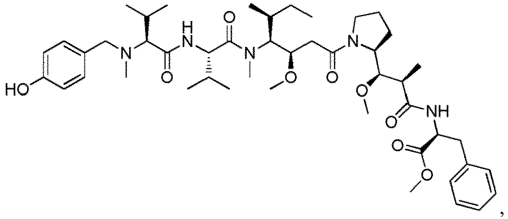 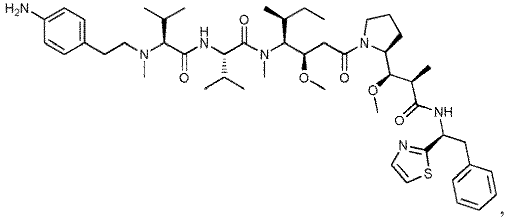 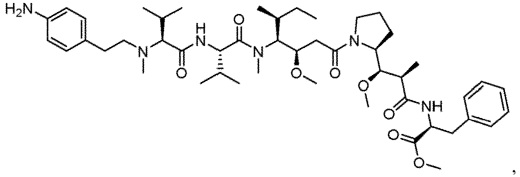 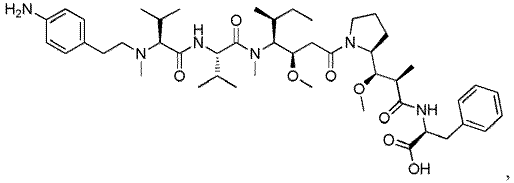 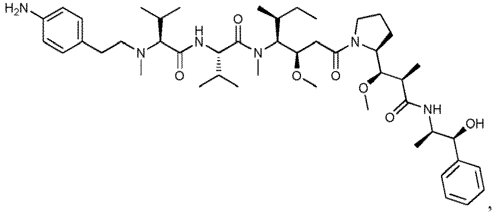 und den pharmazeutisch verträglichen Salzen davon, wie die mit Trifluoressigsäure gebildeten Salze.

10. Verbindung nach einem der Ansprüche 1 bis 9 für die Verwendung als ein medizinisches Produkt.

11. Verbindung nach einem der Ansprüche 1 bis 9 für die Verwendung als medizinisches Produkt für die Behandlung von Krebs oder gutartigen proliferativen Störungen.

12. Pharmazeutische Zusammensetzung, umfassend eine Formel (I)-Verbindung nach einem der Ansprüche 1 bis 9 und mindestens einen pharmazeutisch verträglichen Trägerstoff.

13. Pharmazeutische Zusammensetzung nach Anspruch 12, ferner umfassend einen weiteren Wirkstoff, der vorteilhafterweise ausgewählt ist aus Antikrebsmitteln, insbesondere zytotoxischen Antikrebsmitteln wie Navelbin, Vinflunin, Taxol, Taxoter, 5-Fluorouracil, Methotrexat, Doxorabicin, Camptothecin, Gemcitabin, Etoposid, Cis-Platin oder Carmustin; und hormonelle Antikrebsmittel wie Tamoxifen oder Medroxyprogesteron.

14. Verfahren zur Herstellung einer Formel (I)-Verbindung nach einem der Ansprüche 1 bis 9, umfassend eine Kondensationsreaktion zwischen einer Verbindung der folgenden Formel (VI): 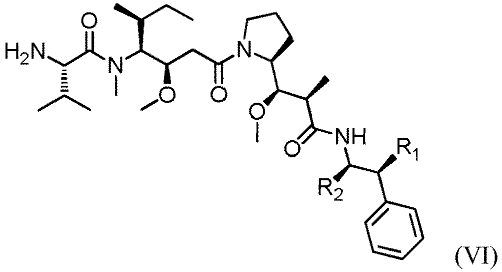
worin R₁ und R₂ wie in Anspruch 1 definiert sind, und
eine Verbindung der folgenden Formel (VII): 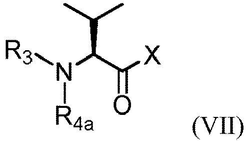
worin R₃ wie in Anspruch 1 definiert ist, R₄ₐ für eine R₄-Gruppe wie in Anspruch 1 definiert steht, wahlweise in geschützter Form und X OH oder Cl ist.

15. Verfahren zur Herstellung einer Formel (I)-Verbindung nach einem der Ansprüche 1 bis 9, umfassend eine Substitutionsreaktion zwischen einer Verbindung der folgenden Formel (VIII): 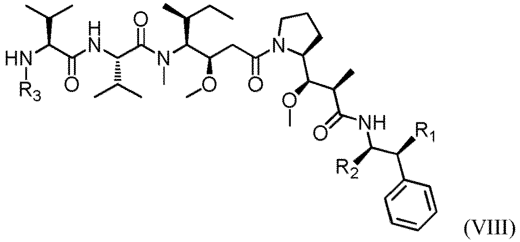
worin R₁, R₂ und R₃ wie in Anspruch 1 definiert sind, und
eine Verbindung der Formel (X):
R₄ₐ-Y (X)
worin R₄ₐ für eine R₄-Gruppe wie in Anspruch 1 definiert steht, wahlweise in geschützter Form, Y eine Abgangsgruppe wie Cl, Br, I, OSO₂CH₃, OSO₂CF₃ oder O-Tosyl ist.

16. Verfahren zur Herstellung einer Formel (I)-Verbindung nach einem der Ansprüche 1 bis 9, worin R₄ für eine -CH₂R_{4b}-Gruppe steht, worin R_{4b} für eine Phenyl- oder Phenylmethylgruppe steht, die durch eine Gruppe substituiert ist, die ausgewählt ist aus OH- und NR₉R₁₀-Gruppen,
umfassend eine reduktive Aminierungsreaktion zwischen einer Verbindung der folgenden Formel (VIII): 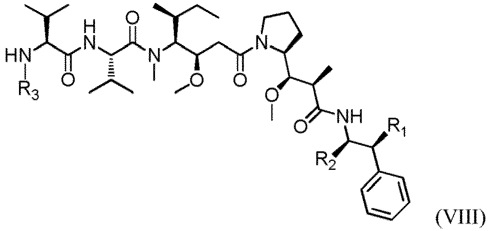
worin R₁, R₂ und R₃ wie in Anspruch 1 definiert sind, und
eine Verbindung der Formel (XI):
R_{4b}-CHO (XI)
worin R_{4b} wie zuvor definiert ist.

## Revendications

1. Composé de formule (I) suivante : 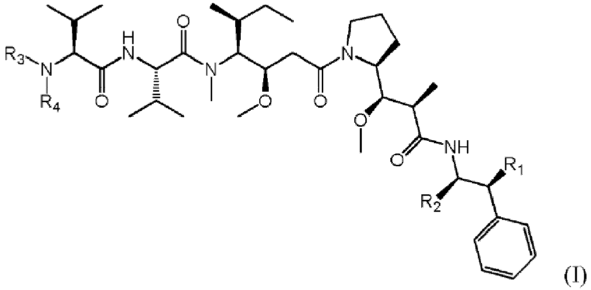 dans laquelle :
- R₁ = OH et R₂ = un groupe méthyle, ou
R₁ = H et R₂ = un groupe COOH, COOMe ou thiazol-2-yle,
- R₃ est un groupe (C₁-C₆)alkyle, et
- R₄ est un groupe phényl-(C₁-C₂)alkyle substitué par un groupe sélectionné parmi les groupes OH et NR₉R₁₀ avec R₉ et R₁₀ représentant chacun, indépendamment l'un de l'autre, H ou un groupe (C₁-C₆)alkyle, dans lequel le groupe phényl-(C₁-C₂)alkyle est lié au reste de la molécule via sa fraction (C₁-C₂)alkyle,
ou un sel, hydrate ou solvate pharmaceutiquement acceptable de celui-ci.

2. Composé selon la revendication 1, **caractérisé en ce que** R₁ représente H et R₂ représente un groupe COOH ou COOMe.

3. Composé selon l'une quelconque des revendications 1 et 2, **caractérisé en ce que** R₃ représente un groupe méthyle.

4. Composé selon l'une quelconque des revendications 1 à 3, **caractérisé en ce que** R₄ représente un groupe phényl-(C₁-C₂)alkyle substitué par un groupe sur la fraction phényle choisi parmi OH et NR₉R₁₀.

5. Composé selon la revendication 4, **caractérisé en ce que** R₄ représente un groupe phényl-(C₁-C₂)alkyle substitué par un groupe NR₉R₁₀ sur la fraction phényle.

6. Composé selon l'une quelconque des revendications 1 à 3, **caractérisé en ce que** R₄ a la formule suivante : 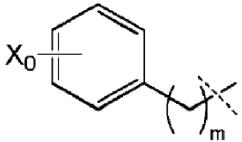 dans laquelle X₀ représente OH ou NR₉R₁₀ et m vaut 1 ou 2.

7. Composé selon la revendication 6, **caractérisé en ce que** X₀ est NR₉R₁₀.

8. Composé selon l'une quelconque des revendications 1 à 3, **caractérisé en ce que** R₄ a la formule suivante : 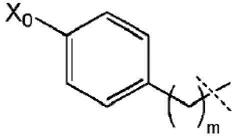 dans laquelle X₀ représente NR₉R₁₀ et m représente 1 ou 2.

9. Composé selon la revendication 1, choisi parmi : 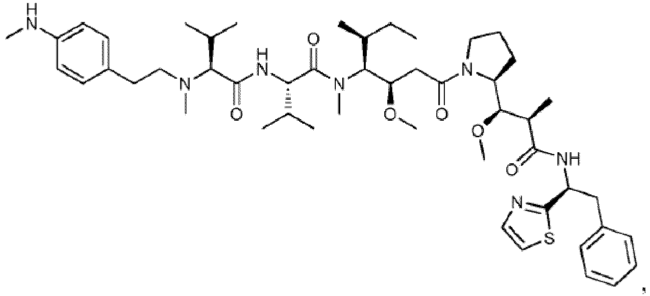 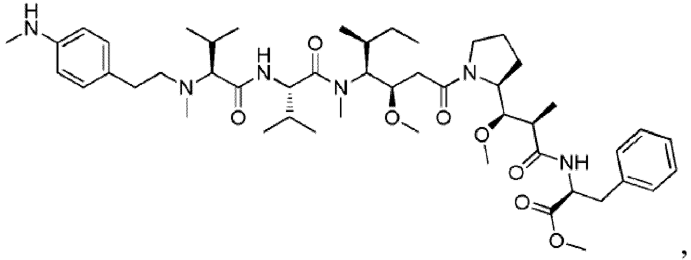 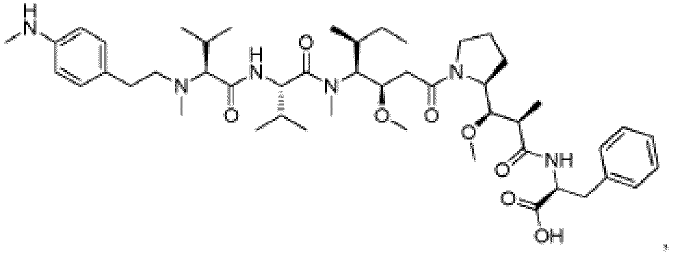 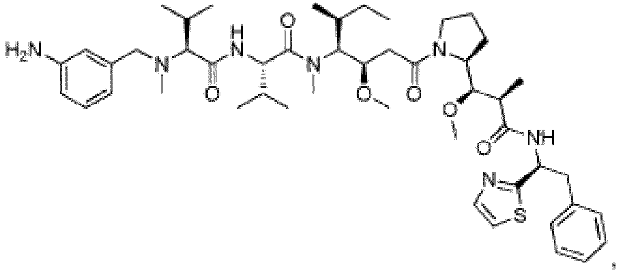 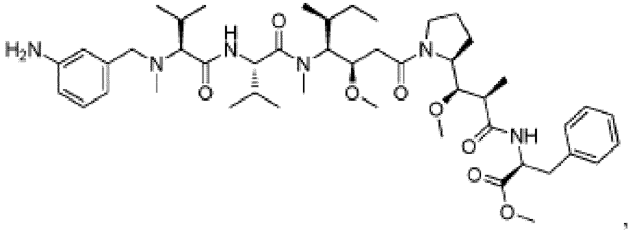 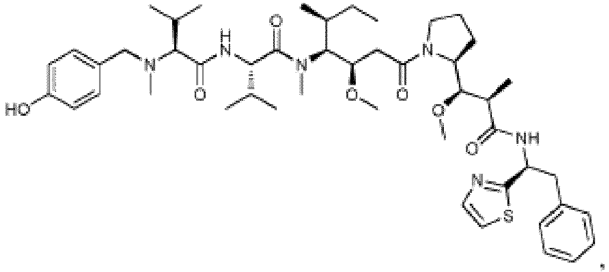 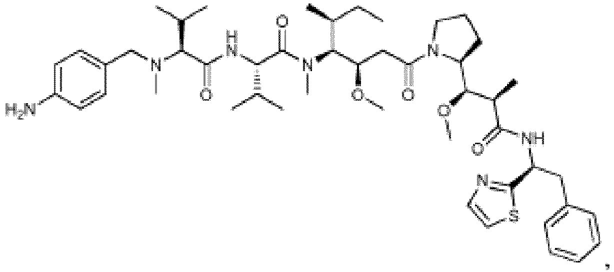 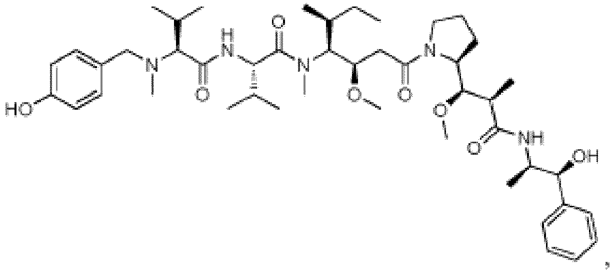 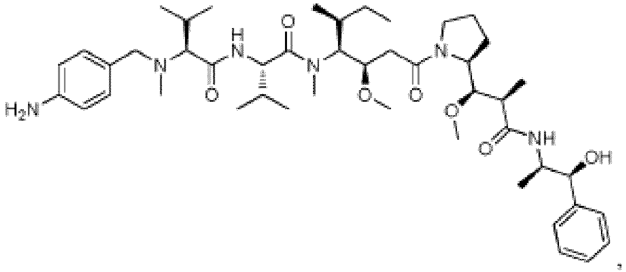 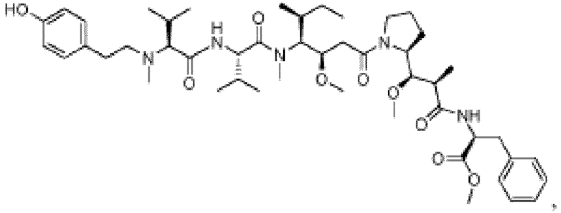 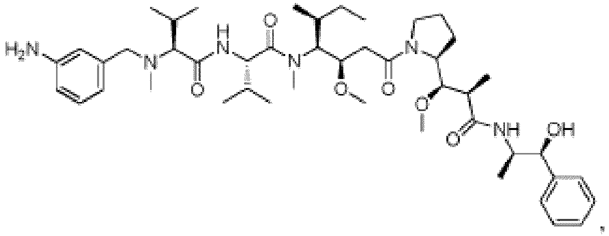 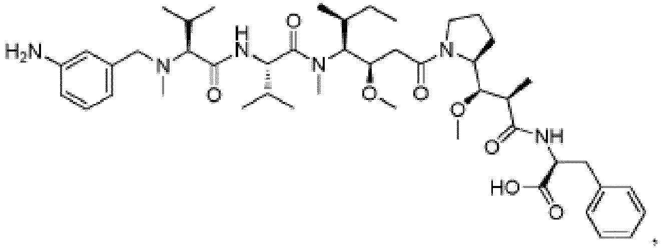 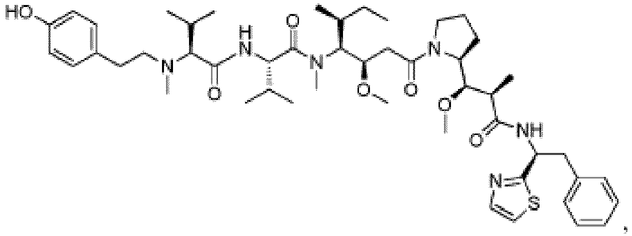 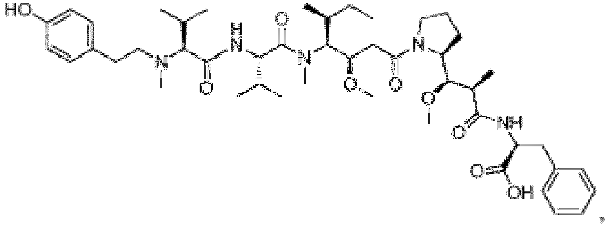 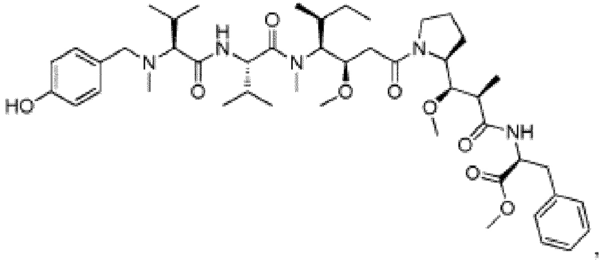 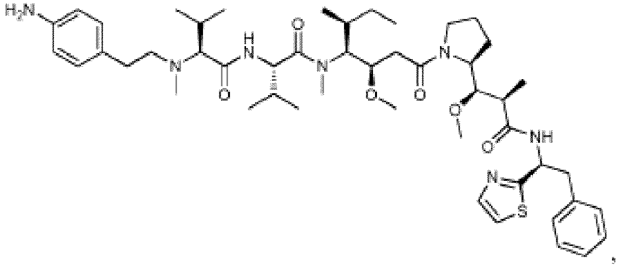 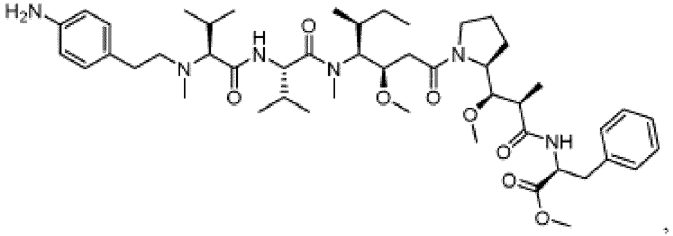 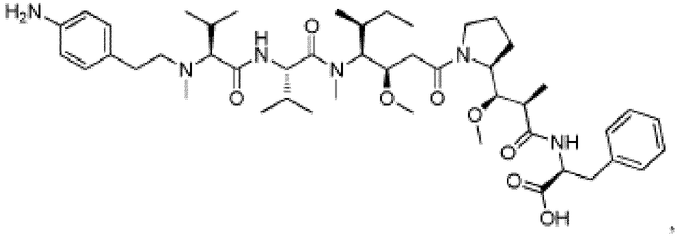 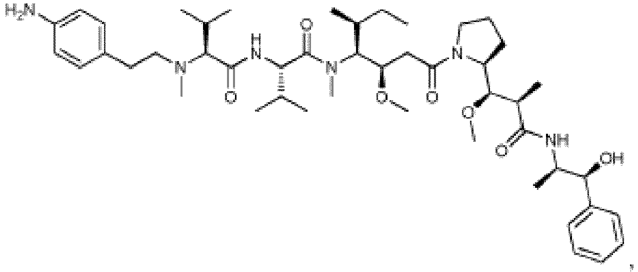 et leurs sels pharmaceutiquement acceptables tels que les sels formés avec l'acide trifluoroacétique.

10. Composé selon l'une quelconque des revendications 1 à 9, pour son utilisation comme médicament.

11. Composé selon l'une quelconque des revendications 1 à 9, pour son utilisation comme médicament destiné au traitement du cancer ou des désordres prolifératifs bénins.

12. Composition pharmaceutique comprenant un composé de formule (I) selon l'une quelconque des revendications 1 à 9 et au moins un excipient pharmaceutiquement acceptable.

13. Composition pharmaceutique selon la revendication 12, comprenant en outre un autre principe actif, avantageusement choisi parmi les agents anticancéreux, comprenant notamment les agents anticancéreux cytotoxiques tels que la navelbine, la vinflunine, le taxol, le taxotère, le 5-fluorouracile, le méthotrexate, la doxorabicine, la camptothécine, la gemcitabine, l'étoposide, le cis-platine ou la carmustine ; et les agents anticancéreux hormonaux tels que le tamoxifène ou la médroxyprogestérone.

14. Procédé de préparation d'un composé de formule (I) selon l'une quelconque des revendications 1 à 9 comprenant une réaction de condensation entre un composé de formule (VI) suivante : 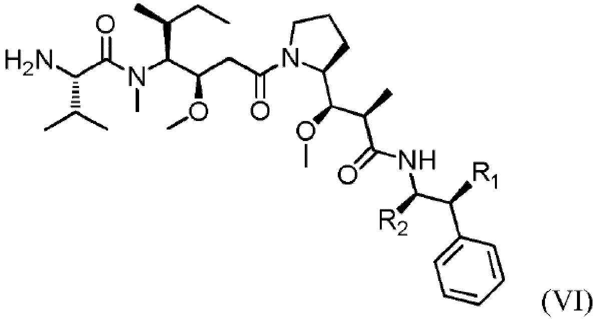
dans laquelle R₁ et R₂ sont tels que définis dans la revendication 1, et
un composé de formule (VII) suivante : 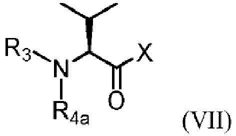
dans laquelle R₃ est tel que défini dans la revendication 1, R₄ₐ représente un groupe R₄ tel que défini dans la revendication 1, éventuellement sous une forme protégée, et X représente OH ou Cl.

15. Procédé de préparation d'un composé de formule (I) selon l'une quelconque des revendications 1 à 9, comprenant une réaction de substitution entre un composé de formule (VIII) suivante : 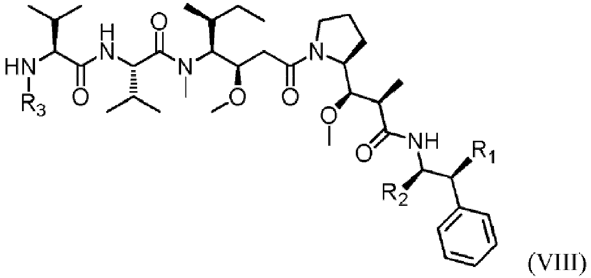
dans laquelle R₁, R₂ et R₃ sont tels que définis dans la revendication 1, et
un composé de formule (X) suivante :
R₄ₐ-Y (X)
dans laquelle R₄ₐ représente un groupe R₄ tel que défini dans la revendication 1, éventuellement sous une forme protégée, et Y représente un groupe partant tel que Cl, Br, I, OSO₂CH₃, OSO₂CF₃ ou O-Tosyle.

16. Procédé de préparation d'un composé de formule (I) selon l'une quelconque des revendications 1 à 9 dans lequel R₄ représente un groupe -CH₂R_{4b} avec R_{4b} représentant un groupe phényle ou phényl-méthyle substitué par un groupe choisi parmi les groupes OH et NR₉R₁₀,
comprenant une réaction d'amination réductrice entre un composé de formule (VIII) suivante : 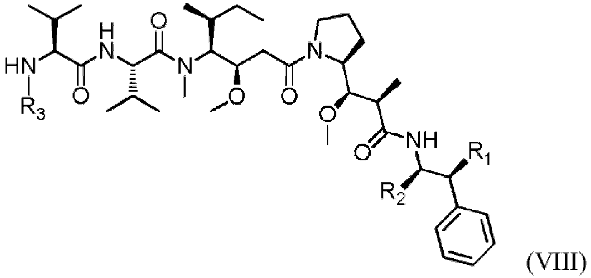
dans laquelle R₁, R₂ et R₃ sont tels que définis dans la revendication 1, et
un composé de formule (XI) suivante :
R_{4b}-CHO (XI)
dans laquelle R_{4b} est tel que défini précédemment.
